# EUROPÄISCHE PATENTSCHRIFT

(11) **EP 1 517 687 B1**
(45) Veröffentlichungstag und Bekanntmachung des Hinweises auf die Patenterteilung: **20.06.2007**
(21) Anmeldenummer: 03740255.9
(22) Anmeldetag: 16.06.2003
(51) Int. Cl.: A61K 31/4184, A61P 31/00

(54) **ARZNEIMITTEL ZUR BEHANDLUNG DES "SYSTEMIC INFLAMMATORY RESPONSE SYNDROME"**
MEDICAMENT FOR THE TREATMENT OF THE SYSTEMIC INFLAMMATORY RESPONSE SYNDROME
MEDICAMENT POUR TRAITER LE SYNDROME DE REPONSE INFLAMMATOIRE SYSTEMIQUE

(30) Priorität: 20.06.2002 DE 10227668
(43) Veröffentlichungstag der Anmeldung: 30.03.2005
(73) Patentinhaber: Boehringer Ingelheim Pharma GmbH & Co. KG, 55216 Ingelheim am Rhein (DE)
(72) Erfinder: RIES, Uwe, 88400 Biberach (DE); WIENEN, Wolfgang, 88400 Biberach-Rissegg (DE); SCHÜLY, Uwe, 88441 Mittelbiberach (AT)
(86) Internationale Anmeldenummer: PCT/EP2003/006318
(87) Internationale Veröffentlichungsnummer: WO 2004/000310

(56) Entgegenhaltungen:
- WO-A-00/01704
- WO-A-03/000682
- WO-A-03/035065
- DE-A- 19 920 936
- US-B1- 6 407 130
- HALL J E ET AL: "ANTI-PNEUMOCYSTIS ACTIVITIES OF AROMATIC DIAMIDOXIME PRODRUGS" ANTIMICROBIAL AGENTS AND CHEMOTHERAPY, AMERICAN SOCIETY FOR MICROBIOLOGY, WASHINGTON, DC, US, Bd. 42, Nr. 3, März 1998 (1998-03), Seiten 666-674, XP000974178 ISSN: 0066-4804

## Beschreibung

Die vorliegende Erfindung betrifft die Verwendung bestimmter Benzimidazole der allgemeinen Formel (I) 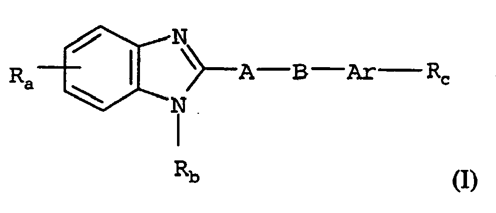 und insbesondere der Verbindung der Formel (II) 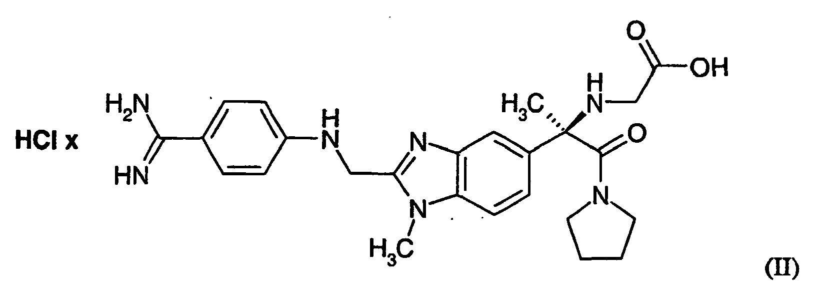 zur Herstellung eines Arzneimittels zur Behandlung des "systemic inflammatory response syndrome".

Das "systemic inflammatory response syndrome" ("Syndrom einer systemischen Entzündungsreaktion", nachfolgend abgekürzt "SIRS"; Bone R.C. et al.: "Definitions for Sepsis and Organ Failure and Guidelines for the Use of Innovative Therapies in Sepsis", ACCP / SCCM Consensus Conference Committee, Chest (1992) 101: 1644) wird im Zusammenhang mit Insulten oder Traumata verschiedenster Art beobachtet. Es treten dabei die typischen Symptome einer systemischen Entzündungsreaktion auf, wie insbesondere:
(a) erhöhte oder erniedrigte Körpertemperatur von über 38°C bzw. unter 36°C,
(b) eine Herzfrequenz von mehr als 90 Schlägen pro Minute,
(c) beschleunigte Atmung mit einer Atemfrequenz von über 20 pro Minute oder Hyperventilation mit einem PaCO₂ kleiner als 32 mmHg,
(d) Veränderungen im weißen Blutbild mit Leukozytenzahlen von mehr als 12.000/mm³ oder unter 4.000/mm³ oder die Anwesenheit von mehr als 10% unreifen Neutrophilen.

Die Ursachen für das Auftreten von SIRS sind vielfältig. Eine bedeutende Ursache sind Infektionen, insbesondere solche verursacht durch gram-positive oder gram-negative Bakterien, Pilze, Viren oder eukaryontische Einzeller, sowie Mischinfektionen mit verschiedenen Erregern (Balk R.A.: "Severe Sepsis and Septic Shock", Critical Care Clinics (2000) 16: 179; Riewald M., Riess H.: "Treatment Options for Clinically Recognized Disseminated Intravascular Coagulation", Seminars in Thrombosis and Hemostasis (1998) 24: 53). Hierbei tritt häufig auch das klinische Bild der Sepsis oder Septikämie auf. Sepsis ist definiert als Allgemeininfektion mit Krankheitserscheinungen, die infolge konstanter oder periodischer Aussaat von Mikroorganismen von einem Herd aus in die Blutbahn auftreten. Häufig vorkommende Sepsiserreger sind gram-negative Erreger wie z.B. Escherichia coli und andere Enterobacteriaceae (Klebsiella, Proteus, Enterobacter), Pseudomonas aeruginosa, Neisseria meningitidis, Salmonella, Serratia und Bacteroides. Gram-positive Erreger sind z.B. Staphylokokken, Streptokokken, Pneumokokken, Enterokokken u. Clostridium perfringens. Klinische Symptome der Sepsis sind typischerweise hohes, intermittierendes Fieber, Schüttelfrost und deutlich beeinträchtigtes Allgemeinbefinden bis zur Verwirrtheit. Im weiteren Verlauf der Krankheit kann es zu (weicher) Milz- und Lebervergrößerung sowie infektiös-toxischer Schädigungen an inneren Organen (Niere, Lunge, Herz) kommen. Die Therapie besteht im Wesentlichen aus einer antibiotischen Behandlung, wobei die Wahl des Antibiotikums sich nach dem Erreger richtet (häufig Cephalosporine od. penicillinasefeste Penicilline in Kombination mit einem Aminoglykosid). Die Prognose ist selbst bei Anwendung intensivmedizinischer Maßnahmen ernst. Die Letalität beträgt um die 50%. Ganz besonders kritisch betroffen sind ältere, kranke oder immunkompromittierte Patienten.

Beispiele für nicht-infektiöse Ursachen für das Auftreten von SIRS sind: Pankreatitis, systemische und organbegrenzte Ischämien, Traumata diverser Natur (z.B. multiple Knochenbrüche), Gewebeverletzungen, großflächige Verbrennungen, ausgedehnte Operationen, Schock verschiedener Ursachen einschließlich des Blutungsschocks und eines Zustandes nach Herz-Kreislaufversagens bis hin zur Pulslosigkeit und Zustand nach Reanimation, das immunvermittelte Organversagen und Entzündungsreaktionen, die durch die Gabe von potentiellen Mediatoren eines Entzündungsprozesses, wie z. B. "Tumor Necrosis Factor" und anderer Cytokinen induziert werden.

Verbindungen der allgemeinen Formel (I) 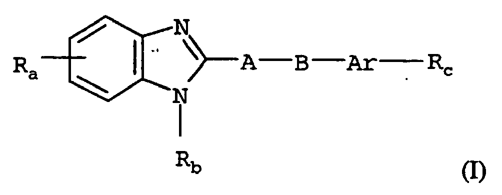 und insbesondere die Verbindung (R)-2-(4-Amidinophenylaminomethyl)-1-methyl-5-[1-(carboxymethylamino)-1-(pyrrolidinocarbonyl)-ethyl]-benzimidazol der Formel (IIa) 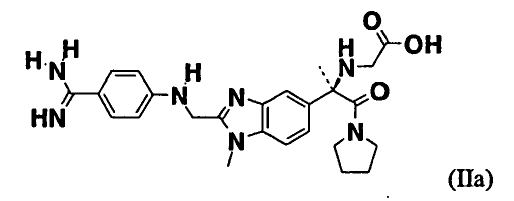 sind aus der WO 00/01704 bekannt. Ebenfalls aus der WO 00/01704 bekannt ist deren antithrombotische Wirkung.

Überraschenderweise wurde nun gefunden, dass Benzimidazole der allgemeinen Formel (I) 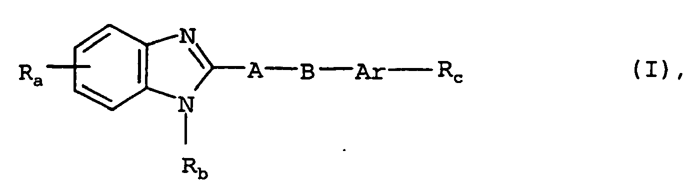 in der
Ar eine gegebenenfalls durch ein Fluor-, Chlor- oder Bromatom, durch eine Trifluormethyl-, C₁₋₃-Alkyl- oder C₁₋₃-Alkoxygruppe substituierte Phenylen- oder Naphthylengruppe,
eine gegebenenfalls im Kohlenstoffgerüst durch eine C₁₋₃-Alkyl-gruppe substituierte Thienylen-, Thiazolylen-, Pyridinylen-, Pyrimidinylen-, Pyrazinylen- oder Pyridazinylengruppe,
A eine C₁₋₃-Alkylengruppe,
B ein Sauerstoff- oder Schwefelatom, eine Methylen-, Carbonyl-, Sulfinyl- oder Sulfonylgruppe, eine gegebenenfalls durch eine C₁₋₃-Alkylgruppe substituierte Iminogruppe, in der der Alkylteil durch eine Carboxygruppe mono- oder disubstituiert sein kann,
Rₐ eine R₁-CO-C₃₋₅-cycloalkylgruppe, in der
   R₁ eine C₁₋₃-Alkoxy-, Amino-, C₁₋₄-Alkylamino- oder Di-(C₁₋₄-Alkyl)-aminogruppe, in denen jeweils der Alkylteil durch eine Carboxygruppe substituiert sein kann,
   eine 4- bis 7-gliedrige Cycloalkylenimino- oder Cycloalkenyleniminogruppe, die durch eine oder zwei C₁₋₃-Alkylgruppen substituiert sein können, wobei ein Alkylsubstituent gleichzeitig durch eine Hydroxy-, C₁₋₃-Alkoxy-, Carboxy-, Carboxy-C₁₋₃-alkoxy-, Carboxy-C₁₋₃-alkylamino-, N-(C₁₋₃-Alkyl)-N-(carboxy-C₁₋₃-alkyl)-amino-, Carboxy-C₁₋₃-alkylaminocarbonyl-, N-(C₁₋₃-Alkyl)-N-(carboxy-C₁₋₃-alkyl)-aminocarbonyl-, Carboxy-C₁₋₃-alkylaminocarbonylamino-, 1-(C₁₋₃-Alkyl)-3-(carboxy-C₁₋₃-alkyl)-aminocarbonylamino-, 3-(C₁₋₃-Alkyl)-3-(carboxy-C₁₋₃-alkyl)-aminocarbonylamino- oder 1,3-Di-(C₁₋₃-alkyl)-3-(carboxy-C₁₋₃-alkyl)-aminocarbonylaminogruppe substituiert sein kann,
   eine durch eine Hydroxygruppe substituierte 4- bis 7-gliedrige Cycloalkyleniminogruppe,
   eine gegebenenfalls durch eine C₁₋₃-Alkylgruppe substituierte 5- bis 7-gliedrige Cycloalkyleniminogruppe, an die über zwei benachbarte Kohlenstoffatome ein Phenylring ankondensiert ist,
   eine Morpholino-, Piperazino-, N-(C₁₋₃-Alkyl)-piperazino-, Pyrrolino-, 3,4-Dehydropiperidino- oder Pyrrol-1-yl-Gruppe darstellt,
eine R₂-CX-C₃₋₅-cycloalkylgruppe, in der
   R₂ eine gegebenenfalls durch eine C₁₋₃-Alkylgruppe substituierte Phenyl-, Naphthyl- oder monocyclische 5- oder 6-gliedrige Heteroarylgruppe, wobei die 6-gliedrige Heteroarylgruppe ein, zwei oder drei Stickstoffatome und die 5-gliedrige Heteroarylgruppe eine gegebenenfalls durch eine C₁₋₃-Alkylgruppe substituierte Iminogruppe, ein Sauerstoff- oder Schwefelatom oder eine gegebenenfalls durch eine C₁₋₃-Alkylgruppe substituierte Iminogruppe und ein Sauerstoff- oder Schwefelatom oder ein oder zwei Stickstoffatome enthält und der vorstehend erwähnte Alkylsubstituent durch eine Carboxy-, Carboxy-C₁₋₃-alkoxy-, Carboxy-C₁₋₃-alkylamino- oder N-(C₁₋₃-Alkyl)-carboxy-C₁₋₃-alkylaminogruppe substituiert sein kann, und
   X ein Sauerstoffatom, eine C₁₋₃-Alkylimino-, C₁₋₃-Alkoxyimino-, C₁₋₃-Alkyl-hydrazino-, Di-(C₁₋₃-Alkyl)-hydrazino-, C₂₋₄-Alkanoylhydrazino-, N-(C₁₋₃-Alkyl)-C₂₋₄-alkanoylhydrazino- oder C₁₋₃-Alkylidengruppe, die jeweils im Alkyl- oder Alkanoylteil oder im Alkyl- und Alkanoylteil durch eine Carboxygruppe substituiert sein können, darstellen,
eine durch eine Imidazol- oder Imidazolongruppe substituierte C₁₋₃-Alkyl- oder C₃₋₅-Cycloalkylgruppe, in denen
   der Imidazolring durch eine Phenyl- oder Carboxygruppe und durch eine oder zwei C₁₋₃-Alkylgruppen oder durch eine, zwei oder drei C₁₋₃-Alkylgruppen substituiert sein kann; wobei die Substituenten gleich oder verschieden sein können und einer der vorstehend erwähnten Alkylsubstituenten gleichzeitig durch eine Carboxygruppe oder in 2- oder 3-Stellung durch eine Amino-, C₂₋₄-Alkanoylamino-, C₁₋₃-Alkylamino-, N-(C₂₋₄-Alkanoyl)-C₁₋₃-alkylamino- oder Di-(C₁₋₃-Alkyl)-aminogruppe substituiert sein kann, und
   der Imidazolonring durch eine C₁₋₃-Alkylgruppe substituiert sein kann, wobei der Alkylsubstituent durch eine Carboxygruppe oder in 2- oder 3-Stellung durch eine Amino-, C₂₋₄-Alkanoylamino-, C₁₋₃-Alkylamino-, N-(C₂₋₄-Alkanoyl)-C₁₋₃-alkylamino- oder Di-(C₁₋₃-Alkyl)-aminogruppe substituiert sein kann, und
   zusätzlich an die vorstehend erwähnten Imidazol- und Imidazolonringe über zwei benachbarte Kohlenstoffatome ein Phenyl- oder Pyridinring ankondensiert sein kann,
eine Imidazolidin-2,4-dion-5-yl-Gruppe, die durch eine oder zwei C₁₋₃-Alkylgruppen substituiert sein kann, wobei gleichzeitig ein Alkylsubstituent durch eine Carboxygruppe substituiert sein kann,
eine C₁₋₄-Alkylgruppe, die
   durch eine C₁₋₃-Alkyl-Y₁-C₁₋₃-alkyl-, HOOC-C₁₋₃-alkyl-Y₁-C₁₋₃-alkyl-, Tetrazolyl-C₁₋₃-alkyl-Y₂-, R₃NR₄- oder R₃NR₄-C₁₋₃-alkyl-Gruppe und
   durch eine gegebenenfalls durch eine C₁₋₃-Alkylgruppe substituierte Isoxazolidinylcarbonylgruppe, durch eine Pyrrolinocarbonyl-, 3,4-Dehydro-piperidinocarbonyl-, Pyrrol-1-yl-carbonyl-, Carboxy-, Aminocarbonyl-, C₁₋₃-Alkylaminocarbonyl-, Di-(C₁₋₃-Alkyl)-aminocarbonyl- oder 4- bis 7-gliedrige Cycloalkyleniminocarbonylgruppe substituiert ist, wobei bei den vorstehend erwähnten Gruppen der Cycloalkyleniminoteil durch eine oder zwei C₁₋₃-Alkylgruppen substituiert und gleichzeitig jeweils ein Alkylteil oder Alkylsubstituent der vorstehend erwähnten C₁₋₃-Alkylaminocarbonyl-, Di-(C₁₋₃-Alkyl)-aminocarbonyl- oder Cycloalkyleniminocarbonylgruppen durch eine Carboxygruppe substituiert sein kann, und die verbleibenden Wasserstoffatome der C₁₋₄-Alkylgruppe ganz oder teilweise durch Fluoratome ersetzt sein können, in denen
      R₃ ein Wasserstoffatom oder eine gegebenenfalls durch eine Carboxygruppe substituierte C₁₋₃-Alkylgruppe und
      R₄ ein Wasserstoffatom, eine C₁₋₃-Alkyl-Y₁-C₁₋₃-alkyl-Y₂-, Carboxy-C₁₋₃-alkyl-Y₁-C₁₋₃-alkyl-Y₂-, C₁₋₃-Alkyl-Y₂- oder Carboxy-C₁₋₃-alkyl-Y₂-Gruppe oder
      R₃ und R₄ zusammen mit dem dazwischen liegenden Stickstoffatom eine gegebenenfalls durch eine Carboxy-, C₁₋₃-Alkyl- oder Carboxy-C₁₋₃-alkylgruppe substituierte 4- bis 7-gliedrige Cycloalkyleniminogruppe darstellen, in denen
   Y₁ eine Kohlenstoff-Kohlenstoff-Bindung, ein Sauerstoffatom, eine Sulfenyl-, Sulfinyl-, Sulfonyl-, -NH-, -NH-CO- oder -NH-CO-NH-Gruppe und
   Y₂ eine Kohlenstoff Stickstoffbindung oder eine Carbonyl-, Sulfonyl-, Imino- oder -NH-CO-Gruppe darstellen, wobei die Carbonylgruppe der -NH-CO-Gruppe mit dem Stickstoffatom der R₃NR₄-Gruppe verknüpft ist, und die bei der Definition der Reste Y₁ und Y₂ vorkommenden Iminogruppen jeweils zusätzlich durch eine C₁₋₃-Alkyl- oder Carboxy-C₁₋₃-alkylgruppe substituiert sein können,
eine durch eine R₅NR₆-Gruppe substituierte C₁₋₃-Alkyl- oder C₃₋₅-Cycloalkylgruppe, in denen
   R₅ ein Wasserstoffatom, eine C₁₋₃-Alkyl-, C₅₋₇-Cycloalkyl-, Phenylcarbonyl-, Phenylsulfonyl- oder Pyridinylgruppe und
   R₆ eine C₁₋₃-Alkyl-, Carboxy-C₁₋₃-alkyl- oder Carboxy-C₁₋₃-alkylcarbonylgruppe darstellen,
eine C₁₋₃-Alkylgruppe, die durch C₂₋₄-Alkanoyl- oder C₅₋₇-Cycloalkanoylgruppe und durch eine durch ein Chlor-, Brom- oder Jodatom substituierte C₁₋₃-Alkylgruppe substituiert ist,
R_{b} ein Wasserstoffatom oder eine C₁₋₃-Alkylgruppe und
R_{c} eine Cyanogruppe oder eine gegebenenfalls durch eine oder zwei C₁₋₃-Alkylgruppen substituierte Amidinogruppe bedeuten, worin
   die bei der Definition der vorstehend erwähnten Reste erwähnten Carboxygruppen außerdem durch eine in-vivo in eine Carboxygruppe überführbare Gruppe oder durch eine unter physiologischen Bedingungen negativ geladene Gruppe ersetzt sein können oder
   die bei der Definition der vorstehend erwähnten Reste erwähnten Amino- und Iminogruppen außerdem durch einen in vivo abspaltbaren Rest substituiert sein können, wobei
   unter einer in-vivo in eine Carboxygruppe überführbaren Gruppe eine Hydroxmethylgruppe, eine mit einem Alkohol veresterte Carboxygruppe, in der der alkoholische Teil ein C₁₋₆-Alkanol, ein Phenyl-C₁₋₃-alkanol, ein C₃₋₉-Cycloalkanol, wobei ein C₅₋₈-Cycloalkanol zusätzlich durch ein oder zwei C₁₋₃-Alkylgruppen substituiert sein kann, ein C₅₋₈-Cycloalkanol, in dem eine Methylengruppe in 3- oder 4-Stellung durch ein Sauerstoffatom oder durch eine gegebenenfalls durch eine C₁₋₃-Alkyl-, Phenyl-C₁₋₃-alkyl-, Phenyl-C₁₋₃-alkoxycarbonyl- oder C₂₋₆-Alkanoylgruppe substituierte Iminogruppe ersetzt ist und der Cycloalkanolteil zusätzlich durch ein oder zwei C₁₋₃-Alkylgruppen substituiert sein kann, ein C₄₋₇-Cycloalkenol, ein C₃₋₅-Alkenol, ein Phenyl-C₃₋₅-alkenol, ein C₃₋₅-Alkinol oder Phenyl- C₃₋₅-alkinol mit der Maßgabe, dass keine Bindung an das Sauerstoffatom von einem Kohlenstoffatom ausgeht, welches eine Doppel- oder Dreifachbindung trägt, ein C₃₋₈-Cycloalkyl-C₁₋₃-alkanol, ein Bicycloalkanol mit insgesamt 8 bis 10 Kohlenstoffatomen, das im Bicycloalkylteil zusätzlich durch eine oder zwei C₁₋₃-Alkylgruppen substituiert sein kann, ein 1,3-Dihydro-3-oxo-1-isobenzfuranol oder ein Alkohol der Formel

      R_{d}-CO-O-(RₑCR_{f})-OH,
   verstanden wird, in dem
   R_{d} eine C₁₋₈-Alkyl-, C₅₋₇-Cycloalkyl-, Phenyl- oder Phenyl- C₁₋₃-alkylgruppe
   Rₑ ein Wasserstoffatom, eine C₁₋₃-Alkyl-, C₅₋₇-Cycloalkyl- oder Phenylgruppe und
   R_{f} ein Wasserstoffatom oder eine C₁₋₃-Alkylgruppe darstellen,
   unter einer unter physiologischen Bedingungen negativ geladenen Gruppe eine Tetrazol-5-yl-, Phenylcarbonylaminocarbonyl-, Trifluormethylcarbonylaminocarbonyl-, C₁₋₆-Alkylsulfonylamino-, Phenylsulfonylamino-, Benzylsulfonylamino-, Trifluormethylsulfonylamino-, C₁₋₆- Alkylsulfonylaminocarbonyl-, Phenylsulfonylminocarbonyl-, Benzylsulfonylaminocarbonyl- oder Perfluor-C₁₋₆-alkylsulfonylaminocarbonylgruppe
   und unter einem von einer Imino- oder Aminogruppe in-vivo abspaltbaren Rest eine Hydroxygruppe, eine gegebenenfalls durch Fluor-, Chlor-, Brom- oder Jodatome, durch C₁₋₃-Alkyl- oder C₁₋₃-Alkoxygruppen mono- oder disubstituierte Benzoylgruppe, wobei die Substituenten gleich oder verschieden sein können, eine Pyridinoylgruppe oder eine C₁₋₁₆-Alkanoylgruppe, eine 3,3,3-Trichlorpropionyl- oder Allyloxycarbonylgruppe, eine C₁₋₁₆-Alkoxycarbonyl- oder C₁₋₁₆-Alkylcarbonyloxygruppe, in denen Wasserstoffatome ganz oder teilweise durch Fluor- oder Chloratome ersetzt sein können, eine Phenyl-C₁₋₆-alkoxycarbonylgruppe, eine 3-Amino-propionylgruppe, in der die Aminogruppe durch C₁₋₆-Alkyl- oder C₃₋₇-Cycloalkylgruppen mono- oder disubstituiert und die Substituenten gleich oder verschieden sein können, eine C₁₋₃-Alkylsulfonyl-C₂₋₄-alkoxycarbonyl-, C₁₋₃-Alkoxy-C₂₋₄-alkoxy-C₂₋₄-alkoxycarbonyl-, R_{d}-CO-O-(R_{d}CR_{f})-O-CO-, C₁₋₆-Alkyl-CO-NH-(R_{g}CRₕ)-O-CO- oder C₁₋₆-Alkyl-CO-O-(R_{g}CRₕ)-(RgCRₕ)-O-CO-Gruppe, in denen R_{d} bis R_{f} wie vorstehend erwähnt definiert sind und
   R_{g} und Rₕ, die gleich oder verschieden sein können, Wasserstoffatome oder C₁₋₃-Alkylgruppen darstellen,
   verstanden werden,
deren Tautomere, deren Stereoisomere, deren Gemische und deren Salze auch zur Herstellung eines Arzneimittels zur Behandlung oder Vorbeugung des SIRS, des akuten Herz-Kreislaufversagens, des Organversagens nach Reanimation, des akuten Lungenversagens und des akuten Atemnotsyndroms des erwachsenen ARDS (acute respiratory distress syndrome) und insbesondere der Sepsis verwendet werden können.

Demgemäß betrifft die Erfindung die Verwendung von Benzimidazolen der oben gezeigten Formel (I), in der Ra, Rb, Rc, A, B und Ar die oben genannten Bedeutungen haben, gegebenenfalls in Form der pharmazeutisch verträglichen Säureadditionssalze sowie gegebenenfalls in Form der Hydrate oder Solvate zur Herstellung eines Arzneimittels zur Bhandlung des SIRS, der Bakteriämie und/oder der Sepsis einschließlich der "severe sepsis", des akuten Herz-Kreislaufversagens, des Organversagens nach Reanimation, des akuten Lungenversagens und des ARDS.

Vorzugsweise werden Benzimidazole der allgemeinen Formel (Ia) 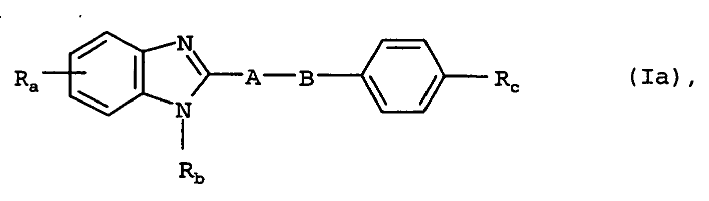 verwendet, in der
A eine C₁₋₃-Alkylengruppe,
B ein Sauerstoff- oder Schwefelatom, eine Methylen-, Carbonyl-, Sulfinyl- oder Sulfonylgruppe, eine gegebenenfalls durch eine C₁₋₃-Alkylgruppe substituierte Iminogruppe, in der der Alkylteil durch eine Carboxygruppe mono- oder disubstituiert sein kann,
Rₐ eine R₁-CO-C₃₋₅-cycloalkylgruppe, in der
   R₁ eine C₁₋₃-Alkoxy-, Amino-, C₁₋₄-Alkylamino- oder Di-(C₁₋₄-Alkyl)-aminogruppe, in denen jeweils der Alkylteil durch eine Carboxygruppe substituiert sein kann,
   eine 4- bis 7-gliedrige Cycloalkylenimino- oder Cycloalkenylenyiminogruppe, die durch eine oder zwei C₁₋₃-Alkylgruppe substituiert sein können, wobei ein Alkylsubstituent gleichzeitig durch eine Hydroxy-, C₁₋₃-Alkoxy-, Carboxy-, Carboxy-C₁₋₃-alkoxy-, Carboxy-C₁₋₃-alkylamino-, N-(C₁₋₃-Alkyl)-N-(carboxy-C₁₋₃-alkyl)-amino-, Carboxy-C₁₋₃-alkylaminocarbonyl-, N-(C₁₋₃-Alkyl)-N-(carboxy-C₁₋₃-alkyl)-aminocarbonyl-, Carboxy-C₁₋₃-alkylaminocarbonylamino-, 1-(C₁₋₃-Alkyl)-3-(carboxy-C₁₋₃-alkyl)-aminocarbonylamino-, 3-(C₁₋₃-Alkyl)-3-(carboxy-C₁₋₃-alkyl)-aminocarbonylamino- oder 1,3-Di-(C₁₋₃-alkyl)-3-(carboxy-C₁₋₃-alkyl)-aminocarbonylaminogruppe substituiert sein kann,
   eine durch eine Hydroxygruppe substituierte 4- bis 7-gliedrige Cycloalkyleniminogruppe,
   eine gegebenenfalls durch eine C₁₋₃-Alkylgruppe substituierte 5- bis 7-gliedrige Cycloalkyleniminogruppe, an die über zwei benachbarte Kohlenstoffatome ein Phenylring ankondensiert ist,
   eine Morpholino-, Piperazino-, N-(C₁₋₃-Alkyl)-piperazino-, Pyrrolino-, 3,4-Dehydro-piperidino- oder Pyrrol-1-yl-Gruppe darstellt,
eine R₂-CX-C₃₋₅-cycloalkylgruppe, in der
   R₂ eine gegebenenfalls durch eine C₁₋₃-Alkylgruppe substituierte Phenyl-, Naphthyl- oder monocyclische 5- oder 6-gliedrige Heteroarylgruppe, wobei die 6-gliedrige Heteroarylgruppe ein, zwei oder drei Stickstoffatome und die 5-gliedrige Heteroarylgruppe eine gegebenenfalls durch eine C₁₋₃-Alkylgruppe substituierte Iminogruppe, ein Sauerstoff- oder Schwefelatom oder eine gegebenenfalls durch eine C₁₋₃-Alkylgruppe substituierte Iminogruppe und ein Sauerstoff- oder Schwefelatom oder ein oder zwei Stickstoffatome enthält und der vorstehend erwähnte Alkylsubstituent durch eine Carboxy-, Carboxy-C₁₋₃-alkoxy-, Carboxy-C₁₋₃-alkylamino- oder N-(C₁₋₃-Alkyl)-carboxy-C₁₋₃-alkylaminogruppe substituiert sein kann, und
   X ein Sauerstoffatom, eine C₁₋₃-Alkylimino-, C₁₋₃-Alkoxyimino-, C₁₋₃-Alkyl-hydrazino-, Di-(C₁₋₃-Alkyl)-hydrazino-, C₂₋₄-Alkanoylhydrazino-, N-(C₁₋₃-Alkyl)-C₂₋₄-alkanoylhydrazino- oder C₁₋₃-Alkylidengruppe, die jeweils im Alkyl- oder Alkanoylteil oder im Alkyl- und Alkanoylteil durch eine Carboxygruppe substituiert sein können, darstellen,
eine durch eine Imidazol- oder Imidazolongruppe substituierte C₁₋₃-Alkyl- oder C₃₋₅-Cycloalkylgruppe, in denen
   der Imidazolring durch eine Phenyl- oder Carboxygruppe und durch eine oder zwei C₁₋₃-Alkylgruppen oder durch eine, zwei oder drei C₁₋₃-Alkylgruppen substituiert sein kann, wobei die Substituenten gleich oder verschieden sein können und einer der vorstehend erwähnten Alkylsubstituenten gleichzeitig durch eine Carboxygruppe oder in 2- oder 3-Stellung durch eine Amino-, C₂₋₄-Alkanoylamino-, C₁₋₃-Alkylamino-, N-(C₂₋₄-Alkanoyl)-C₁₋₃-alkylamino- oder Di-(C₁₋₃-Alkyl)-aminogruppe substituiert sein kann, und
   der Imidazolonring durch eine C₁₋₃-Alkylgruppe substituiert sein kann, wobei der Alkylsubstituent durch eine Carboxygruppe oder in 2- oder 3-Stellung durch eine Amino-, C₂₋₄-Alkanoylamino-, C₁₋₃-Alkylamino-, N-(C₂₋₄-Alkanoyl)-C₁₋₃-alkylamino- oder Di-(C₁₋₃-Alkyl)-aminogruppe substituiert sein kann, und
   zusätzlich an die vorstehend erwähnten Imidazol- und Imidazolonringe über zwei benachbarte Kohlenstoffatome ein Phenyl- oder Pyridinring ankondensiert sein kann,
eine Imidazolidin-2,4-dion-5-yl-Gruppe, die durch eine oder zwei C₁₋₃-Alkylgruppen substituiert sein kann, wobei gleichzeitig ein Alkylsubstituent durch eine Carboxygruppe substituiert sein kann,
eine C₁₋₄-Alkylgruppe, die
   durch eine C₁₋₃-Alkyl-Y₁-C₁₋₃-alkyl-, HOOC-C₁₋₃-alkyl-Y₁-C₁₋₃-alkyl-, Tetrazolyl-C₁₋₃-alkyl-Y₂-, R₃NR₄- oder R₃NR₄-C₁₋₃-alkyl-Gruppe und
   durch eine gegebenenfalls durch eine C₁₋₃-Alkylgruppe substituierte Isoxazolidin-1-ylcarbonylgruppe, durch eine Pyrrolinocarbonyl-, 2,3-Dehydro-piperidinocarbonyl-, Pyrrol-1-yl-carbonyl-, Carboxy-, Aminocarbonyl-, C₁₋₃-Alkylaminocarbonyl-, Di-(C₁₋₃-Alkyl)-aminocarbonyl- oder 4- bis 7-gliedrige Cycloalkyleniminocarbonylgruppe substituiert ist, wobei bei den vorstehend erwähnten Gruppen der Cycloalkyleniminoteil durch eine oder zwei C₁₋₃-Alkylgruppen substituiert und gleichzeitig jeweils ein Alkylteil oder Alkylsubstituent der vorstehend erwähnten C₁₋₃-Alkylaminocarbonyl-, Di-(C₁₋₃-Alkyl)-aminocarbonyl- oder Cycloalkyleniminocarbonylgruppen durch eine Carboxygruppe substituiert sein kann, und die verbleibenden Wasserstoffatome der C₁₋₄-Alkylgruppe ganz oder teilweise durch Fluoratome ersetzt sein können, in denen
      R₃ ein Wasserstoffatom oder eine gegebenenfalls durch eine Carboxygruppe substituierte C₁₋₃-Alkylgruppe und
      R₄ ein Wasserstoffatom, eine C₁₋₃-Alkyl-Y₁-C₁₋₃-alkyl-Y₂-, Carboxy-C₁₋₃-alkyl-Y₁-C₁₋₃-alkyl-Y₂-, C₁₋₃-Alkyl-Y₂- oder Carboxy-C₁₋₃-alkyl-Y₂-Gruppe oder
      R₃ und R₄ zusammen mit dem dazwischen liegenden Stickstoffatom eine gegebenenfalls durch eine Carboxy-, C₁₋₃-Alkyl- oder Carboxy-C₁₋₃-alkylgruppe substituierte 4- bis 7-gliedrige Cycloalkyleniminogruppe darstellen, in denen
   Y₁ eine Kohlenstoff-Kohlenstoff-Bindung, ein Sauerstoffatom, eine Sulfenyl-, Sulfinyl-, Sulfonyl-, -NH-, -NH-CO- oder -NH-CO-NH-Gruppe und
   Y₂ eine Kohlenstoff-Stickstoffbindung oder eine Carbonyl-, Sulfonyl-, Imino- oder -NH-CO-Gruppe darstellen, wobei die Carbonylgruppe der -NH-CO-Gruppe mit dem Stickstoffatom der R₃NR₄-Gruppe verknüpft ist, und die bei der Definition der Reste Y₁ und Y₂ vorkommenden Iminogruppen jeweils zusätzlich durch eine C₁₋₃-Alkyl- oder Carboxy-C₁₋₃-alkylgruppe substituiert sein können,
eine durch eine R₅NR₆-Gruppe substituierte C₁₋₃-Alkyl- oder C₃₋₅-Cycloalkylgruppe, in denen
   R₅ ein Wasserstoffatom, eine C₁₋₃-Alkyl-, C₅₋₇-Cycloalkyl-, Phenylcarbonyl-, Phenylsulfonyl- oder Pyridinylgruppe und
   R₆ eine C₁₋₃-Alkyl-, Carboxy-C₁₋₃-alkyl- oder Carboxy-C₁₋₃-alkylcarbonylgruppe darstellen,
eine C₁₋₃-Alkylgruppe, die durch C₂₋₄-Alkanoyl- oder C₅₋₇-Cycloalkanoylgruppe und durch eine durch ein Chlor-, Brom- oder Jodatom substituierte C₁₋₃-Alkylgruppe substituiert ist,
R_{b} ein Wasserstoffatom oder eine C₁₋₃-Alkylgruppe und
R_{c} eine Cyanogruppe oder eine Amidinogruppe, die durch eine Hydroxygruppe, durch eine oder zwei C₁₋₃-Alkylgruppen, durch eine oder zwei C₁₋₈-Alkoxycarbonylgruppen substituiert sein kann, bedeuten,
wobei die bei der Definition der vorstehend erwähnten Reste erwähnten Carboxy-, Amino- und Iminogruppen außerdem durch einen wie oben definierten in vivo abspaltbaren Rest substituiert sein können,
sowie deren Tautomere, deren Stereoisomere und deren Salze.

Weiter vorzugsweise werden Benzimidazole der oben gezeigten allgemeinen Formel Ia verwendet, in der
A eine C₁₋₃-Alkylengruppe,
B ein Sauerstoffatom, eine Methylen-, Imino- oder N-(C₁₋₃-Alkyl)-iminogruppe, in der der Alkylteil durch eine Carboxygruppe substituiert sein kann,
Rₐ eine in 1-Stellung durch den R₁-CO-Rest substituierte C₃₋₅-Cycloalkylgruppe, in der
   R₁ eine C₁₋₃-Alkoxy-, Amino-, C₁₋₄-Alkylamino- oder Di-(C₁₋₄-Alkyl)-aminogruppe, in denen jeweils der Alkylteil durch eine Carboxygruppe substituiert sein kann,
   eine 4- bis 7-gliedrige Cycloalkyleniminogruppe, die durch eine Hydroxygruppe oder durch eine oder zwei C₁₋₃-Alkylgruppe substituiert sein kann, wobei ein Alkylsubstituent gleichzeitig durch eine Hydroxy-, C₁₋₃-Alkoxy-, Carboxy-, Carboxy-C₁₋₃-alkoxy-, Carboxy-C₁₋₃-alkylamino-, N-(C₁₋₃-Alkyl)-N-(carboxy-C₁₋₃-alkyl)-amino-, Carboxy-C₁₋₃-alkylaminocarbonyl-, N-(C₁₋₃-Alkyl)-N-(carboxy-C₁₋₃-alkyl)-aminocarbonyl-, Carboxy-C₁₋₃-alkylaminocarbonylamino-, 1-(C₁₋₃-Alkyl)-3-(carboxy-C₁₋₃-alkyl)-aminocarbonylamino-, 3-(C₁₋₃-Alkyl)-3-(carboxy-C₁₋₃-alkyl)-aminocarbonylamino- oder 1,3-Di-(C₁₋₃-alkyl)-3-(carboxy-C₁₋₃-alkyl)-aminocarbonylaminogruppe substituiert sein kann,
   eine gegebenenfalls durch eine C₁₋₃-Alkylgruppe substituierte 5- bis 7-gliedrige Cycloalkyleniminogruppe, an die über zwei benachbarte Kohlenstoffatome ein Phenylring ankondensiert ist,
   eine Morpholino-, Piperazino-, N-(C₁₋₃-Alkyl)-piperazino-, Pyrrolino-, 3,4-Dehydropiperidino- oder Pyrrol-1-yl-Gruppe darstellt,
eine in 1-Stellung durch den R₂-CX-Rest substituierte C₃₋₅-Cycloalkylgruppe, in der
   R₂ eine gegebenenfalls durch eine C₁₋₃-Alkylgruppe substituierte Phenyl-, Naphthyl- oder monocyclische 5- oder 6-gliedrige Heteroarylgruppe, wobei die 6-gliedrige Heteroarylgruppe ein, zwei oder drei Stickstoffatome und die 5-gliedrige Heteroarylgruppe eine gegebenenfalls durch eine C₁₋₃-Alkylgruppe substituierte Iminogruppe, ein Sauerstoff- oder Schwefelatom oder eine gegebenenfalls durch eine C₁₋₃-Alkylgruppe substituierte Iminogruppe und ein Sauerstoff- oder Schwefelatom oder ein oder zwei Stickstoffatome enthält und der vorstehend erwähnte Alkylsubstituent durch eine Carboxy-, Carboxy-C₁₋₃-alkoxy-, Carboxy-C₁₋₃-alkylamino- oder N-(C₁₋₃-Alkyl)-carboxy-C₁₋₃-alkylaminogruppe substituiert sein kann, und
   X ein Sauerstoffatom, eine C₁₋₃-Alkylimino-, C₁₋₃-Alkoxyimino- oder C₁₋₃-Alkylidengruppe, die jeweils im Alkyl- oder Alkoxyteil durch eine Carboxygruppe substituiert sein können, darstellen,
eine in 1-Stellung durch eine Imidazol- oder Imidazolongruppe substituierte C₁₋₃-Alkylgruppe, in denen
   der Imidazolring durch eine Phenyl- oder Carboxygruppe und durch eine oder zwei C₁₋₃-Alkylgruppen oder durch eine, zwei oder drei C₁₋₃-Alkylgruppen substituiert sein kann, wobei die Substituenten gleich oder verschieden sein können und einer der vorstehend erwähnten Alkylsubstituenten gleichzeitig durch eine Carboxygruppe oder in 2- oder 3-Stellung durch eine Amino-, C₂₋₄-Alkanoylamino-, C₁₋₃-Alkylamino-, N-(C₂₋₄-Alkanoyl)-C₁₋₃-alkylamino- oder Di-(C₁₋₃-Alkyl)-aminogruppe substituiert sein kann, und
   der Imidazolonring durch eine C₁₋₃-Alkylgruppe substituiert sein kann, wobei der Alkylsubstituent durch eine Carboxygruppe oder in 2- oder 3-Stellung durch eine Amino-, C₂₋₄-Alkanoylamino-, C₁₋₃-Alkylamino-, N-(C₂₋₄-Alkanoyl)-C₁₋₃-alkylamino- oder Di-(C₁₋₃-Alkyl)-aminogruppe substituiert sein kann, und
   zusätzlich an die vorstehend erwähnten Imidazol- und Imidazolonringe über zwei benachbarte Kohlenstoffatome ein Phenyl- oder Pyridinring ankondensiert sein kann,
eine Imidazolidin-2,4-dion-5-yl-Gruppe, die durch eine oder zwei C₁₋₃-Alkylgruppen substituiert sein kann, wobei gleichzeitig ein Alkylsubstituent durch eine Carboxygruppe substituiert sein kann,
eine C₁₋₄-Alkylgruppe, die in 1-Stellung
   durch eine R₃NR₄- oder R₃NR₄-C₁₋₃-alkyl-Gruppe und
   durch eine Pyrrolinocarbonyl-, 2,3-Dehydro-piperidinocarbonyl-, Imidazol-1-yl-carbonyl-, Carboxy-, Aminocarbonyl-, C₁₋₃-Alkylaminocarbonyl-, Di-(C₁₋₃-Alkyl)-aminocarbonyl-, Isoxazolidin-1-ylcarbonyl- oder 4- bis 7-gliedrige Cycloalkyleniminocarbonylgruppe substituiert ist, wobei bei den vorstehend erwähnten Gruppen der Cycloalkyleniminoteil durch eine oder zwei C₁₋₃-Alkylgruppen substituiert und gleichzeitig jeweils ein Alkylteil oder Alkylsubstituent der vorstehend erwähnten C₁₋₃-Alkylaminocarbonyl-, Di-(C₁-₃-Alkyl)-anünocarbonyl- oder Cycloalkyleniminocarbonylgruppen durch eine Carboxygruppe substituiert sein kann, und die verbleibenden Wasserstoffatome der C₁₋₄Alkylgruppe ganz oder teilweise durch Fluoratome ersetzt sein können, in denen
      R₃ ein Wasserstoffatom oder eine gegebenenfalls durch eine Carboxygruppe substituierte C₁₋₃-Alkylgruppe und
      R₄ ein Wasserstoffatom, C₁₋₃-Alkyl-Y₂- oder Carboxy-C₁₋₃-alkyl-Y₂-Gruppe oder
      R₃ und R₄ zusammen mit dem dazwischen liegenden Stickstoffatom eine gegebenenfalls in 1-Stellung durch eine Carboxy-, C₁₋₃-Alkyl- oder Carboxy-C₁₋₃-alkylgruppe substituierte 4- bis 7-gliedrige Cycloalkyleniminogruppe darstellen, in denen
   Y₂ eine Kohlenstoff-Stickstoffbindung oder eine Carbonyl-, Imino- oder -NH-CO-Gruppe darstellt, wobei die Carbonylgruppe der -NH-CO-Gruppe mit dem Stickstoffatom der R₃NR₄-Gruppe verknüpft ist, und die bei der Definition des Restes Y₂ vorkommende Iminogruppe zusätzlich durch eine C₁₋₃-Alkyl- oder Carboxy-C₁₋₃-alkylgruppe substituiert sein können,
eine in 1-Stellung durch eine R₅NR₆-Gruppe substituierte C₁₋₃-Alkyl- oder C₃₋₅-Cycloalkylgruppe, in denen
   R₅ ein Wasserstoffatom, eine C₁₋₃-Alkyl-, C₅₋₇-Cycloalkyl-, Phenylcarbonyl-, Phenylsulfonyl- oder Pyridinylgruppe und
   R₆ eine C₁₋₃-Alkyl-, Carboxy-C₁₋₃-alkyl- oder Carboxy-C₁₋₃-alkylcarbonylgruppe darstellen,
eine C₁₋₃-Alkylgruppe, die durch C₂₋₄-Alkanoyl- oder C₅₋₇-Cycloalkanoylgruppe und durch eine durch ein Chlor-, Brom- oder Jodatom substituierte C₁₋₃-Alkylgruppe substituiert ist,
R_{b} eine C₁₋₃-Alkylgruppe und
R_{c} eine gegebenenfalls durch eine 2,2,2-Trichlorethoxycarbonyl-, C₁₋₈-Alkoxycarbonyl-, Acetoxymethyloxycarbonyl-, Benzyloxycarbonyl- oder Benzoylgruppe substituierte Amidinogruppe bedeuten, wobei der Benzoylteil durch Fluor-, Chlor-, Brom- oder Jodatome, durch C₁₋₃-Alkyl- oder C₁₋₃-Alkoxygruppen mono- oder disubstituiert und die Substituenten gleich oder verschieden sein können,
sowie deren C₁₋₃-Alkanolester, deren Tautomere, deren Stereoisomere und deren Salze.

In einer besonders bevorzugten Ausführungsform werden zur Herstellung des oben genannten Arzneimittels Benzimidazole der oben gezeigten allgemeinen Formel (Ia) verwendet, in der
A eine Methylengruppe,
B ein Sauerstoffatom oder eine Iminogruppe,
Rₐ eine in 1-Stellung durch den R₁-CO-Rest substituierte Cyclopropylgruppe, in der
   R₁ eine gegebenenfalls durch eine Methyl- oder Ethylgruppe substituierte Pyrrolidino- oder Piperidinogruppe, in denen jeweils der Methyl- oder Ethylteil durch eine Carboxy-, Carboxy-C₁₋₃-alkoxy-, Carboxy-C₁₋₃-alkylamino- oder N-(C₁₋₃-Alkyl)-carboxy-C₁₋₃-alkylaminogruppe substituiert sein kann, darstellt,
eine in 1-Stellung durch den R₂-CX-Rest substituierte Cyclopropylgruppe, in der
   R₂ eine gegebenenfalls durch eine C₁₋₃-Alkylgruppe substituierte Phenyl-, Pyridyl-Pyrazolylgruppe und
   X ein Sauerstoffatom, eine C₁₋₃-Alkoxyimino- oder C₁₋₃-Alkylidengruppe, die jeweils im Alkyl- oder Alkoxyteil durch eine Carboxygruppe substituiert sind, darstellen,
   eine in 1-Stellung durch eine Imidazolgruppe substituierte C₁₋₂-Alkylgruppe, in der der Imidazolring durch eine Phenyl- oder Carboxygruppe und durch eine oder zwei C₁₋₃-Alkylgruppen oder durch eine, zwei oder drei C₁₋₃-Alkylgruppen substituiert sein kann, wobei die Substituenten gleich oder verschieden sein können und einer der vorstehend erwähnten Alkylsubstituenten gleichzeitig durch eine Carboxygruppe oder in 2- oder 3-Stellung durch eine Amino-, C₂₋₄-Alkanoylamino-, C₁₋₃-Alkylamino-, N-(C₂₋₄-Alkanoyl)-C₁₋₃-alkylamino- oder Di-(C₁₋₃-Alkyl)-aminogruppe substituiert sein kann, wobei zusätzlich an die vorstehend erwähnten Imidazolringe über zwei benachbarte Kohlenstoffatome ein Phenyl- oder Pyridinring ankondensiert sein kann,
   eine in 1-Stellung durch eine Benzimidazolon-1-yl-Gruppe substituierte C₁₋₂-Alkylgruppe, wobei der Imidazolonring durch eine gegebenenfalls durch eine Carboxygruppe substituierte Methyl- oder Ethylgruppe substituiert sein kann, darstellen,
eine Methyl- oder Ethylgruppe, die in 1-Stellung
   durch eine R₃NR₄- oder R₃NR₄-C₁₋₃-alkyl-Gruppe und
   durch eine Di-(C₁₋₃-Alkyl)-aminocarbonylgruppe, durch eine Isoxazolidin-1-ylcarbonylgruppe, durch eine gegebenenfalls durch eine C₁₋₃-Alkylgruppe substituierte Pyrrolidinocarbonyl- oder Piperidinocarbonylgruppe, wobei bei den vorstehend erwähnten Gruppen jeweils ein Alkylteil oder Alkylsubstituent durch eine Carboxygruppe substituiert sein kann, in denen
      R₃ ein Wasserstoffatom oder eine gegebenenfalls durch eine Carboxygruppe substituierte C₁₋₃-Alkylgruppe und
      R₄ ein Wasserstoffatom, eine C₁₋₃-Alkyl-Y₂- oder Carboxy-C₁₋₃-alkyl-Y₂-Gruppe oder
      R₃ und R₄ zusammen mit dem dazwischen liegenden Stickstoffatom eine gegebenenfalls durch eine Carboxygruppe substituierte 4- bis 7-gliedrige Cycloalkyleniminogruppe darstellen, in denen
   Y₂ eine Kohlenstoff Stickstoffbindung, eine Carbonylgruppe oder eine gegebenenfalls durch eine C₁₋₃-Alkylgruppe substituierte Iminogruppe darstellt,
eine in 1-Stellung durch eine R₅NR₆-Gruppe substituierte C₁₋₂-Alkylgruppe, in der
   R₅ eine Pyridinyl-, Phenylcarbonyl- oder Phenylsulfonylgruppe und
   R₆ eine C₁₋₃-Alkyl- oder Carboxy-C₁₋₃-alkylgruppe darstellen,
eine durch eine in 3-Stellung durch ein Chloratom substituierte n-Propylgruppe, die in 1-Stellung durch eine Cyclopentylcarbonylgruppe substituiert ist,
eine in 1-Stellung durch eine Cyclopentylaminogruppe substituierte Cyclopropylgruppe, die am Stickstoffatom durch eine Carboxy-C₁₋₃-alkylcarbonylgruppe substituiert ist,
R_{b} eine Methylgruppe und
R_{c} eine gegebenenfalls durch eine C₁₋₈-Alkoxycarbonyl-, Acetoxymethyloxycarbonyl-, 2,2,2-Trichlorethoxycarbonyl-, Benzyloxycarbonyl- oder Benzoylgruppe substituierte Amidinogruppe bedeuten,
oder deren C₁₋₃-Alkanolester, deren Tautomere, deren Stereoisomere oder deren Salze.
Weiter vorzugsweise werden Benzimidazole der oben gezeigten allgemeinen Formel (Ia) verwendet, in der
A eine Methylengruppe,
B eine Iminogruppe,
Rₐ eine in 1-Stellung durch den R₁-CO-Rest substituierte Cyclopropylgruppe, in der
   R₁ eine gegebenenfalls durch eine Methyl- oder Ethylgruppe substituierte Pyrrolidino- oder Piperidinogruppe, in denen jeweils der Methyl- oder Ethylteil durch eine Carboxy-, Carboxy-C₁₋₃-alkoxy-, Carboxy-C₁₋₃-alkylamino- oder N-(C₁₋₃-Alkyl)-carboxy-C₁₋₃-alkylaminogruppe substituiert sein kann, darstellt,
eine in 1-Stellung durch den R₂-CX-Rest substituierte Cyclopropylgruppe, in der
   R₂ eine gegebenenfalls durch eine C₁₋₃-Alkylgruppe substituierte Phenyl-, Pyridyl-Pyrazolylgruppe und
   X ein Sauerstoffatom, eine C₁₋₃-Alkoxyimino- oder C₁₋₃-Alkylidengruppe, die jeweils im Alkyl- oder Alkoxyteil durch eine Carboxygruppe substituiert sind, darstellen,
eine in 1-Stellung durch eine Imidazolgruppe substituierte C₁₋₂-Alkylgruppe, in der der Imidazolring durch 1 bis 3 Methylgruppen substituiert sein kann oder durch zwei Methylgruppen und eine Ethylgruppe substituiert ist, wobei zusätzlich einer der vorstehend erwähnten Methyl- oder Ethylsubstituenten gleichzeitig durch eine Carboxygruppe substituiert sein kann,
eine Methyl- oder Ethylgruppe, die in 1-Stellung
   durch eine R₃NR₄- oder R₃NR₄-CH₂-Gruppe und
   durch eine Di-(C₁₋₃-Alkyl)-aminocarbonylgruppe, eine gegebenenfalls durch eine C₁₋₃-Alkylgruppe substituierte Pyrrolidinocarbonyl- oder Piperidinocarbonylgruppe, wobei bei den vorstehend erwähnten Gruppen jeweils ein Alkylteil oder Alkylsubstituent durch eine Carboxygruppe substituiert sein kann, in denen
   R₃ ein Wasserstoffatom oder eine gegebenenfalls durch eine Carboxygruppe substituierte C₁₋₃-Alkylgruppe und
   R₄ eine C₁₋₃-Alkyl-Y₂- oder Carboxy-C₁₋₃-alkyl-Y₂-Gruppe darstellen, in denen
Y₂ eine Kohlenstoff-Stickstoffbindung, eine Carbonylgruppe oder eine gegebenenfalls durch eine C₁₋₃-Alkylgruppe substituierte Iminogruppe darstellt,
R_{b} eine Methylgruppe und
R_{c} eine gegebenenfalls durch eine C₁₋₈-Alkoxycarbonyl-, Acetoxymethyloxycarbonyl-, 2,2,2-Trichlorethoxycarbonyl-, Benzyloxycarbonyl- oder Benzoylgruppe substituierte Amidinogruppe bedeuten,
oder deren C₁₋₃-Alkanolester, deren Tautomere, deren Stereoisomere oder deren Salze.
Ganz besonders bevorzugt ist die Verwendung von Benzimidazolen der oben angegebenen allgemeinen Formel (I) und den oben angegebenen Substituenten, wobei der Rest Rₐ in 5-Stellung steht, oder deren Tautomere, deren Stereoisomere oder deren Salze.

Beispiele für bevorzugte Verbindungen sind:
(a) 2-(4-Amidinophenylaminomethyl)-1-methyl-5-[1-(pyrrolidin-1-yl-carbonyl)-cyclopropyl]-benzimidazol,
(b) (E/Z)-2-(4-Amidinophenylaminomethyl)-1-methyl-5-[1-[(pyridin-2-yl)-(carboxymethyloxyimino)methylen]cyclopropyl]-benzimidazol,
(c) 2-(4-An-üdinophenylan-dnomethyl)-1-methyl-5-[1-(2-carboxy-ethylarnino)-1-(pyrrolidin-1-yl-carbonyl)-ethyl]-benzimidazol,
(d) 2-(4-Amidinophenylaminomethyl)-1-methyl-5-[1-[2-(2-carboxyethyl)-pyrrolidin-1-yl-carbonyl]cyclopropyl]-benzimidazol,
(e) 2-(4-Amidinophenylaminomethyl)-1-methyl-5-[2-(2-carboxyethyl)-4,5-dimethylimidazol-1-yl-methyl]-benzimidazol,
(f) 2-(4-Amidinophenylaminomethyl)-1-methyl-5-[1-(carboxymethylamino)-1-(pyrrolidin-1-yl-carbonyl)-ethyl]-benzimidazol,
(g) 2-(4-Amidinophenylaminomethyl)-1-methyl-5-[1-(N-methyl-carboxymethylcarbonylaminomethyl)-1-methyl-1-(pyrrolidin-1-yl-carbonyl)-ethyl]-benzimidazol,
(h) 2-(4-Amidinophenylaminomethyl)-1-methyl-5-[1-(carboxymethylamino)-1-(pyrrolidinocarbonyl)-ethyl]-benzimidazol und
(i) (R)-2-(4-Amidinophenylaminomethyl)-1-methyl-5-[1-(carboxymethylamino)-1-(pyrrolidinocarbonyl)-ethyl]-benzimidazol, gezeigt in Formel (IIa),
deren C₁₋₃-Alkanolester, deren N-(C₁₋₈-Alkoxycarbonyl)-, N-Benzyloxycarbonyl- und N-Benzoyl-amidine, deren Tautomere, deren Stereoisomere und deren Salze.

So können die genannten Benzimidazolverbindungen z.B. als freie Base, als Zwitterion oder in Form pharmazeutisch verträglicher Säureeaditionssalze eingesetzt werden. Unter pharmazeutisch verträglichen Säureadditionssalzen sind in diesem Zusammenhang Salze der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure und Maleinsäure zu verstehen, wobei die Salze der Salzsäure, Bromwasserstoffsäure, Schwefelsäure, Phosphorsäure und Essigsäure besonders bevorzugt sind. Besonders bevorzugt sind hierbei Salze der Salzsäure, z.B. das Monohydrochlorid oder das Dihydrochlorid.

Gemäß einer besonders bevorzugten Ausführungsform wird bei der Behandlung bzw. bei der Herstellung eines Arzneimittels zur Behandlung der o.g. Erkrankungen das oben genannte (R)-2-(4-Amidinophenylaminomethyl)-1-methyl-5-[1-(carboxymethylamino)-1-(pyrrolidinocarbonyl)-ethyl]-benzimidazol als freie Base, gezeigt in Formel (IIa) 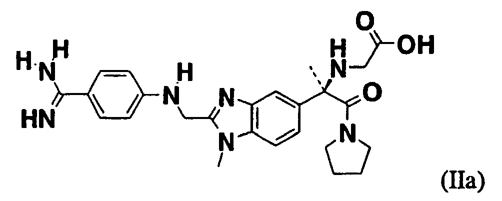 oder als Monohydrochlorid, gezeigt in Formel (II) 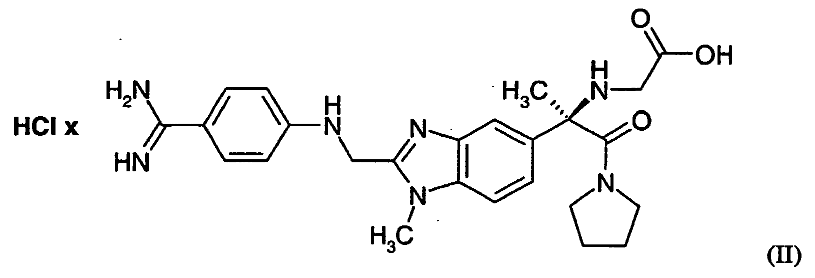 verwendet. In wässriger Lösung, so etwa bei der Formulierung als Infusionslösung, wird diese Verbindung typischerweise als Zwitterion vorliegen.

Wie vorstehend erwähnt, ist die antithrombotische Wirkung der oben genannten Verbindungen z.B. aus der WO 00/01704 bekannt. Überraschenderweise wurde nun gefunden, dass sich die oben genannten Verbindungen und insbesondere Verbindungen der Formel (II) oder (IIa) zur Behandlung der Sepsis oder von Bakteriämien und, allgemeiner, des SIRS, des akuten Herz-Kreislaufversagens, des Organversagens nach Reanimation, des akuten Lungenversagens und des ARDS eignen. Aus Untersuchungen zu anderen, im Stand der Technik bekannten antithrombotischen Wirkstoffen war nämlich bekannt, dass eine generelle Wirksamkeit derartiger Wirkstoffe in der Indikation Sepsis nicht gegeben ist (vgl. z.B. die Kybersept-Studie: Warren et al., JAMA 2001; Knaub S, Keinecke HO, Juers M, Schindel F, Heinrichs H, Opal S: "High-dose antithrombin III in patients with severe sepsis - The kybersept trial" Thromb. Haemost. (2001), 6-12 July 2001 (Abs P523)), also eine Besonderheit der oben genannten Verbindungen und insbesondere des (R)-2-(4-Amidinophenylanvnomethyl)-1-methyl-5-[1-(carboxymethylamino)-1-(pyrrolidinocarbonyl)-ethyl]-benzimidazols darstellt.

Wie einleitend angesprochen, lässt sich das SIRS in mehrere Untergruppen einteilen. Eine bedeutende Untergruppe stellt das durch Infektionen verursachte SIRS dar, das dem Krankheitsbild der Sepsis (Septikämie; 'Blutvergiftung') entspricht. Eine weitere Untergruppe wären SIRS, die unabhängig von Infektionen auftreten.

Von besonderer klinischer Bedeutung ist unter den nicht-infektiösen Ursachen insbesondere das Herz-Kreislaufversagen, da es in einer großen Zahl von Patienten auftritt (geschätzte Zahl in den USA bis zu 450.000 Todesfälle) und mit einer Überlebensrate von unter 5% ein äußerst gefährliches Krankheitsbild darstellt (Weisfeldt ML, JAMA 2002; 288: 3035). Auch bei primärem Überleben des Herz-Kreislauf-Stillstandes ist die Prognose der Patienten sehr ernst, wobei das fortbestehende Organversagen bei diesen Patienten zu einer geringen Überlebensrate auch nach primär erfolgreicher Wiederbelebung beiträgt. Dieses Organversagen wird durch die Kombination aus Aktivierung des Gerinnungssystems während des Herz-Kreislauf-Stillstandes und der nachfolgenden Immunreaktion unterhalten (zu Parametern der Immunreaktion s. Adrie C, Circulation 2002; 106: 562). Aufgrund der Verbindung von Gerinnungsaktivierung und Immunreaktion ist gerade die gefundene Kombination aus Wirksamkeit der erfindungsgemäßen Verbindungen der Formel I und speziell der Verbindungen der Formeln II und IIa als Antikoagulanz und die gleichzeitige Reduktion der Immunparameter bei Patienten nach Herz-Kreislauf-Stillstand wichtig. Dabei können die erfindungsgemäßen Verbindungen der Formel I verabreicht werden als intravenöse Gabe (Bolus oder Infusion) während der Durchführung der Reanimationsmaßnahmen und erhöht hierdurch die Erfolgswahrscheinlichkeit der Wiederherstellung der Herz-Kreislauffunktion oder als intravenöse Gabe (Bolus oder Infusion) nach wiederhergestellter Herz-Kreislauffunktion zur Vermeidung und Reduktion des Versagens verschiedener Organe. Die Gabe kann bis zur völligen Genesung des Patienten erfolgen. Im weiteren Verlauf eines SIRS kann es zu (weicher) Milz- und Lebervergrößerung sowie Schädigungen an inneren Organen (Niere, Lunge, Herz) kommen. Durch die Gabe der erfindungsgemäßen Verbindungen der Formel I, wobei die Gabe der Verbindungen der Formeln II und IIa besonders bevorzugt ist, bei Patienten mit Risiken der Ausbildung eines SIRS kann die Ausbildung von Organversagen verhindert werden oder bei Gabe unter einem bereits ablaufenden SIRS die Ausbildung von weiteren Organschäden bzw. deren Dauer verkürzt werden.
Als Formen des Organversagens sind aufzuführen: das Versagen der Herzfunktion, das bei Patienten zum kritischen Abfall des Blutdruckes mit weiteren Organversagen und Todesfolge führt; das Nierenversagen, das unbehandelt zum Tod führen kann und als Behandlungsmaßnahme den Einsatz von Nierenersatztherapieformen (z. B. Dialyse oder kontinuierliche Filtration) notwendig macht, und das akute Lungenversagen. Das Organversagen der Lunge, als akutes Lungenversagen (engl: ALI, acute lung injury) oder akutes Atemnotsyndrom des Erwachsenen (engl.: ARDS, acute respiratory distress syndrome) ist von besonderer Bedeutung, da das SIRS durch die Kombination aus aktivierter Entzündungsreaktion und aktivierter Gerinnung sehr häufig zu dieser Folge führt. Die Verwendung der erfindungsgemäßen Verbindungen der Formel I, wobei die Verbindungen der Formeln II und IIa besonders bevorzugt sind, führt zur Prävention des akuten Lungenversagens bei Patienten mit noch ohne Zeichen des Lungenversagens oder Frühformen (z. B. Patienten mit entsprechenden Krankheitsbildern [Sepsis, Trauma] und noch ohne Veränderungen im Röntgenbild oder beginnenden Lungeninfiltraten) oder zur Verringerung der Beatmungsdauer bei Patienten mit voll ausgebildetem Lungenversagen. Die Gabe kann bei den Krankheitsbildern intravenös als Bolus oder Infusion beginnen, sobald eine Risikosituation gefunden wird oder als Therapie bei bereits bestehenden Zeichen der Organdysfunktion (z. B. deutlich reduzierter Blutdruck, ansteigende Nierenretentionswerte oder Verschlechterung der Blutgaswerte). Die Gabe wird fortgesetzt, solange eine Risikosituation besteht. (Literatur: Abraham E, Crit Care Med 2000; 28: 232).

Demgemäß stellt die Erfindung ein Verfahren zur Herstellung eines Arzneimittels zur Behandlung oder Vorbeugung oder Begleitbehandlung von Erkrankungen dar, die unter den Oberbegriff "SIRS" zu subsumieren sind, wie z.B.: SIRS verursacht durch Krankheitserreger wie gramnegative Erreger (wie z.B. Escherichia coli, Klebsiella, Proteus, Enterobacter, Pseudomonas aeruginosa, Neisseria meningitidis, Salmonella, Serratia, Bacteroides etc.), grampositive Erreger (wie z.B. Staphylokokken, Streptokokken, Pneumokokken, Enterokokken und Clostridium perfringens), Viren, einzellige eukaryontische Parasiten oder Pilze, SIRS mit und ohne Organversagen, septischer Schock, septisches Syndrom, SIRS verursacht durch Pankreatitis, durch systemische Ischämien, durch organbegrenzte Ischämien, durch Verbrennungen, durch Gewebeverletzungen oder durch sonstige Traumata, SIRS auftretend im Zusammenhang mit Tumorerkrankungen, SIRS auftretend in der Folge ausgedehnter Operationen, als Folge einer Organtransplantation oder in Folge von Schock verschiedener Ursachen, so z.B. auch in Folge eines Blutungsschocks, in Folge eines Herz-Kreislaufversagens, eines immunvermittelten Organversagens oder von Entzündungsreaktionen, sowie SIRS, das in Folge der Behandlung mit Entzündungsmediatoren wie z.B. dem Tumor Necrose Faktor alpha und/oder dem Tumor Necrose Faktor beta und/oder anderer Cytokinen auftritt, weiterhin das akute Lungenverasgen und ARDS. Als Begleiterscheinung von SIRS können Lungenschäden, Schäden des Herz-Kreislaufsystems mit Hypotonie, Nierenversagen, hämatologische Veränderungen, Azidose sowie das "multiple organ dysfunction syndrome" (MODS) auftreten, zu deren Behandlung die genannten Verbindungen demzufolge ebenfalls gemäß der Erfindung eingesetzt werden können.

Gemäß einer bevorzugten Ausführungsform betrifft die Erfindung die Verwendung von (R)-2-(4-Amidinophenylaminomethyl)-1-methyl-5-[1-(carboxymethylamino)-1-(pyrrolidinocarbonyl)-ethyl]-benzimidazol und insbesondere dessen Monohydrochlorids zur Herstellung eines Arzneimittels zur Behandlung oder Begleitbehandlung von Sepsis, infektionsbedingtem SIRS und/oder SIRS in Folge von Bakteriämien, von akutem Herz-Kreislaufversagen, von Organversagen nach reanimation, von akutem Lungenversagen und ARDS.

Neben der Verwendung als Monotherapeutikum können die oben genannten Benzimidazolverbindungen und insbesondere das (R)-2-(4-Amidinophenylanvnomethyl)-1-methyl-5-[1-(carboxymethylamino)-1-(pyrrolidinocarbonyl)-ethyl]-benzimidazol gemäß einer weiteren bevorzugten Ausführungsform in Kombination mit geeigneten weiteren Wirkstoffen eingesetzt werden.

In Betracht kommen hierbei z.B.:
(a) Hemmer der Plättchenfunktion wie etwa Acetylsalicylsäure, Fibrinogen-Rezeptorantagonisten (z.B. Abciximab, Eptifibatide, Tirofiban, Roxifiban), Inhibitoren der ADP-induzierten Aggregation (z.B. Clopidogrel, Ticlopidin), P₂T-Rezeptorantagonisten (z.B. Cangrelor) und kombinierte Thromboxan Rezeptorantagonisten / Synthetaseinhibitoren (z.B. Terbogrel),
(b) thrombolytisch wirksame Substanzen, wie zum Beispiel Alteplase, Reteplase, Tenecteplase, Urokinase, Staphylokinase und Streptokinase,
(c) physiologische Aktivatoren und Inhibitoren des Gerinnungssystems und deren rekombinante Analoga (z. B. Protein C, rekombinantes humanes aktiviertes Protein C (rhAPC), tissue factor pathway inhibitor (TFPI), Antithrombin),
(d) herkömmliche bei Sepsis eingesetzte Wirkstoffe wie z.B. Substanzen mit antagonistischer Wirkung gegen Endotoxine, Interleukine, TNF, Bradykinin, Prostaglandine, Cyclooxygenasen, NO, PAF wie etwa solche, die an den jeweiligen Rezeptoren antagonistisch wirken, solche, die mit dem jeweiligen Wirkprinzip interferieren, sowie spezifisch gegen die Substanzen gerichtete Antikörper, etc.,
(e) Platelet activating factor acetylhydrolase (PAF-AH), vorzugsweise humane PAF-AH (beschrieben z.B. in Tjoelker LW, Stafforini DM: "Platelet-activating factor acetylhydrolases in health and disease" Biochim Biophys Acta (2000) 1488:102-123, WO 95/09921 und WO 95/00649) sowie PAF-AH-Derivate wie insbesondere verkürzte PAF-AH-Proteine, wie z.B. beschrieben in WO 99/09147,
(f) herkömmliche Therapeutika, die zur Entzündungshemmung und Immunsuppression eingesetzt werden, wie z.B. Basistherapeutika rheumatischer Erkrankungen wie Chloroquin, Goldpräparate, D-Penicillamin, Methothrexat, Chlorambucil, Cyclophosphamid, Kortikosteroide in allen Formen, Ciclosporin, Tacrolimus, Sirolimus, Azathioprin, Mycophenolatmofetil, etc., und
(g) Mittel, die herkömmlicherweise im Rahmen einer Sepsistherapie eingesetzt werden, wie etwa Antibiotika, kreislaufwirksame Substanzen wie Katecholamine, etc.

Die Erfindung umfasst demgemäß auch Kombinationen der oben genannten Benzimidazolverbindungen und insbesondere des (R)-2-(4-Amidinophenylaminomethyl)-1-methyl-5-[1-(carboxymethylamino)-1-(pyrrolidinocarbonyl)-ethyl]-benzimidazols, gegebenenfalls in Form der pharmazeutisch verträglichen Säureadditionssalze sowie gegebenenfalls in Form der Hydrate oder Solvate, mit einer oder mehreren der oben unter (a) bis (g) angeführten Substanzen. Besonders vorteilhafte Wirkungen sind für die Kombination von (R)-2-(4-Amidinophenylaminomethyl)-1-methyl-5-[1-(carboxymethylamino)-1-(pyrrolidinocarbonyl)-ethyl]-benzimidazol mit einer PAF-Acetylhydrolase und insbesondere einem der in der WO 99/09147 beschriebenen PAF-AH-Derivate, wie z.B. dem darin genannten rPH.2 oder rPH.9 (Seite 10 ff. der WO 99/09147) gegeben. Ebenfalls besonders vorteilhaft ist die Kombination von (R)-2-(4-Amidinophenylaminomethyl)-1-methyl-5-[1-(carboxymethylamino)-1-(pyrrolidinocarbonyl)-ethyl]-benzimidazol mit einem anti-humanTNFalpha-Antikörper, wie z.B. Afelimomab (INN).

Die Applikation der genannten Benzimidazol-Wirkstoffe bzw. Wirkstoffkombinationen erfolgt in üblicher Weise, vorzugsweise parenteral und weiter vorzugsweise intravenös (i.v.), gegebenenfalls auch subkutan. Eine parenterale Applikation kann beispielsweise durch i.v. Infusionen erfolgen, die je nach Krankheitsbild unter Umständen auch über einen längeren Zeitraum (Stunden oder Tage) zur Anwendung gelangen können (kontinuierliche Dauerinfusion). Dosierungen für die i.v.-Anwendung können z.B. im Bereich von 0,05 bis 2000 mg/24 h liegen. Die optimale therapeutische Dosis ist von der Indikation und Darreichungsform abhängig und kann in dem Fachmann bekannter Weise experimentell bestimmt werden. Als Mengenbereich für die Dosierung z.B. des Wirkstoffs (R)-2-(4-Amidinophenylaminomethyl)-1-methyl-5-[1-(carboxymethylamino)-1-(pyrrolidinocarbonyl)-ethyl]-benzimidazol wird der Bereich von 0,0001 mg/kg/h bis 1 mg/kg/h, vorzugsweise von 0,001 mg/kg/h bis 0,5 mg/kg/h und weiter vorzugsweise von 0,01 mg/kg/h bis 0,3 mg/kg/h vorgeschlagen. Für den Fachmann ist natürlich ersichtlich, dass es gegebenenfalls erforderlich sein kann, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelapplikationen über den Tag zu verteilen.

Dem Fachmann sind Verfahren bekannt, wie er die genannten Benzimidazol-Wirkstoffe und Wirkstoffkombinationen für bestimmte Anwendungsweisen formulieren kann (Gennaro, Alfonso R.: Remington's Pharmaceutical Sciences. Easton Mack). Injektions- und Infusionslösungen werden in üblicher Weise, z.B. unter Zusatz von Puffern, Isotonantien, Konservierungsmitteln, wie p-Hydroxybenzoaten, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösemittel als Lösevermittler bzw. Hilflösemittel eingesetzt werden können, hergestellt und in Injektionsflaschen oder Ampullen oder Infusionsflaschen abgefüllt.

Die nachfolgenden Beispiele illustrieren die Erfindung.

### Beispiele

### Beispiel 1: Herstellung von (R)-2-(4-Amidinophenylaminomethyl)-1-methyl-5-[1-(carboxymethylamino)-1-(pyrrolidinocarbonyl)-ethyl]-benzimidazol-hydrochlorid

### a.) 2-Amino-2-(4-chlor-3-nitro-phenyl)-propionsäureethylester

Eine Mischung von 28 g (0.11 Mol) 2-Amino-2-(4-chlor-3-nitro-phenyl)-propionsäure in 200 ml 5.6N ethanolischer Salzsäure wird 36 Stunden unter Rückfluß erhitzt. Nach Abdampfen des Lösungsmittels wird der Rückstand in 300 ml Essigester aufgeschlämmt und mit 300 ml gesättigter Natriumhydrogencarbonatlösung versetzt. Die organische Phase wird zweimal mit gesättigter Natriumhydrogencarbonatlösung und einmal mit Wasser gewaschen, über Natriumsulfat getrocknet und eingedampft.
Ausbeute: 21.1 g (68% der Theorie) hellbraunes Öl.

### b.) (R)-(+)-2-Amino-2-(4-chlor-3-nitro-phenyl)-propinsäureethylester

17.33 g (63.6 mMol) 2-Amino-2-(4-chlor-3-nitro-phenyl)-propionsäureethylester werden in 247 ml Isopropanol und 207 ml Methanol gelöst und mit 9.54 g (63.6 mMol) L-(+)-Weinsäure versetzt. Das Reaktionsgemisch wird auf 100°C erhitzt, wobei eine klare Lösung entsteht. Die Lösung wird innerhalb von 3 Stunden auf 27°C abgekühlt, der gebildete Niederschlag wird abgesaugt, mit Ethanol gewaschen und getrocknet. Anschließend wird der gebildete Feststoff (21.5 g) in 400 ml Essigester aufgeschlämmt und mit 400 ml gesättigter Natriumhydrogencarbonatlösung versetzt. Nach Extraktion und Phasentrennung wird die organische Phase mit Wasser gewaschen, getrocknet und eingedampft.
Ausbeute: 7.68 g (44.4% der Theorie) hellgelben Öl,
[α]²⁰ = + 4.38° (Essigester)
HPLC-Analyse: ee-Wert >98.6%

### c.) (R)-(-)-2-Amino-2-(4-chlor-3-nitro-phenyl)-propionsäure

150 mg (R)-(+)-2-Amino-2-(4-chlor-3-nitro-phenyl)-propionsäureethylester und 2.5 ml 2N Natronlauge werden in 10 ml Tetrahydrofuran 5 Stunden bei Raumtemperatur gerührt. Das Tetrahydrofuran wird abdestilliert und der Rückstand mit Salzsäure auf pH 5 eingestellt. Das kristalline Produkt wird abgesaugt, mit Wasser gewaschen und getrocknet.
Ausbeute: 63% der Theorie,
[α]²⁰= -59.6° (Methanol/Wasser 1:1)

### d.) (R)-2-tert.Butyloxycarbonylamino-2-(4-chlor-3-nitro-phenyl)-propionsäure

5.7 g (R)-(-)-2-Amino-2-(4-chlor-3-nitro-phenyl)-propionsäure werden in 50 ml Dioxan gelöst und nach Zugabe von 5.5 ml (39.1 mMol) Triethylamin und 4.8 g Pyrokohlensäure-di-tert.butyldicarbonat 18 Stunden bei Raumtemperatur gerührt. Anschließend wird mit 0.5 M Kaliumhydrogensulfatlösung verdünnt und mit Essigester extrahiert. Die vereinigten organischen Extrakte werden getrocknet und eingedampft.
Ausbeute: 100% der Theorie.

### e.) (R)-2-tert.Butyloxycarbonylamino-2-(4-methylamino-3-nitro-phenyl)-propionsäure

20.0 g (R)-2-tert.Butyloxycarbonylamino-2-(4-chlor-3-nitro-phenyl)-propionsäure und 100 ml Methylaminlösung (40%ig in H₂O) werden in einem Druckgefäß fünf Stunden auf 80°C erhitzt. Der Inhalt wird zur Trockene eingedampft, in Wasser gelöst und mit Eisessig angesäuert. Das ausgefallene Produkt wird abgesaugt, mit Wasser gewaschen und getrocknet.
Ausbeute: 69% der Theorie.

### f.) (R)-2-(4-Methylamino-3-nitro-phenyl)-2-tert.butyloxycarbonylamino-1-pyrrolidino-propanon

12.3 g (R)-2-tert.Butyloxycarbonylamino-2-(4-methylamino-3-nitro-phenyl)-propionsäure (64.8 mMol) werden in 90 ml Tetrahydrofuran gelöst und mit 12.6 g (78 mMol) Carbonyldiimidazol versetzt. Nach 30 Minuten bei Raumtemperatur werden 10.9 ml (130 mMol) Pyrrolidin zugegeben. Nach weiteren 12 Stunden bei Raumtemperatur wird die Reaktionslösung mit 800 ml Wasser versetzt. Der gebildete Feststoff wird abfiltriert, mit Wasser gewaschen und getrocknet. Ausbeute: 48% der Theorie.

### g.) (R)-2-(4-Methylamino-3-amino-phenyl)-2-tert.butyloxycarbonylamino-1-pyrrolidino-propanon

3.1 g (R)-2-(4-Methylamino-3-nitro-phenyl)-2-tert.butyloxycarbonylamino-1-pyrrolidino-propanon (7.9 mMol) werden in 30 ml Methanol gelöst und unter Zugabe von Wasserstoff/10% Palladium auf Aktivkohle 2 Stunden bei Raumtemperatur hydriert. Der Katalysator wird abfiltriert und das Lösungsmittel wird abdestilliert. Der Rückstand wird in 50 ml Methyl-tert.butylether bei 45°C gelöst. Der nach 12 Stunden bei 5°C gebildete Feststoff wird abgesaugt und getrocknet.
Ausbeute: 87% der Theorie.

### h.) (R)-2-(4-Cyanophenylaminomethyl)-1-methyl-5-[1-(N-tert.butyloxycarbonylamino)-1-(pyrrolidinocarbonyl)-ethyl]-benzimidazol

11.2 g (63 mMol) 4-Cyanophenylglycin und 10.95 g (67.5 mMol) Carbonyldiimidazol werden in 320 ml Tetrahydrofuran bei Raumtemperatur 2 Stunden gerührt. Nach Zugabe von 23 g (60 mMol) (R)-2-(4-Methylamino-3-amino-phenyl)-2-tert.butyloxycarbonylamino-1-pyrrolidino-propanon wird das Reaktionsgemisch 2 Stunden unter Rückfluß erhitzt. Das Solvens wird abdestilliert, der Rückstand in 320 ml Eisessig aufgenommen und 1 Stunde unter Rückfluß erhitzt. Der nach Zugabe von 500 ml Eiswasser gebildete Feststoff wird abfiltriert, gewaschen und getrocknet. Ausbeute: 96% der Theorie.

### i.) (R)-2-(4-Cyanophenylaminomethyl)-1-methyl-5-[1-amino-1-(pyrrolidinocarbonyl)-ethyl]-benzimidazol

1.3 g (R)-2-(4-Cyanophenylaminomethyl)-1-methyl-5-[1-(N-tert.butyloxycarbonylamino)-1-(pyrrolidinocarbonyl)-ethyl]-benzimidazol werden in 20 ml Dioxan gelöst und nach Zugabe von 6N Salzsäure zwei Stunden bei Raumtemperatur gerührt. Die Lösung wird mit Eis versetzt, mit Ammoniak alkalisch gestellt und mit Essigester extrahiert. Die vereinigten organischen Extrakte werden getrocknet und eingedampft.
Ausbeute: 76% der Theorie.

### k.) (R)-2-(4-Cyanophenylaminomethyl)-1-methyl-5-[1-(ethoxycarbonylmethylamino)-1-(pyrrolidinocarbonyl)-ethyl]-benzimidazol

1.2 g (R)-2-(4-Cyanophenylaminomethyl)-1-methyl-5-[1-amino-1-(pyrrolidino-carbonyl)-ethyl]-benzimidazol werden in 20 ml Aceton gelöst und nach Zugabe von 0.39 ml Jodessigsäureethylester und 0.56 g Kaliumcarbonat 3Stunden unter Rückfluß erhitzt. Das Reaktionsgemisch wird filtriert und eingedampft, der Rückstand an Kieselgel chromatographiert, wobei mit Methylenchlorid/Ethanol (20:1 und 4:1) eluiert wird. Ausbeute: 75% der Theorie.

### l.) (R)-2-(4-Amidinophenylaminomethyl)-1-methyl-5-[1-(ethoxycarbonylmethylamino)-1-(pyrrolidinocarbonyl)-ethyl]-benzimidazol-hydrochlorid

1.0 g (R)-2-(4-Cyanophenylaminomethyl)-1-methyl-5-[1-(ethoxycarbonylmethylamino)-1-(pyrrolidinocarbonyl)-ethyl]-benzimidazol werden in 50 ml gesättigter ethanolischer Salzsäure gelöst und 5 Stunden bei Raumtemperatur gerührt. Das Solvens wird abdestilliert, der Rückstand in 50 ml absolutem Ethanol gelöst und mit 2.3 g (25 mMol) Ammoniumcarbonat versetzt. Nach 60 Stunden bei Raumtemperatur wird zur Trockne eingedampft. Der Rückstand wird an Kieselgel chromatographiert, wobei mit Methylenchlorid/Methanol (7:1) eluiert wird.
Ausbeute: 95% der Theorie.

### m.) (R)-2-(4-Amidinophenylaminomethyl)-1-methyl-5-[1-(carboxymethylamino)-1-(pyrrolidinocarbonyl)-ethyl]-benzimidazol-hydrochlorid

150 mg (R)-2-(4-Amidinophenylaminomethyl)-1-methyl-5-[1-(ethoxycarbonylmethylamino)-1-(pyrrolidinocarbonyl)-ethyl]-benzimidazol und 2.5 ml 2N Natronlauge werden in 10 ml Ethanol 5 Stunden bei Raumtemperatur gerührt. Der Alkohol wird abdestilliert und der Rückstand mit Salzsäure auf pH 5 eingestellt. Das kristalline Produkt wird abgesaugt, mit Wasser gewaschen und getrocknet.
Ausbeute: 100% der Theorie,
C₂₅H₃₁N₇O₃ x 2HCl(477.57/550.5)
Massenspektrum: (M+H)⁺ = 478
(M-H+HCl)⁻ = 512/514 (Cl)
(M-H+2HCl)⁻ = 448/550/552 (Cl₂)

### Beispiel 2: Tiermodell zum "systemic inflammatory response syndrome" (SIRS): Intravenöse Lipopolysaccharid-Stimulation (modifiziert nach Isobe et al. Circulation 2001: 104: 1171-1175)

### Methode 1

Ratten (männl., ca. 300 g CrlGlxBrlHan:Wi) wurden mit Pentobarbital (60 mg/kg i.p.) anästhesiert. Zur Aufrechterhaltung der Narkose wurde eine Dauerinfusion Pentobarbital (22.5 mg/kg/h i.p.) angelegt. Die Arteria carotis wurde zur Blutentnahme, die Vena jugularis links zur Substanzapplikation und die Vena jugularis rechts zur LPS-Applikation kanüliert. Die Applikation von (R)-2-(4-Amidinophenylaminomethyl)-1-methyl-5-[1-(carboxymethylamino)-1-(pyrrolidinocarbonyl)-ethyl]-benzimidazol (0.1 mg/kg/h) wurde zum Zeitpunkt t = - 60 min als Dauerinfusion gestartet und bis zum Ende des Experimentes aufrechterhalten. Eine Stunde nach Beginn der Substanzinfusion erfolgte eine einmalige Bolus-Applikation von LPS (Lipopolysaccharid von E. coli Serotyp 0127:B8 Sigma L-3129 5 mg/kg i.v. Bolus) zum Zeitpunkt t = 0 min. Zur Bestimmung der Koagulations- und Inflammationsparameter wurde Blut über die arterielle Kanüle zu den Zeitpunkten a) vor Infusion von Placebo oder (R)-2-(4-Amidinophenylaminomethyl)-1-methyl-5-[1-(carboxymethylamino)-1-(pyrrolidinocarbonyl)-ethyl]-benzimidazol (- 60 Minuten), b) vor Applikation des LPS-Bolus (0 Minuten) und c) 240 Minuten nach Applikation von LPS entnommen. Aus den Blutproben wurden als Parameter einer systemischen inflammatorischen Reaktion mittels ELISA der TAT-Komplex (Thrombin-Antithrombin) und Interleukin-6 (IL-6) bestimmt.

### Ergebnis

Wie aus Tabelle 1 ersichtlich ist, trat unter Kontrollbedingungen über den Beobachtungszeitraum keine Änderung der gemessenen Parameter auf. Stimulation mit LPS induzierte hingegen eine systemische inflammatorische Reaktion, wie anhand des deutlichen Anstiegs der Plasmaspiegel des TAT-Komplexes um das 8,6-fache und des IL-6 um das ca. 400-fache am Ende des Versuchszeitraums ersichtlich ist (Tabelle 2). Wie der Tabelle 3 zu entnehmen ist, inhibierte die Behandlung mit (R)-2-(4-Amidinophenylaminomethyl)-1-methyl-5-[1-(carboxymethylamino)-1-(pyrrolidinocarbonyl)-ethyl]-benzimidazol die Aktivierung der Koagulationskaskade: Die Plasmaspiegel des TAT-Komplexes waren gegenüber den unbehandelten LPS-Tieren signifikant niedriger (p<0.02) und lagen im Kontrollbereich. Überraschenderweise resultierte die Behandlung mit (R)-2-(4-Amidinophenylaminomethyl)-1-methyl-5-[1-(carboxymethylamino)-1-(pyrrolidinocarbonyl)-ethyl]-benzimidazol zudem in einer signifikanten (p < 0.02) Verringerung der Plasmaspiegel von IL-6 (Tabelle 3).

**Tabelle 1:**

| **Kontrollen ohne LPS** | | | |
|---|---|---|---|
| **Parameter** | **- 60 min** | **0 min** | **+ 240 min** |
| TAT (ng/ml) | 4.163 +/- 1.45 | 6.45 +/- 2.78 | 5.71 +/- 1.48 |
| IL-6 (ng/ml) | 0.053 +/- 0.011 | 0.065 +/- 0.019 | 0.064 +/- 0.018 |

**Tabelle 2:**

| **Kontrollen mit LPS** | | | |
|---|---|---|---|
| **Parameter** | **- 60 min** | **0 min** | **+ 240 min** |
| TAT (ng/ml) | 1.43 +/- 0.40 | 4.13 +/- 0.89 | 35.69 +/- 5.54 |
| IL-6 (ng/ml) | 0.117 +/- 0.070 | 0.158 +/- 0.067 | 62.884 +/- 5.438 |

**Tabelle 3:**

| **LPS + Wirkstoff (100 µg/kg/h)** | | | | |
|---|---|---|---|---|
| **Parameter** | **- 60 min** | **0 min** | **+ 240 min** | **p value vs LPS** |
| TAT (ng/ml) | 2.33 +/- 0.89 | 2.21 +/- 0.79 | 6.52 +/- 1.33 * | 0,021 |
| IL-6 (ng/ml) | 0.139 +/- 0.086 | 0.143 +/- 0.083 | 43.454 +/- 2.975 * | 0,022 |

| | | | | |
|---|---|---|---|---|
| Wirkstoff = (R)-2-(4-Amidinophenylaminomethyl)-1-methyl-5-[1-(carboxymethylamino)-1-(pyrrolidinocarbonyl)-ethyl]-benzimidazol (Base) | | | | |

### Beispiel 3: Tiermodell zum "systemic inflammatory response syndrome" (SIRS): Intravenöse Lipopolysaccharid-Stimulation (modifiziert nach Aoki et al. Drug Res. 50: 809: 2000 und Yamazaki et al. Blood Coag. Fibrinol. 10: 321; 1999)

### Methode 2

Ratten (männl., ca. 300 g CrlGlxBrlHan:Wi) wurden mit Pentobarbital (60 mg/kg i.p.) anästhesiert. Zur Aufrechterhaltung der Narkose wurde eine Dauerinfusion Pentobarbital (22.5 mg/kg/h i.p.) angelegt. Die Vena jugularis links wurde zur Substanzapplikation und die Vena jugularis rechts zur LPS-Applikation kanüliert. LPS wurde als Dauerinfusion über 4 Stunden (Lipopolysaccharid von E. coli Serotyp 0127:B8 Sigma L-3129 7.5 mg/kg/h) gegeben. Die Applikation von (R)-2-(4-Amidinophenylaminomethyl)-1-methyl-5-[1-(carboxymethylamino)-1-(pyrrolidinocarbonyl)-ethyl]-benzimidazol erfolgte als Dauerinfusion (100 µg/kg/h) eine Stunde nach Beginn der LPS-Infusion mit einer Gesamtdauer von 3 Stunden. Zur Bestimmung der Inflammationsparameter wurde Blut nach 4 Stunden Gesamtversuchsdauer über die Arteria abdominalis entnommen. Aus den Blutproben wurden als Parameter einer systemischen inflammatorischen Reaktion mittels ELISA der TAT-Komplex (Thrombin-Antithrombin), Interleukin-1β (IL-1β), Interleukin-6 (IL-6) und der Tumor-Nekrose-Faktor-alpha (TNF-alpha) bestimmt. Als Enzymparameter für Organschäden der Leber und Niere dienten Alanin-aminotransferase (ALT) und Kreatinin.

**Tabelle 4:**

| | **Kontrollen** | **LPS-behandelt** | **LPS + BIBT 986** |
|---|---|---|---|
| | **(Phys. NaCl)** | **7.5 mg/kg/h x 4 h** | **100 µg/kg/h** |
| **IL-1β** | 17.1 ± 8.9* | 8947 ± 1501§ | 4036 ± 1000*§ |
| **[pg/ml]** | (10) | (8) | (8) |
| **IL-6** | 31.6 ± 8.4* | 209904 ± 30076§ | 89579 ± 10069*§ |
| **[pg/ml]** | (10) | (7) | (8) |
| **TNF-alpha** | 3.2 ± 2.4* | 4241 ± 1045§ | 1003 ± 292* |
| **[pg/ml]** | (10) | (8) | (8) |
| | | | |
| **ALT** | 36.4 ± 7.7* | 206.9 ± 82.8§ | 44.2 ± 7.1* |
| **[U/ml]** | (10) | (7) | (8) |
| **Kreatinin** | 31.1 ± 3.0* | 57 ± 4.1 § | 40.1 ± 1.8* |
| **[µmol/L]** | (10) | (8) | (8) |

| | | | |
|---|---|---|---|
| Mittelwerte ± Standardfehler; Anzahl der Tiere in Klammern. * p<0.05 vs. LPS behandelten Tieren; § p<0.05 vs. Kontrollen (Tukey Kramer Test) | | | |

### Ergebnis

Wie in Tabelle 4 gezeigt ist, induziert die kontinuierliche Infusion von LPS einen siginifikanten Anstieg der Indikatoren eines systemischen inflammatorischen Prozesses (TAT-Komplex, IL-1β, IL-6 und TNF alpha) und von Markern für Organschäden (ALT und Kreatinin). Die Behandlung mit (R)-2-(4-Amidinophenylaminomethyl)-1-methyl-5-[1-(carboxymethylamino)-1-(pyrrolidinocarbonyl)-ethyl]-benzimidazol führte überraschenderweise zu einer signifikanten Senkung des TAT Komplexes und zu einer signifikanten (p < 0.02) Verringerung.der Plasmaspiegel von IL-1β, IL-6 und TNF alpha. ALT und Kreatinin waren unter Behandlung mit (R)-2-(4-Amidinophenylaminomethyl)-1-methyl-S-[1-(carboxymethylamino)-1-(pyrrolidinocarbonyl)-ethyl]-benzimidazol ebenfalls nicht signifikant verschieden von unbehandelten Kontrolltieren.

Die gezeigten Ergebnisse lassen den Schluß zu, dass die Behandlung mit dem kombinierten Faktor Xa/Thrombin Hemmer (R)-2-(4-Amidinophenylaminomethyl)-1-methyl-5-[1-(carboxymethylamino)-1-(pyrrolidinocarbonyl)-ethyl]-benzimidazol ein wirksames Therapeutikum für die Behandlung einer systemischen inflammatorischen Reaktion / SIRS und insbesondere der Lipopolysaccharid-induzierten systemischen inflammatorischen Reaktion und der Sepsis (auch der "severe sepsis") darstellt.

### Beispiel 4: Pharmazeutische Formulierungen

### a.) Trockenampulle mit 75 mg Wirkstoff pro 10 ml

### Zusammensetzung:

| | |
|---|---|
| Wirkstoff | 75,0 mg |
| Mannitol | 50,0 mg |
| Wasser für Injektionszwecke | ad 10,0 ml |

### Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

### b.) Trockenampulle mit 35 mg Wirkstoff pro 2 ml

### Zusammensetzung:

| | |
|---|---|
| Wirkstoff | 35,0 mg |
| Mannitol | 100,0 mg |
| Wasser für Injektionszwecke | ad 2,0 ml |

### Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

### c.) Ampullenlösung

| | |
|---|---|
| Wirkstoff | 2 mg |
| Natriumchlorid | 9 mg |
| Aqua pro inj. | 5 ml |

### Beispiel 5: Kombination von (R)-2-(4-Amidinophenylaminomethyl)-1-methyl-5-[1-(carboxymethylamino)-1-(pyrrolidinocarbonyl)-ethyl]-benzimidazol mit einer PAF-AH

Eine Trockenampulle gemäß Beispiel 3a) oder 3b) enthaltend (R)-2-(4-Amidinophenylaminomethyl)-1-methyl-5-[1-(carboxymethylamino)-1-(pyrrolidinocarbonyl)-ethyl]-benzimidazolmonohydrochlorid als Wirkstoff wird mit einem Vial enthaltend ein PAF-AH-Lyophilisat in einer gemeinsamen Umverpackung kombiniert. Zur Herstellung einer Infusionslösung werden die Trockensubstanzen mit Wasser für Injektionszwecke gelöst und dem Patienten gemeinsam oder sequentiell per Infusion verabreicht.

## Patentansprüche

1. Verwendung von Benzimidazolen der allgemeinen Formel (I) 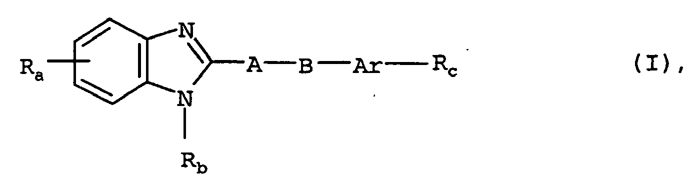 in der
Ar eine gegebenenfalls durch ein Fluor-, Chlor- oder Bromatom, durch eine Trifluormethyl-, C₁₋₃-Alkyl- oder C₁₋₃-Alkoxygruppe substituierte Phenylen- oder Naphthylengruppe,
eine gegebenenfalls im Kohlenstoffgerüst durch eine C₁₋₃-Alkylgruppe substituierte Thienylen-, Thiazolylen-, Pyridinylen-, Pyrimidinylen-, Pyrazinylen- oder Pyridazinylengruppe,
A eine C₁₋₃-Alkylengruppe,
B ein Sauerstoff- oder Schwefelatom, eine Methylen-, Carbonyl-, Sulfinyl- oder Sulfonylgruppe, eine gegebenenfalls durch eine C₁₋₃-Alkylgruppe substituierte Iminogruppe, in der der Alkylteil durch eine Carboxygruppe mono- oder disubstituiert sein kann,
Rₐ eine R₁-CO-C₃₋₅-cycloalkylgruppe, in der
R₁ eine C₁₋₃-Alkoxy-, Amino-, C₁₋₄-Alkylamino- oder Di-(C₁₋₄-Alkyl)-aminogruppe, in denen jeweils der Alkylteil durch eine Carboxygruppe substituiert sein kann,
eine 4- bis 7-gliedrige Cycloalkylenimino- oder Cycloalkenyleniminogruppe, die durch eine oder zwei C₁₋₃-Alkylgruppen substituiert sein können, wobei ein Alkylsubstituent gleichzeitig durch eine Hydroxy-, C₁₋₃-Alkoxy-, Carboxy-, Carboxy-C₁₋₃-alkoxy-, Carboxy-C₁₋₃-alkylamino-, N-(C₁₋₃-Alkyl)-N-(carboxy-C₁₋₃-alkyl)-amino-, Carboxy-C₁₋₃-alkylaminocarbonyl-, N-(C₁₋₃-Alkyl)-N-(carboxy-C₁₋₃-alkyl)-aminocarbonyl-, Carboxy-C₁₋₃-alkylaminocarbonylamino-, 1-(C₁₋₃-Alkyl)-3-(carboxy-C₁₋₃-alkyl)-aminocarbonylamino-, 3-(C₁₋₃-Alkyl)-3-(carboxy-C₁₋₃-alkyl)-aminocarbonylamino- oder 1,3-Di-(C₁₋₃-alkyl)-3-(carboxy-C₁₋₃-alkyl)-aminocarbonylaminogruppe substituiert sein kann,
eine durch eine Hydroxygruppe substituierte 4- bis 7-gliedrige Cycloalkyleniminogruppe,
eine gegebenenfalls durch eine C₁₋₃-Alkylgruppe substituierte 5- bis 7-gliedrige Cycloalkyleniminogruppe, an die über zwei benachbarte Kohlenstoffatome ein Phenylring ankondensiert ist,
eine Morpholino-, Piperazino-, N-(C₁₋₃-Alkyl)-piperazino-, Pyrrolino-, 3,4-Dehydro-piperidino- oder Pyrrol-1-yl-Gruppe darstellt,
eine R₂-CX-C₃₋₅-cycloalkylgruppe, in der
R₂ eine gegebenenfalls durch eine C₁₋₃-Alkylgruppe substituierte Phenyl-, Naphthyl- oder monocyclische 5- oder 6-gliedrige Heteroarylgruppe, wobei die 6-gliedrige Heteroarylgruppe ein, zwei oder drei Stickstoffatome und die 5-gliedrige Heteroarylgruppe eine gegebenenfalls durch eine C₁₋₃-Alkylgruppe substituierte Iminogruppe, ein Sauerstoff- oder Schwefelatom oder eine gegebenenfalls durch eine C₁₋₃-Alkylgruppe substituierte Iminogruppe und ein Sauerstoff- oder Schwefelatom oder ein oder zwei Stickstoffatome enthält und der vorstehend erwähnte Alkylsubstituent durch eine Carboxy-, Carboxy-C₁₋₃-alkoxy-, Carboxy-C₁₋₃-alkylamino- oder N-(C₁₋₃-Alkyl)-carboxy-C₁₋₃-alkylaminogruppe substituiert sein kann, und
X ein Sauerstoffatom, eine C₁₋₃-Alkylimino-, C₁₋₃-Alkoxyimino-, C₁₋₃-Alkylhydrazino-, Di-(C₁₋₃-Alkyl)-hydrazino-, C₂₋₄-Alkanoylhydrazino-, N-(C₁₋₃-Alkyl)-C₂₋₄-alkanoylhydrazino- oder C₁₋₃-Alkylidengruppe, die jeweils im Alkyl- oder Alkanoylteil oder im Alkyl- und Alkanoylteil durch eine Carboxygruppe substituiert sein können, darstellen,
eine durch eine Imidazol- oder Imidazolongruppe substituierte C₁₋₃-Alkyl- oder C₃₋₅-Cycloalkylgruppe, in denen
der Imidazolring durch eine Phenyl- oder Carboxygruppe und durch eine oder zwei C₁₋₃-Alkylgruppen oder durch eine, zwei oder drei C₁₋₃-Alkylgruppen substituiert sein kann, wobei die Substituenten gleich oder verschieden sein können und einer der vorstehend erwähnten Alkylsubstituenten gleichzeitig durch eine Carboxygruppe oder in 2- oder 3-Stellung durch eine Amino-, C₂₋₄-Alkanoylamino-, C₁₋₃-Alkylamino-, N-(C₂₋₄-Alkanoyl)-C₁₋₃-alkylamino- oder Di-(C₁₋₃-Alkyl)-aminogruppe substituiert sein kann, und
der Imidazolonring durch eine C₁₋₃-Alkylgruppe substituiert sein kann, wobei der Alkylsubstituent durch eine Carboxygruppe oder in 2- oder 3-Stellung durch eine Amino-, C₂₋₄-Alkanoylamino-, C₁₋₃-Alkylamino-, N-(C₂₋₄-Alkanoyl)-C₁₋₃-alkylamino- oder Di-(C₁₋₃-Alkyl)-aminogruppe substituiert sein kann, und
zusätzlich an die vorstehend erwähnten Imidazol- und Imidazolonringe über zwei benachbarte Kohlenstoffatome ein Phenyl- oder Pyridinring ankondensiert sein kann,
eine Imidazolidin-2,4-dion-5-yl-Gruppe, die durch eine oder zwei C₁₋₃-Alkylgruppen substituiert sein kann, wobei gleichzeitig ein Alkylsubstituent durch eine Carboxygruppe substituiert sein kann,
eine C₁₋₄-Alkylgruppe, die
durch eine C₁₋₃-Alkyl-Y₁-C₁₋₃-alkyl-, HOOC-C₁₋₃-alkyl-Y₁-C₁₋₃-alkyl-, Tetrazolyl-C₁₋₃-alkyl-Y₂-, R₃NR₄- oder R₃NR₄-C₁₋₃-alkyl-Gruppe und
durch eine gegebenenfalls durch eine C₁₋₃-Alkylgruppe substituierte Isoxazolidinylcarbonylgruppe, durch eine Pyrrolinocarbonyl-, 3,4-Dehydro-piperidinocarbonyl-, Pyrrol-1-yl-carbonyl-, Carboxy-, Aminocarbonyl-, C₁₋₃-Alkylaminocarbonyl-, Di-(C₁₋₃-Alkyl)-aminocarbonyl- oder 4- bis 7-gliedrige Cycloalkyleniminocarbonylgruppe substituiert ist, wobei bei den vorstehend erwähnten Gruppen der Cycloalkyleniminoteil durch eine oder zwei C₁₋₃-Alkylgruppen substituiert und gleichzeitig jeweils ein Alkylteil oder Alkylsubstituent der vorstehend erwähnten C₁₋₃-Alkylaminocarbonyl-, Di-(C₁₋₃-Alkyl)-aminocarbonyl- oder Cycloalkyleniminocarbonylgruppen durch eine Carboxygruppe substituiert sein kann, und die verbleibenden Wasserstoffatome der C₁₋₄-Alkylgruppe ganz oder teilweise durch Fluoratome ersetzt sein können, in denen
R₃ ein Wasserstoffatom oder eine gegebenenfalls durch eine Carboxygruppe substituierte C₁₋₃-Alkylgruppe und
R₄ ein Wasserstoffatom, eine C₁₋₃-Alkyl-Y₁-C₁₋₃-alkyl-Y₂-, Carboxy-C₁₋₃-alkyl-Y₁-C₁₋₃-alkyl-Y₂-, C₁₋₃-Alkyl-Y₂- oder Carboxy-C₁₋₃-alkyl-Y₂-Gruppe oder
R₃ und R₄ zusammen mit dem dazwischen liegenden Stickstoffatom eine gegebenenfalls durch eine Carboxy-, C₁₋₃-Alkyl- oder Carboxy-C₁₋₃-alkylgruppe substituierte 4- bis 7-gliedrige Cycloalkyleniminogruppe darstellen, in denen
Y₁ eine Kohlenstoff-Kohlenstoff-Bindung, ein Sauerstoffatom, eine Sulfenyl-, Sulfinyl-, Sulfonyl-, -NH-, -NH-CO- oder -NH-CO-NH-Gruppe und
Y₂ eine Kohlenstoff Stickstoffbindung oder eine Carbonyl-, Sulfonyl-, Imino- oder -NH-CO-Gruppe darstellen, wobei die Carbonylgruppe der -NH-CO-Gruppe mit dem Stickstoffatom der R₃NR₄-Gruppe verknüpft ist, und die bei der Definition der Reste Y₁ und Y₂ vorkommenden Iminogruppen jeweils zusätzlich durch eine C₁₋₃-Alkyl- oder Carboxy-C₁₋₃-alkylgruppe substituiert sein können,
eine durch eine R₅NR₆-Gruppe substituierte C₁₋₃-Alkyl- oder C₃₋₅-Cycloalkylgruppe, in denen
R₅ ein Wasserstoffatom, eine C₁₋₃-Alkyl-, C₅₋₇-Cycloalkyl-, Phenylcarbonyl-, Phenylsulfonyl- oder Pyridinylgruppe und
R₆ eine C₁₋₃-Alkyl-, Carboxy-C₁₋₃-alkyl- oder Carboxy-C₁₋₃-alkylcarbonylgruppe darstellen,
eine C₁₋₃-Alkylgruppe, die durch C₂₋₄-Alkanoyl- oder C₅₋₇-Cycloalkanoylgruppe und durch eine durch ein Chlor-, Brom- oder Jodatom substituierte C₁₋₃-Alkylgruppe substituiert ist,
R_{b} ein Wasserstoffatom oder eine C₁₋₃-Alkylgruppe und
R_{c} eine Cyanogruppe oder eine gegebenenfalls durch eine oder zwei C₁₋₃-Alkylgruppen substituierte Amidinogruppe bedeuten, worin
die bei der Definition der vorstehend erwähnten Reste erwähnten Carboxygruppen außerdem durch eine in-vivo in eine Carboxygruppe überführbare Gruppe oder durch eine unter physiologischen Bedingungen negativ geladene Gruppe ersetzt sein können oder
die bei der Definition der vorstehend erwähnten Reste erwähnten Amino- und Iminogruppen außerdem durch einen in vivo abspaltbaren Rest substituiert sein können, wobei
unter einer in-vivo in eine Carboxygruppe überführbaren Gruppe eine Hydroxymethylgruppe, eine mit einem Alkohol veresterte Carboxygruppe, in der der alkoholische Teil ein C₁₋₆-Alkanol, ein Phenyl-C₁₋₃-alkanol, ein C₃₋₉-Cycloalkanol, wobei ein C₅₋₈-Cycloalkanol zusätzlich durch ein oder zwei C₁₋₃-Alkylgruppen substituiert sein kann, ein C₅₋₈-Cycloalkanol, in dem eine Methylengruppe in 3- oder 4-Stellung durch ein Sauerstoffatom oder durch eine gegebenenfalls durch eine C₁₋₃-Alkyl-, Phenyl-C₁₋₃-alkyl-, Phenyl-C₁₋₃-alkoxycarbonyl- oder C₂₋₆-Alkanoylgruppe substituierte Iminogruppe ersetzt ist und der Cycloalkanolteil zusätzlich durch ein oder zwei C₁₋₃-Alkylgruppen substituiert sein kann, ein C₄₋₇-Cycloalkenol, ein C₃₋₅-Alkenol, ein Phenyl-C₃₋₅-alkenol, ein C₃₋₅-Alkinol oder Phenyl- C₃₋₅-alkinol mit der Maßgabe, dass keine Bindung an das Sauerstoffatom von einem Kohlenstoffatom ausgeht, welches eine Doppel- oder Dreifachbindung trägt, ein C₃₋₈-Cycloalkyl-C₁₋₃-alkanol, ein Bicycloalkanol mit insgesamt 8 bis 10 Kohlenstoffatomen, das im Bicycloalkylteil zusätzlich durch eine oder zwei C₁₋₃-Alkylgruppen substituiert sein kann, ein 1,3-Dihydro-3-oxo-1-isobenzfuranol oder ein Alkohol der Formel
R_{d}-CO-O-(RₑCR_{f})-OH,
verstanden wird, in dem
R_{d} eine C₁₋₈-Alkyl-, C₅₋₇-Cycloalkyl-, Phenyl- oder Phenyl- C₁₋₃-alkylgruppe
Rₑ ein Wasserstoffatom, eine C₁₋₃-Alkyl-, C₅₋₇-Cycloalkyl- oder Phenylgruppe und
R_{f} ein Wasserstoffatom oder eine C₁₋₃-Alkylgruppe darstellen,
unter einer unter physiologischen Bedingungen negativ geladenen Gruppe eine Tetrazol-5-yl-, Phenylcarbonylaminocarbonyl-, Trifluormethylcarbonylaminocarbonyl-, C₁₋₆-Alkylsulfonylamino-, Phenylsulfonylamino-, Benzylsulfonylamino-, Trifluormethylsulfonylamino-, C₁₋₆-Alkylsulfonylaminocarbonyl-, Phenylsulfonylaminocarbonyl-, Benzylsulfonylaminocarbonyl- oder Perfluor-C₁₋₆-alkylsulfonylaminocarbonylgruppe
und unter einem von einer Imino- oder Aminogruppe in-vivo abspaltbaren Rest eine Hydroxygruppe, eine gegebenenfalls durch Fluor-, Chlor-, Brom- oder Jodatome, durch C₁₋₃-Alkyl- oder C₁₋₃-Alkoxygruppen mono- oder disubstituierte Benzoylgruppe, wobei die Substituenten gleich oder verschieden sein können, eine Pyridinoylgruppe oder eine C₁₋₁₆-Alkanoylgruppe, eine 3,3,3-Trichlorpropionyl- oder Allyloxycarbonylgruppe, eine C₁₋₁₆-Alkoxycarbonyl- oder C₁₋₁₆-Alkylcarbonyloxygruppe, in denen Wasserstoffatome ganz oder teilweise durch Fluor- oder Chloratome ersetzt sein können, eine Phenyl-C₁₋₆-alkoxycarbonylgruppe, eine 3-Amino-propionylgruppe, in der die Aminogruppe durch C₁₋₆-Alkyl- oder C₃₋₇-Cycloalkylgruppen mono- oder disubstituiert und die Substituenten gleich oder verschieden sein können, eine C₁₋₃-Alkylsulfonyl-C₂₋₄-alkoxycarbonyl-, C₁₋₃-Alkoxy-C₂₋₄-alkoxy-C₂₋₄-alkoxycarbonyl-, R_{d}-CO-O-(R_{d}CR_{f})-O-CO-, C₁₋₆-Alkyl-CO-NH-(R_{g}CRₕ)-O-CO- oder C₁₋₆-Alkyl-CO-O-(R_{g}CRₕ)-(R_{g}CRₕ)-O-CO-Gruppe, in denen R_{d} bis R_{f} wie vorstehend erwähnt definiert sind und
R_{g} und Rₕ, die gleich oder verschieden sein können, Wasserstoffatome oder C₁₋₃-Alkylgruppen darstellen,
verstanden werden,
deren Tautomere, deren Stereoisomere, deren Gemische und deren Salze
gegebenenfalls in Form der pharmazeutisch verträglichen Säureadditionssalze sowie gegebenenfalls in Form der Hydrate oder Solvate zur Herstellung eines Arzneimittels zur Vorbeugung oder Behandlung von Erkrankungen ausgewählt aus der Gruppe bestehend aus dem "systemic inflammatory response syndrome" (SIRS), der Sepsis und der Bakteriämie.

2. Verwendung nach Anspruch 1, wobei als Benzimidazol das (R)-2-(4-Amidinophenylaminomethyl)-1-methyl-5-[1-(carboxymethylamino)-1-(pyrrolidinocarbonyl)-ethyl]-benzimidazol, gegebenenfalls in Form der pharmazeutisch verträglichen Säureadditionssalze sowie gegebenenfalls in Form der Hydrate oder Solvate eingesetzt wird.

3. Verwendung nach Anspruch 2, wobei das Monohydrochloridsalz des (R)-2-(4-Amidinophenylaminomethyl)-1-methyl-5-[1-(carboxymethylamino)-1-(pyrrolidinocarbonyl)-ethyl]-benzimidazol eingesetzt wird.

4. Verwendung nach einem der Ansprüche 1 bis 3, wobei die Erkrankung ausgewählt ist aus der Gruppe bestehend aus durch gram-positive Erreger verursachtem SIRS, durch gram-negative Erreger verursachtem SIRS, durch Viren verursachtem SIRS, durch einzellige eukaryontische Parasiten verursachtem SIRS, durch Pilze verursachtem SIRS, Sepsis ohne Organversagen, Sepsis mit Organversagen, septischem Schock, dem septischen Syndrom, SIRS verursacht durch Pankreatitis, SIRS verursacht durch systemische Ischämien, SIRS verursacht durch organbegrenzte Ischämien, SIRS verursacht durch Traumata, SIRS im Zusammenhang mit Tumorerkrankungen, SIRS verursacht durch Gewebeverletzungen, SIRS verursacht durch Verbrennungen, SIRS in Folge ausgedehnter Operationen, SIRS in Folge einer Organtransplantation, SIRS in Folge von Schock verschiedener Ursachen, SIRS in Folge eines Blutungsschocks, SIRS in Folge eines Herz-Kreislaufversagens, SIRS in Folge immunvermitteltem Organversagens, SIRS in Folge von Entzündungsreaktionen, SIRS in Folge der Behandlung mit Entzündungsmediatoren wie z.B. Tumor Necrose Faktoren alpha und/oder beta und/oder anderer Cytokine und weiter bestehend aus im Zusammenhang mit SIRS auftretenden Lungenschäden, akutem Lungenversagen und ARDS (acute respiratory distress syndrome), akutem Herz-Kreislaufversagen, Organversagen nach nach Reanimation, Schock, Nierenversagen, Herz-Kreislauf-Versagen, hämatologischen Schäden, Azidose und "multiple organ dysfunction syndrome" (MODS).

5. Verwendung nach einem der Ansprüche 1 bis 4, wobei das Arzneimittel zur Begleitbehandlung bei Bakteriämien vorgesehen ist.

6. Verwendung nach einem der Ansprüche 1 bis 5, wobei das Arzneimittel zur subkutanen oder zur parenteralen und insbesondere zur intravenösen Anwendung vorgesehen ist.

7. Verwendung nach einem der Ansprüche 1 bis 6, wobei zusätzlich zu einer der in den Ansprüchen 1 und 2 genannten Substanzen eine pharmakologisch wirksame Substanz ausgewählt aus der Gruppe bestehend aus Hemmern der Plättchenfunktion wie insbesondere Acetylsalicylsäure, Fibrinogen-Rezeptorantagonisten, Inhibitoren der ADP-induzierten Aggregation, P₂T-Rezeptorantagonisten und kombinierten Thromboxan Rezeptorantagonisten / Synthetaseinhibitoren, weiter bestehend aus thrombolytisch wirksamen Substanzen, wie insbesondere Alteplase, Reteplase, Tenecteplase, Urokinase, Staphylokinase und Streptokinase, weiter bestehend aus physiologischen Aktivatoren und Inhibitoren des Gerinnungssystems und deren rekombinanten Analoga wie insbesondere Protein C, rekombinantem humanen aktivierten Protein C, TFPI und Antithrombin, weiter bestehend aus Substanzen mit antagonistischer Wirkung gegen Endotoxine, sowie weiter bestehend aus Interleukinen, TNF, Bradykinin, Prostaglandinen, Cyclooxygenasen, NO, platelet activating factor Acetylhydrolasen, Entzündungshemmern, immunsupprimierenden Substanzen, Antibiotika und Katecholaminen eingesetzt wird.

8. Pharmazeutische Zusammensetzung enthaltend
(a) mindestens einen Wirkstoff ausgewählt aus der Gruppe von Benzimidazolen der allgemeinen Formel (I) 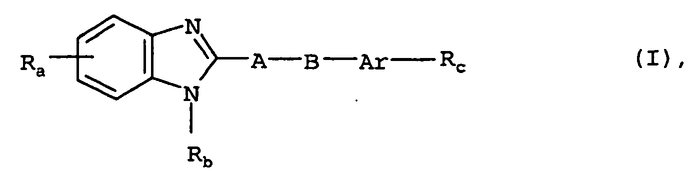 in der
Ar eine gegebenenfalls durch ein Fluor-, Chlor- oder Bromatom, durch eine Trifluormethyl-, C₁₋₃-Alkyl- oder C₁₋₃-Alkoxygruppe substituierte Phenylen- oder Naphthylengruppe,
eine gegebenenfalls im Kohlenstoffgerüst durch eine C₁₋₃-Alkyl-gruppe substituierte Thienylen-, Thiazolylen-, Pyridinylen-, Pyrimidinylen-, Pyrazinylen- oder Pyridazinylengruppe,
A eine C₁₋₃-Alkylengruppe,
B ein Sauerstoff- oder Schwefelatom, eine Methylen-, Carbonyl-, Sulfinyl- oder Sulfonylgruppe, eine gegebenenfalls durch eine C₁₋₃-Alkylgruppe substituierte Iminogruppe, in der der Alkylteil durch eine Carboxygruppe mono- oder disubstituiert sein kann,
Rₐ eine R₁-CO-C₃₋₅-cycloalkylgruppe, in der
R₁ eine C₁₋₃-Alkoxy-, Amino-, C₁₋₄-Alkylamino- oder Di-(C₁₋₄-Alkyl)-aminogruppe, in denen jeweils der Alkylteil durch eine Carboxygruppe substituiert sein kann,
eine 4- bis 7-gliedrige Cycloalkylenimino- oder Cycloalkenyleniminogruppe, die durch eine oder zwei C₁₋₃-Alkylgruppen substituiert sein können, wobei ein Alkylsubstituent gleichzeitig durch eine Hydroxy-, C₁₋₃-Alkoxy-, Carboxy-, Carboxy-C₁₋₃-alkoxy-, Carboxy-C₁₋₃-alkylamino-, N-(C₁₋₃-Alkyl)-N-(carboxy-C₁₋₃-alkyl)-amino-, Carboxy-C₁₋₃-alkylaminocarbonyl-, N-(C₁₋₃-Alkyl)-N-(carboxy-C₁₋₃-alkyl)-aminocarbonyl-, Carboxy-C₁₋₃-alkylaminocarbonylamino-, 1-(C₁₋₃-Alkyl)-3-(carboxy-C₁₋₃-alkyl)-aminocarbonylamino-, 3-(C₁₋₃-Alkyl)-3-(carboxy-C₁₋₃-alkyl)-aminocarbonylamino- oder 1,3-Di-(C₁₋₃-alkyl)-3-(carboxy-C₁₋₃-alkyl)-aminocarbonylaminogruppe substituiert sein kann,
eine durch eine Hydroxygruppe substituierte 4- bis 7-gliedrige Cycloalkyleniminogruppe,
eine gegebenenfalls durch eine C₁₋₃-Alkylgruppe substituierte 5- bis 7-gliedrige Cycloalkyleniminogruppe, an die über zwei benachbarte Kohlenstoffatome ein Phenylring ankondensiert ist,
eine Morpholino-, Piperazino-, N-(C₁₋₃-Alkyl)-piperazino-, Pyrrolino-, 3,4-Dehydro-piperidino- oder Pyrrol-1-yl-Gruppe darstellt,
eine R₂-CX-C₃₋₅-cycloalkylgruppe, in der
R₂ eine gegebenenfalls durch eine C₁₋₃-Alkylgruppe substituierte Phenyl-, Naphthyl- oder monocyclische 5- oder 6-gliedrige Heteroarylgruppe, wobei die 6-gliedrige Heteroarylgruppe ein, zwei oder drei Stickstoffatome und die 5-gliedrige Heteroarylgruppe eine gegebenenfalls durch eine C₁₋₃-Alkylgruppe substituierte Iminogruppe, ein Sauerstoff- oder Schwefelatom oder eine gegebenenfalls durch eine C₁₋₃-Alkylgruppe substituierte Iminogruppe und ein Sauerstoff- oder Schwefelatom oder ein oder zwei Stickstoffatome enthält und der vorstehend erwähnte Alkylsubstituent durch eine Carboxy-, Carboxy-C₁₋₃-alkoxy-, Carboxy-C₁₋₃-alkylamino- oder N-(C₁₋₃-Alkyl)-carboxy-C₁₋₃-alkylaminogruppe substituiert sein kann, und
X ein Sauerstoffatom, eine C₁₋₃-Alkylimino-, C₁₋₃-Alkoxyimino-, C₁₋₃-Alkyl-hydrazino-, Di-(C₁₋₃-Alkyl)-hydrazino-, C₂₋₄-Alkanoylhydrazino-, N-(C₁₋₃-Alkyl)-C₂₋₄-alkanoylhydrazino- oder C₁₋₃-Alkylidengruppe, die jeweils im Alkyl- oder Alkanoylteil oder im Alkyl- und Alkanoylteil durch eine Carboxygruppe substituiert sein können, darstellen,
eine durch eine Imidazol- oder Imidazolongruppe substituierte C₁₋₃-Alkyl- oder C₃₋₅-Cycloalkylgruppe, in denen
der Imidazolring durch eine Phenyl- oder Carboxygruppe und durch eine oder zwei C₁₋₃-Alkylgruppen oder durch eine, zwei oder drei C₁₋₃-Alkylgruppen substituiert sein kann, wobei die Substituenten gleich oder verschieden sein können und einer der vorstehend erwähnten Alkylsubstituenten gleichzeitig durch eine Carboxygruppe oder in 2- oder 3-Stellung durch eine Amino-, C₂₋₄-Alkanoylamino-, C₁₋₃-Alkylamino-, N-(C₂₋₄-Alkanoyl)-C₁₋₃-alkylamino- oder Di-(C₁₋₃-Alkyl)-aminogruppe substituiert sein kann, und
der Imidazolonring durch eine C₁₋₃-Alkylgruppe substituiert sein kann, wobei der Alkylsubstituent durch eine Carboxygruppe oder in 2- oder 3-Stellung durch eine Amino-, C₂₋₄-Alkanoylamino-, C₁₋₃-Alkylamino-, N-(C₂₋₄-Alkanoyl)-C₁₋₃-alkylamino- oder Di-(C₁₋₃-Alkyl)-aminogruppe substituiert sein kann, und
zusätzlich an die vorstehend erwähnten Imidazol- und Imidazolonringe über zwei benachbarte Kohlenstoffatome ein Phenyl- oder Pyridinring ankondensiert sein kann,
eine Imidazolidin-2,4-dion-5-yl-Gruppe, die durch eine oder zwei C₁₋₃-Alkylgruppen substituiert sein kann, wobei gleichzeitig ein Alkylsubstituent durch eine Carboxygruppe substituiert sein kann,
eine C₁₋₄-Alkylgruppe, die
durch eine C₁₋₃-Alkyl-Y₁-C₁₋₃-alkyl-, HOOC-C₁₋₃-alkyl-Y₁-C₁₋₃-alkyl-, Tetrazolyl-C₁₋₃-alkyl-Y₂-, R₃NR₄- oder R₃NR₄-C₁₋₃-alkyl-Gruppe und
durch eine gegebenenfalls durch eine C₁₋₃-Alkylgruppe substituierte Isoxazolidinylcarbonylgruppe, durch eine Pyrrolinocarbonyl-, 3,4-Dehydro-piperidinocarbonyl-, Pyrrol-1-yl-carbonyl-, Carboxy-, Aminocarbonyl-, C₁₋₃-Alkylaminocarbonyl-, Di-(C₁₋₃-Alkyl)-aminocarbonyl- oder 4- bis 7-gliedrige Cycloalkyleniminocarbonylgruppe substituiert ist, wobei bei den vorstehend erwähnten Gruppen der Cycloalkyleniminoteil durch eine oder zwei C₁₋₃-Alkylgruppen substituiert und gleichzeitig jeweils ein Alkylteil oder Alkylsubstituent der vorstehend erwähnten C₁₋₃-Alkylaininocarbonyl-, Di-(C₁₋₃-Alkyl)-aminocarbonyl- oder Cycloalkyleniminocarbonylgruppen durch eine Carboxygruppe substituiert sein kann, und die verbleibenden Wasserstoffatome der C₁₋₄-Alkylgruppe ganz oder teilweise durch Fluoratome ersetzt sein können, in denen
R₃ ein Wasserstoffatom oder eine gegebenenfalls durch eine Carboxygruppe substituierte C₁₋₃-Alkylgruppe und
R₄ ein Wasserstoffatom, eine C₁₋₃-Alkyl-Y₁-C₁-₃-alkyl-Y₂-, Carboxy-C₁₋₃-alkyl-Y₁-C₁₋₃-alkyl-Y₂-, C₁₋₃-Alkyl-Y₂- oder Carboxy-C₁₋₃-alkyl-Y₂-Gruppe oder
R₃ und R₄ zusammen mit dem dazwischen liegenden Stickstoffatom eine gegebenenfalls durch eine Carboxy-, C₁₋₃-Alkyl- oder Carboxy-C₁₋₃-alkylgruppe substituierte 4- bis 7-gliedrige Cycloalkyleniminogruppe darstellen, in denen
Y₁ eine Kohlenstoff-Kohlenstoff-Bindung, ein Sauerstoffatom, eine Sulfenyl-, Sulfinyl-, Sulfonyl-, -NH-, -NH-CO- oder -NH-CO-NH-Gruppe und
Y₂ eine Kohlenstoff-Stickstoffbindung oder eine Carbonyl-, Sulfonyl-, Imino- oder -NH-CO-Gruppe darstellen, wobei die Carbonylgruppe der -NH-CO-Gruppe mit dem Stickstoffatom der R₃NR₄-Gruppe verknüpft ist, und die bei der Definition der Reste Y₁ und Y₂ vorkommenden Iminogruppen jeweils zusätzlich durch eine C₁₋₃-Alkyl- oder Carboxy-C₁₋₃-alkylgruppe substituiert sein können,
eine durch eine R₅NR₆-Gruppe substituierte C₁₋₃-Alkyl- oder C₃₋₅-Cycloalkylgruppe, in denen
R₅ ein Wasserstoffatom, eine C₁₋₃-Alkyl-, C₅₋₇-Cycloalkyl-, Phenylcarbonyl-, Phenylsulfonyl- oder Pyridinylgruppe und
R₆ eine C₁₋₃-Alkyl-, Carboxy-C₁₋₃-alkyl- oder Carboxy-C₁₋₃-alkylcarbonylgruppe darstellen,
eine C₁₋₃-Alkylgruppe, die durch C₂₋₄-Alkanoyl- oder C₅₋₇-Cycloalkanoylgruppe und durch eine durch ein Chlor-, Brom- oder Jodatom substituierte C₁₋₃-Alkylgruppe substituiert ist,
R_{b} ein Wasserstoffatom oder eine C₁₋₃-Alkylgruppe und
R_{c} eine Cyanogruppe oder eine gegebenenfalls durch eine oder zwei C₁₋₃-Alkylgruppen substituierte Amidinogruppe bedeuten, worin
die bei der Definition der vorstehend erwähnten Reste erwähnten Carboxygruppen außerdem durch eine in-vivo in eine Carboxygruppe überführbare Gruppe oder durch eine unter physiologischen Bedingungen negativ geladene Gruppe ersetzt sein können oder
die bei der Definition der vorstehend erwähnten Reste erwähnten Amino- und Iminogruppen außerdem durch einen in vivo abspaltbaren Rest substituiert sein können, wobei
unter einer in-vivo in eine Carboxygruppe überführbaren Gruppe eine Hydroxymethylgruppe, eine mit einem Alkohol veresterte Carboxygruppe, in der der alkoholische Teil ein C₁₋₆-Alkanol, ein Phenyl-C₁₋₃-alkanol, ein C₃₋₉-Cycloalkanol, wobei ein C₅₋₈-Cycloalkanol zusätzlich durch ein oder zwei C₁₋₃-Alkylgruppen substituiert sein kann, ein C₅₋₈-Cycloalkanol, in dem eine Methylengruppe in 3- oder 4-Stellung durch ein Sauerstoffatom oder durch eine gegebenenfalls durch eine C₁₋₃-Alkyl-, Phenyl-C₁₋₃-alkyl-, Phenyl-C₁₋₃-alkoxycarbonyl- oder C₂₋₆-Alkanoylgruppe substituierte Iminogruppe ersetzt ist und der Cycloalkanolteil zusätzlich durch ein oder zwei C₁₋₃-Alkylgruppen substituiert sein kann, ein C₄₋₇-Cycloalkenol, ein C₃₋₅-Alkenol, ein Phenyl-C₃₋₅-alkenol, ein C₃₋₅-Alkinol oder Phenyl- C₃₋₅-alkinol mit der Maßgabe, dass keine Bindung an das Sauerstoffatom von einem Kohlenstoffatom ausgeht, welches eine Doppel- oder Dreifachbindung trägt, ein C₃₋₈-Cycloalkyl-C₁₋₃-alkanol, ein Bicycloalkanol mit insgesamt 8 bis 10 Kohlenstoffatomen, das im Bicycloalkylteil zusätzlich durch eine oder zwei C₁₋₃-Alkylgruppen substituiert sein kann, ein 1,3-Dihydro-3-oxo-1-isobenzfuranol oder ein Alkohol der Formel
R_{d}-CO-O-(RₑCR_{f})-OH,
verstanden wird, in dem
R_{d} eine C₁₋₈-Alkyl-, C₅₋₇-Cycloalkyl-, Phenyl- oder Phenyl- C₁₋₃-alkylgruppe
Rₑ ein Wasserstoffatom, eine C₁₋₃-Alkyl-, C₅₋₇-Cycloalkyl- oder Phenylgruppe und
R_{f} ein Wasserstoffatom oder eine C₁₋₃-Alkylgruppe darstellen,
unter einer unter physiologischen Bedingungen negativ geladenen Gruppe eine Tetrazol-5-yl-, Phenylcarbonylaminocarbonyl-, Trifluormethylcarbonylaminocarbonyl-, C₁₋₆-Alkylsulfonylamino-, Phenylsulfonylamino-, Benzylsulfonylamino-, Trifluormethylsulfonylamino-, C₁₋₆-Alkylsulfonylaminocarbonyl-, Phenylsulfonylaminocarbonyl-, Benzylsulfonylaminocarbonyl- oder Perfluor-C₁₋₆-alkylsulfonylaminocarbon ylgruppe
und unter einem von einer Imino- oder Aminogruppe in-vivo abspaltbaren Rest eine Hydroxygruppe, eine gegebenenfalls durch Fluor-, Chlor-, Brom- oder Jodatome, durch C₁₋₃-Alkyl- oder C₁₋₃-Alkoxygruppen mono- oder disubstituierte Benzoylgruppe, wobei die Substituenten gleich oder verschieden sein können, eine Pyridinoylgruppe oder eine C₁₋₁₆-Alkanoylgruppe, eine 3,3,3-Trichlorpropionyl- oder Allyloxycarbonylgruppe, eine C₁₋₁₆-Alkoxycarbonyl- oder C₁₋₁₆-Alkylcarbonyloxygruppe, in denen Wasserstoffatome ganz oder teilweise durch Fluor- oder Chloratome ersetzt sein können, eine Phenyl-C₁₋₆-alkoxycarbonylgruppe, eine 3-Amino-propionylgruppe, in der die Aminogruppe durch C₁₋₆-Alkyl- oder C₃₋₇-Cycloalkylgruppen mono- oder disubstituiert und die Substituenten gleich oder verschieden sein können, eine C₁₋₃-Alkylsulfonyl-C₂₋₄-alkoxycarbonyl-, C₁₋₃-Alkoxy-C₂₋₄-alkoxy-C₂₋₄-alkoxycarbonyl-, R_{d}-CO-O-(R_{d}CR_{f})-O-CO-, C₁₋₆-Alkyl-CO-NH-(R_{g}CRₕ)-O-CO- oder C₁₋₆-Alkyl-CO-O-(R_{g}CRₕ)-(R_{g}CRₕ)-O-CO-Gruppe, in denen R_{d} bis R_{f} wie vorstehend erwähnt definiert sind und
R_{g} und Rₕ, die gleich oder verschieden sein können, Wasserstoffatome oder C₁₋₃-Alkylgruppen darstellen,
verstanden werden,
deren Tautomeren, deren Stereoisomeren, deren Gemischen und deren Salzen und
(b) mindestens einen Wirkstoff ausgewählt aus der Gruppe bestehend aus Hemmern der Plättchenfunktion - wie insbesondere Acetylsalicylsäure, Fibrinogen-Rezeptorantagonisten, Inhibitoren der ADP-induzierten Aggregation, P₂T-Rezeptorantagonisten und kombinierten Thromboxan Rezeptorantagonisten / Synthetaseinhibitoren -, thrombolytisch wirksamen Substanzen - wie insbesondere Alteplase, Reteplase, Tenecteplase, Urokinase, Staphylokinase und Streptokinase -, physiologischen Aktivatoren und Inhibitoren des Gerinnungssystems und deren rekombinanten Analoga - wie insbesondere Protein C, rekombinantem humanen aktivierten Protein C, TFPI und Antithrombin -, Substanzen mit antagonistischer Wirkung gegen Endotoxine, Interleukinen, TNF, Bradykinin, Prostaglandinen, Cyclooxygenasen, NO, platelet activating factor Acetylhydrolasen, Entzündungshemmern, immun supprimieren den Substanzen, Antibiotika und Katecholaminen.

9. Pharmazeutische Zusammensetzung gemäß Anspruch 8, enthaltend
(a) (R)-2-(4-Amidinophenylaminomethyl)-1-methyl-5-[1-(carboxymethylamino)-1-(pyrrolidinocarbonyl)-ethyl]-benzimidazol, gegebenenfalls in Form der pharmazeutisch verträglichen Säureadditionssalze sowie gegebenenfalls in Form der Hydrate oder Solvate, und
(b) eine PAF-AH oder ein PAF-AH Derivat.

10. Arzneimittelkit enthaltend mindestens
(a) (R)-2-(4-Amidinophenylaminomethyl)-1-methyl-5-[1-(carboxymethylamino)-1-(pyrrolidinocarbonyl)-ethyl]-benzimidazol, gegebenenfalls in Form der pharmazeutisch verträglichen Säureadditionssalze sowie gegebenenfalls in Form der Hydrate oder Solvate, und
(b) eine PAF-AH oder ein PAF-AH Derivat.

11. Arzneimittelkit enthaltend mindestens
(a) (R)-2-(4-Amidinophenylaminomethyl)-1-methyl-5-[1-(carboxymethylamino)-1-(pyrrolidinocarbonyl)-ethyl]-benzimidazol, gegebenenfalls in Form der pharmazeutisch verträglichen Säureadditionssalze sowie gegebenenfalls in Form der Hydrate oder Solvate, und
(b) einen Tumor Necrose Faktor alpha (TNF-alpha)-Antagonisten.

12. Verwendung einer PAF-AH oder eines PAF-AH-Derivats oder eines TNF-alpha-Antagonisten zur Herstellung eines Arzneimittels zur kombinierten Anwendung mit einem (R)-2-(4-Amidinophenylaminomethyl)-1-methyl-5-[1-(carboxymethylamino)-1-(pyrrolidinocarbonyl)-ethyl]-benzimidazol.

## Claims

1. Use of benzimidazoles of general formula (1) 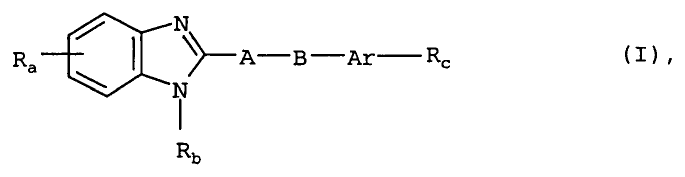 wherein
Ar denotes a phenylene or naphthylene group optionally substituted by a fluorine, chlorine or bromine atom, by a trifluoromethyl, C₁₋₃-alkyl or C₁₋₃-alkoxy group,
a thienylene, thiazolylene, pyridinylene, pyrimidinylene, pyrazinylene or pyridazinylene group optionally substituted in the carbon skeleton by a C₁₋₃-alkyl group,
A denotes a C₁₋₃-alkylene group,
B denotes an oxygen or sulphur atom, a methylene, carbonyl, sulphinyl or sulphonyl group, an imino group optionally substituted by a C₁₋₃-alkyl group wherein the alkyl moiety may be mono- or disubstituted by a carboxy group,
Rₐ denotes an R₁-CO-C₃₋₅-cycloalkyl group wherein
R₁ denotes a C₁₋₃-alkoxy, amino, C₁₋₄-alkylamino or di-(C₁₋₄-alkyl)-amino group, wherein in each case the alkyl moiety may be substituted by a carboxy group,
a 4- to 7-membered cycloalkyleneimino or cycloalkenyleneimino group which may be substituted by one or two C₁₋₃-alkyl groups, while an alkyl substituent may simultaneously be substituted by a hydroxy, C₁₋₃-alkoxy, carboxy, carboxy-C₁₋₃-alkoxy, carboxy-C₁₋₃-alkylamino, N-(C₁₋₃-alkyl)-N-(carboxy-C₁₋₃-alkyl)-amino, carboxy-C₁₋₃-alkylaminocarbonyl, N-(C₁₋₃-alkyl)-N-(carboxy-C₁₋₃-alkyl)-aminocarbonyl, carboxy-C₁₋₃-alkylaminocarbonylamino, 1-(C₁₋₃-alkyl)-3-(carboxy-C₁₋₃-alkyl)-aminocarbonylamino, 3-(C₁₋₃-alkyl)-3-(carboxy-C₁₋₃-alkyl)-aminocarbonylamino or 1,3-di-(C₁₋₃-alkyl)-3-(carboxy-C₁₋₃-alkyl)-aminocarbonylamino group,
a 4- to 7-membered cycloalkyleneimino group substituted by a hydroxy group,
a 5- to 7-membered cycloalkyleneimino group optionally substituted by a C₁₋₃-alkyl group, to which a phenyl ring is fused via two adjacent carbon atoms,
a morpholino, piperazino, N-(C₁₋₃-alkyl)-piperazino, pyrrolino, 3,4-dehydro-piperidino or pyrrol-1-yl group,
a R₂-CX-C₃₋₅-cycloalkyl group wherein
R₂ denotes a phenyl, naphthyl or monocyclic 5- or 6-membered heteroaryl group optionally substituted by a C₁₋₃-alkyl group, wherein the 6-membered heteroaryl group contains one, two or three nitrogen atoms and the 5-membered heteroaryl group contains an imino group optionally substituted by a C₁₋₃-alkyl group, an oxygen or sulphur atom or an imino group optionally substituted by a C₁₋₃-alkyl group and an oxygen or sulphur atom or one or two nitrogen atoms and the above-mentioned alkyl substituent may be substituted by a carboxy, carboxy-C₁₋₃-alkoxy, carboxy-C₁₋₃-alkylamino or N-(C₁₋₃-alkyl)-carboxy-C₁₋₃-alkylamino group, and
X denotes an oxygen atom, a C₁₋₃-alkylimino, C₁₋₃-alkoxyimino, C₁₋₃-alkylhydrazino, di-(C₁₋₃-alkyl)-hydrazino, C₂₋₄-alkanoylhydrazino, N-(C₁₋₃-alkyl)-C₂₋₄-alkanoylhydrazino or C₁₋₃-alkylidene group each of which may be substituted by a carboxy group in the alkyl or alkanoyl moiety or in the alkyl and alkanoyl moiety,
a C₁₋₃-alkyl or C₃₋₅-cycloalkyl group substituted by an imidazole or imidazolone group, wherein
the imidazole ring may be substituted by a phenyl or carboxy group and by one or two C₁₋₃-alkyl groups or by one, two or three C₁₋₃-alkyl groups, while the substituents may be identical or different and one of the above-mentioned alkyl substituents may simultaneously be substituted by a carboxy group or in the 2 or 3 position by an amino, C₂₋₄-alkanoylamino, C₁₋₃-alkylamino, N-(C₂₋₄-alkanoyl)-C₁₋₃-alkylamino or di-(C₁₋₃-alkyl)-amino group, and
the imidazolone ring may be substituted by a C₁₋₃-alkyl group, while the alkyl substituent may be substituted by a carboxy group or in the 2 or 3 position by an amino, C₂₋₄-alkanoylamino, C₁₋₃-alkylamino, N-(C₂₋₄-alkanoyl)-C₁₋₃-alkylamino or di-(C₁₋₃-alkyl)-amino group, and
additionally a phenyl or pyridine ring may be fused to the above-mentioned imidazole and imidazolone rings via two adjacent carbon atoms,
an imidazolidin-2,4-dion-5-yl group which may be substituted by one or two C₁₋₃-alkyl groups, while simultaneously an alkyl substituent may be substituted by a carboxy group,
a C₁₋₄-alkyl group which is substituted
by a C₁₋₃-alkyl-Y₁-C₁₋₃-alkyl, HOOC-C₁₋₃-alkyl-Y₁-C₁₋₃-alkyl, tetrazolyl-C₁₋₃-alkyl-Y₂, R₃NR₄ or R₃NR₄-C₁₋₃-alkyl group and
by an isoxazolidinylcarbonyl group optionally substituted by a C₁₋₃-alkyl group, by a pyrrolinocarbonyl, 3,4-dehydro-piperidinocarbonyl, pyrrol-1-yl-carbonyl, carboxy, aminocarbonyl, C₁₋₃-alkylaminocarbonyl, di-(C₁₋₃-alkyl)-aminocarbonyl or 4- to 7-membered cycloalkyleneiminocarbonyl group, while in the above-mentioned groups the cycloalkyleneimino moiety may be substituted by one or two C₁₋₃-alkyl groups and simultaneously in each case an alkyl moiety or alkyl substituent of the above-mentioned C₁₋₃-alkylaminocarbonyl, di-(C₁₋₃-alkyl)-aminocarbonyl or cycloalkyleneiminocarbonyl groups may be substituted by a carboxy group, and the remaining hydrogen atoms of the C₁₋₄-alkyl group may be wholly or partly replaced by fluorine atoms, wherein
R₃ denotes a hydrogen atom or a C₁₋₃-alkyl group optionally substituted by a carboxy group and
R₄ denotes a hydrogen atom, a C₁₋₃-alkyl-Y₁-C₁₋₃-alkyl-Y₂, carboxy-C₁₋₃-alkyl-Y₁-C₁₋₃-alkyl-Y₂, C₁₋₃-alkyl-Y₂ or carboxy-C₁₋₃-alkyl-Y₂ group or
R₃ and R₄ together with the nitrogen atom between them denote a 4- to 7-membered cycloalkyleneimino group optionally substituted by a carboxy, C₁₋₃-alkyl or carboxy-C₁₋₃-alkyl group, wherein
Y₁ denotes a carbon-carbon bond, an oxygen atom, a sulphenyl, sulphinyl, sulphonyl, -NH, -NH-CO or -NH-CO-NH group and
Y₂ denotes a carbon-nitrogen bond or a carbonyl, sulphonyl, imino or -NH-CO group, while the carbonyl group of the -NH-CO group is linked to the nitrogen atom of the R₃NR₄ group, and the imino groups mentioned in the definition of the groups Y₁ and Y₂ may each additionally be substituted by a C₁₋₃-alkyl or carboxy-C₁₋₃-alkyl group,
a C₁₋₃-alkyl or C₃₋₅-cycloalkyl group substituted by a R₅NR₆ group, wherein
R₅ denotes a hydrogen atom, a C₁₋₃-alkyl, C₅₋₇-cycloalkyl, phenylcarbonyl, phenylsulphonyl or pyridinyl group and
R₆ denotes a C₁₋₃-alkyl, carboxy-C₁₋₃-alkyl or carboxy-C₁₋₃-alkylcarbonyl group,
a C₁₋₃-alkyl group which is substituted by a C₂₋₄-alkanoyl or C₅₋₇-cycloalkanoyl group and by a C₁₋₃-alkyl group substituted by a chlorine, bromine or iodine atom,
R_{b} denotes a hydrogen atom or a C₁₋₃-alkyl group and
R_{c} denotes a cyano group or an amidino group optionally substituted by one or two C₁₋₃-alkyl groups, wherein
the carboxy groups mentioned in the definition of the above-mentioned groups may also be replaced by a group which may be converted *in vivo* into a carboxy group or by a group which is negatively charged under physiological conditions or
the amino and imino groups mentioned in the definition of the above-mentioned groups may also be substituted by a group which can be cleaved *in vivo,* while
by a group which may be converted into a carboxy group *in vivo* is meant a hydroxymethyl group, a carboxy group esterified with an alcohol wherein the alcoholic moiety is a C₁₋₆-alkanol, a phenyl-C₁₋₃-alkanol, a C₃₋₉-cycloalkanol, while a C₅₋₈₋cycloalkanol may additionally be substituted by one or two C₁₋₃-alkyl groups, a C₅₋₈-cycloalkanol wherein a methylene group in the 3 or 4 position is replaced by an oxygen atom or by an imino group optionally substituted by a C₁₋₃-alkyl, phenyl-C₁₋₃-alkyl, phenyl-C₁₋₃-alkoxycarbonyl or C₂₋₆-alkanoyl group and the cycloalkanol moiety may additionally be substituted by one or two C₁₋₃-alkyl groups, a C₄₋₇-cydoalkenol, a C₃₋₅-alkenol, a phenyl-C₃₋₅-alkenol, a C₃₋₅-alkynol or phenyl- C₃₋₅-alkynol, with the proviso that no bond to the oxygen atom starts from a carbon atom which carries a double or triple bond, a C₃₋₈-cycloalkyl-C₁₋₃-alkanol, a bicycloalkanol with a total of 8 to 10 carbon atoms which may additionally be substituted in the bicycloalkyl moiety by one or two C₁₋₃-alkyl groups, a 1,3-dihydro-3-oxo-1-isobenzofuranol or an alcohol of formula
R_{d}-CO-O-(RₑCR_{f})-OH,
wherein
R_{d} denotes a C₁₋₈-alkyl, C₅₋₇-cycloalkyl, phenyl or phenyl-C₁₋₃-alkyl group
Rₑ denotes a hydrogen atom, a C₁₋₃-alkyl, C₅₋₇-cydoalkyl or phenyl group and
R_{f} denotes a hydrogen atom or a C₁₋₃-alkyl group,
by a group which is negatively charged under physiological conditions is meant a tetrazol-5-yl, phenylcarbonylaminocarbonyl, trifluoromethylcarbonylaminocarbonyl, C₁-₆-alkylsulphonylamino, phenylsulphonylamino, benzylsulphonylamino, trifluoromethylsulphonylamino, C₁₋₆-alkylsulphonylaminocarbonyl, phenylsulphonylaminocarbonyl, benzylsulphonylaminocarbonyl or perfluoro-C₁₋₆-alkylsulphonylaminocarbonyl group
and by a group which can be cleaved from an imino or amino group *in vivo* is meant a hydroxy group, a benzoyl group optionally mono- or disubstituted by fluorine, chlorine, bromine or iodine atoms, by C₁₋₃-alkyl or C₁₋₃-alkoxy groups, while the substituents may be identical or different, a pyridinoyl group or a C₁₋₁₆-alkanoyl group, a 3,3,3-trichloropropionyl or allyloxycarbonyl group, a C₁₋₁₆-alkoxycarbonyl or C₁₋₁₆-alkylcarbonyloxy group, wherein hydrogen atoms may be wholly or partly replaced by fluorine or chlorine atoms, a phenyl-C₁₋₆-alkoxycarbonyl group, a 3-amino-propionyl group wherein the amino group may be mono- or disubstituted by C₁₋₆-alkyl or C₃₋₇-cycloalkyl groups and the substituents may be identical or different, a C₁₋₃-alkylsulphonyl-C₂₋₄-alkoxycarbonyl, C₁₋₃-alkoxy-C₂₋₄-alkoxy-C₂₋₄-alkoxycarbonyl, R_{d}-CO-O-(R_{d}CR_{f})-O-CO, C₁₋₆-alkyl-CO-NH-(R_{g}CRₕ)-O-CO or C₁₋₆-alkyl-CO-O-(R_{g}CRₕ)-(R_{g}CRₕ)-O-CO group, wherein R_{d} to R_{f} are as hereinbefore defined and
R_{g} and Rₕ, which may be identical or different, denote hydrogen atoms or C₁₋₃-alkyl groups,
the tautomers, stereoisomers, mixtures thereof and the salts thereof,
optionally in the form of the pharmaceutically acceptable acid addition salts thereof, as well as optionally in the form of the hydrates or solvates thereof, for preparing a pharmaceutical composition for the prevention or treatment of diseases from the group consisting of systemic inflammatory response syndrome (SIRS), sepsis and bacteraemia.

2. Use according to claim 1, wherein the benzimidazole used is (R)-2-(4-amidinophenylaminomethyl)-1-methyl-5-[1-(carboxymethylamino)-1-(pyrrolidinocarbonyl)-ethyl]-benzimidazole, optionally in the form of the pharmaceutically acceptable acid addition salts thereof, and optionally in the form of the hydrates or solvates thereof.

3. Use according to claim 2, wherein the monohydrochloride salt of (R)-2-(4-amidinophenylaminomethyl)-1-methyl-5-[1-(carboxymethylamino)-1-(pyrrolidinocarbonyl)-ethyl]-benzimidazole is used.

4. Use according to one of claims 1 to 3, wherein the condition is selected from the group consisting of SIRS caused by gram-positive pathogens, SIRS caused by gram-negative pathogens, SIRS caused by viruses, SIRS caused by single-cell eukaryotic parasites, SIRS caused by fungi, sepsis without organ failure, sepsis with organ failure, septic shock, septic syndrome, SIRS caused by pancreatitis, SIRS caused by systemic ischaemia, SIRS caused by organ-limited ischaemia, SIRS caused by trauma, SIRS occurring in connection with tumour diseases, SIRS caused by tissue damage, SIRS caused by bums, SIRS occurring after lengthy operations, SIRS as a consequence of organ transplants, SIRS as the result of shock of various kinds, SIRS caused by blood loss, SIRS as the result of cardiovascular failure, SIRS as the result of immuno-mediated organ failure, SIRS as the result of inflammatory reactions, SIRS as the result of treatment with inflammation mediators such as for example tumour necrosis factor alpha and/or beta and/or other cytokines, and also consisting of lung damage, acute lung failure and ARDS (acute respiratory distress syndrome), acute cardiovascular failure, organ failure after resuscitation, shock, kidney failure, cardiovascular failure, haematological damage, acidosis and multiple organ dysfunction syndrome (MODS) occurring in connection with SIRS.

5. Use according to one of claims 1 to 4, wherein the pharmaceutical composition is intended as an accompanying treatment for bacteraemia.

6. Use according to one of claims 1 to 5, wherein the pharmaceutical composition is intended for subcutaneous or parenteral and particularly intravenous administration.

7. Use according to one of claims 1 to 6, wherein, in addition to one of the substances mentioned in claims 1 and 2, a pharmacologically active substance selected from the group consisting of inhibitors of platelet function such as in particular acetylsalicylic acid, fibrinogen receptor antagonists, inhibitors of ADP-induced aggregation, P₂T receptor antagonists and combined thromboxane receptor antagonists / synthetase inhibitors, further consisting of thrombolytically active substances, such as in particular alteplase, reteplase, tenecteplase, urokinase, staphylokinase and streptokinase, further consisting of physiological activators and inhibitors of the clotting system and their recombinant analogues such as particularly Protein C, recombinant human activated Protein C, TFPI and antithrombin, further consisting of substances with an antagonistic effect on endotoxins, and further consisting of interleukins, TNF, bradykinin, prostaglandins, cyclooxygenases, NO, platelet activating factor, acetylhydrolases, inflammation inhibitors, immunosuppressant substances, antibiotics and catecholamines, is used.

8. Pharmaceutical composition containing
(a) at least one active substance selected from the group of benzimidazoles of general formula (1) 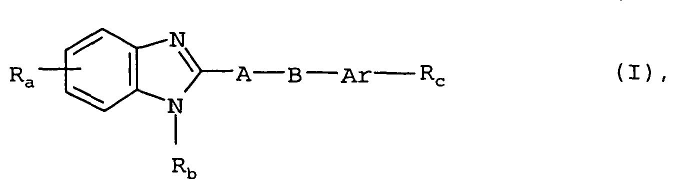 wherein
Ar denotes a phenylene or naphthylene group optionally substituted by a fluorine, chlorine or bromine atom, by a trifluoromethyl, C₁₋₃-alkyl or C₁₋₃-alkoxy group,
a thienylene, thiazolylene, pyridinylene, pyrimidinylene, pyrazinylene or pyridazinylene group optionally substituted in the carbon skeleton by a C₁₋₃-alkyl group,
A denotes a C₁₋₃-alkylene group,
B denotes an oxygen or sulphur atom, a methylene, carbonyl, sulphinyl or sulphonyl group, an imino group optionally substituted by a C₁₋₃-alkyl group wherein the alkyl moiety may be mono- or disubstituted by a carboxy group,
Rₐ denotes a R₁-CO-C₃₋₅-cycloalkyl group wherein
R₁ denotes a C₁₋₃-alkoxy, amino, C₁₋₄-alkylamino or di-(C₁₋₄-alkyl)-amino group, wherein in each case the alkyl moiety may be substituted by a carboxy group,
a 4- to 7-membered cycloalkyleneimino or cycloalkenyleneimino group which may be substituted by one or two C₁₋₃-alkyl groups, wherein an alkyl substituent may simultaneously be substituted by a hydroxy, C₁₋₃-alkoxy, carboxy, carboxy-C₁₋₃-alkoxy, carboxy-C₁₋₃-alkylamino, N-(C₁₋₃-alkyl)-N-(carboxy-C₁₋₃-alkyl)-amino, carboxy-C₁₋₃-alkylaminocarbonyl, N-(C₁₋₃-alkyl)-N-(carboxy-C₁₋₃-alkyl)-aminocarbonyl, carboxy-C₁₋₃-alkylaminocarbonylamino, 1-(C₁₋₃-alkyl)-3-(carboxy-C₁₋₃-alkyl)-aminocarbonylamino, 3-(C₁₋₃-alkyl)-3-(carboxy-C₁₋₃-alkyl)-aminocarbonylamino or 1,3-di-(C₁₋₃-alkyl)-3-(carboxy-C₁₋₃-alkyl)-aminocarbonylamino group,
a 4- to 7-membered cycloalkyleneimino group substituted by a hydroxy group,
a 5- to 7-membered cycloalkyleneimino group optionally substituted by a C₁₋₃-alkyl group, to which a phenyl ring is fused via two adjacent carbon atoms,
a morpholino, piperazino, N-(C₁₋₃-alkyl)-piperazino, pyrrolino, 3,4-dehydro-piperidino or pyrrol-1-yl group,
a R₂-CX-C₃₋₅-cycloalkyl group wherein
R₂ denotes a phenyl, naphthyl or monocyclic 5- or 6-membered heteroaryl group optionally substituted by a C₁₋₃-alkyl group, wherein the 6-membered heteroaryl group contains one, two or three nitrogen atoms and the 5-membered heteroaryl group contains an imino group optionally substituted by a C₁₋₃-alkyl group, an oxygen or sulphur atom or an imino group optionally substituted by a C₁₋₃-alkyl group and an oxygen or sulphur atom or one or two nitrogen atoms and the above-mentioned alkyl substituent may be substituted by a carboxy, carboxy-C₁₋₃-alkoxy, carboxy-C₁₋₃-alkylamino or N-(C₁₋₃-alkyl)-carboxy-C₁₋₃-alkylamino group, and
X denotes an oxygen atom, a C₁₋₃-alkylimino, C₁₋₃-alkoxyimino, C₁₋₃-alkylhydrazino, di-(C₁₋₃-alkyl)-hydrazino, C₂₋₄-alkanoylhydrazino, N-(C₁₋₃-alkyl)-C₂₋₄-alkanoylhydrazino or C₁₋₃-alkylidene group each of which may be substituted by a carboxy group in the alkyl or alkanoyl moiety or in the alkyl and alkanoyl moiety,
a C₁₋₃-alkyl or C₃₋₅-cycloalkyl group substituted by an imidazole or imidazolone group, wherein
the imidazole ring may be substituted by a phenyl or carboxy group and by one or two C₁₋₃-alkyl groups or by one, two or three C₁₋₃-alkyl groups, wherein the substituents may be identical or different and one of the above-mentioned alkyl substituents may simultaneously be substituted by a carboxy group or in the 2 or 3 position by an amino, C₂₋₄-alkanoylamino, C₁₋₃-alkylamino, N-(C₂₋₄-alkanoyl)-C₁₋₃-alkylamino or di-(C₁₋₃-alkyl)-amino group, and
the imidazolone ring may be substituted by a C₁₋₃-alkyl group, while the alkyl substituent may be substituted by a carboxy group or in the 2 or 3 position by an amino, C₂₋₄-alkanoylamino, C₁₋₃-alkylamino, N-(C₂₋₄-alkanoyl)-C₁₋₃-alkylamino or di-(C₁₋₃-alkyl)-amino group, and
additionally a phenyl or pyridine ring may be fused to the above-mentioned imidazole and imidazolone rings via two adjacent carbon atoms,
an imidazolidin-2,4-dion-5-yl group which may be substituted by one or two C₁₋₃-alkyl groups, while simultaneously an alkyl substituent may be substituted by a carboxy group,
a C₁₋₄-alkyl group which is substituted
by a C₁₋₃-alkyl-Y₁-C₁₋₃-alkyl, HOOC-C₁₋₃-alkyl-Y₁-C₁₋₃-alkyl, tetrazolyl-C₁₋₃-alkyl-Y₂, R₃NR₄ or R₃NR₄-C₁₋₃-alkyl group and
by an isoxazolidinylcarbonyl group optionally substituted by a C₁₋₃-alkyl group, by a pyrrolinocarbonyl, 3,4-dehydro-piperidinocarbonyl, pyrrol-1-yl-carbonyl, carboxy, aminocarbonyl, C₁₋₃-alkylaminocarbonyl, di-(C₁₋₃-alkyl)-aminocarbonyl or 4- to 7-membered cycloalkyleneiminocarbonyl group, while in the above-mentioned groups the cycloalkyleneimino moiety may be substituted by one or two C₁₋₃-alkyl groups and simultaneously in each case an alkyl moiety or alkyl substituent of the above-mentioned C₁₋₃-alkylaminocarbonyl, di-(C₁₋₃-alkyl)-aminocarbonyl or cycloalkyleneiminocarbonyl groups may be substituted by a carboxy group, and the remaining hydrogen atoms of the C₁₋₄-alkyl group may be wholly or partly replaced by fluorine atoms, wherein
R₃ denotes a hydrogen atom or a C₁₋₃-alkyl group optionally substituted by a carboxy group and
R₄ denotes a hydrogen atom, a C₁₋₃-alkyl-Y,-C₁₋₃-alkyl-Y₂, carboxy-C₁₋₃-alkyl-Y₁-C₁₋₃-alkyl-Y₂, C₁₋₃-alkyl-Y₂ or carboxy-C₁₋₃-alkyl-Y₂ group or
R₃ and R₄ together with the nitrogen atom between them denote a 4- to 7-membered cycloalkyleneimino group optionally substituted by a carboxy, C₁₋₃-alkyl or carboxy-C₁₋₃-alkyl group, wherein
Y₁ denotes a carbon-carbon bond, an oxygen atom, a sulphenyl, sulphinyl, sulphonyl, -NH, -NH-CO or -NH-CO-NH group and
Y₂ denotes a carbon-nitrogen bond or a carbonyl, sulphonyl, imino or -NH-CO group, while the carbonyl group of the -NH-CO group is linked to the nitrogen atom of the R₃NR₄ group, and the imino groups mentioned in the definition of the groups Y₁ and Y₂ may each additionally be substituted by a C₁₋₃-alkyl or carboxy-C₁₋₃-alkyl group,
a C₁₋₃-alkyl or C₃₋₅-cycloalkyl group substituted by a R₅NR₆ group, wherein
R₅ denotes a hydrogen atom, a C₁₋₃-alkyl, C₅₋₇-cycoalkyl, phenylcarbonyl, phenylsulphonyl or pyridinyl group and
R₆ denotes a C₁₋₃-alkyl, carboxy-C₁₋₃-alkyl or carboxy-C₁₋₃-alkylcarbonyl group,
a C₁₋₃-alkyl group which is substituted by a C₂₋₄-alkanoyl or C₅₋₇-cycloalkanoyl group and by a C₁₋₃-alkyl group substituted by a chlorine, bromine or iodine atom,
R_{b} denotes a hydrogen atom or a C₁₋₃-alkyl group and
R_{c} denotes a cyano group or an amidino group optionally substituted by one or two C₁₋₃-alkyl groups, wherein
the carboxy groups mentioned in the definition of the above-mentioned groups may also be replaced by a group which may be converted in *vivo* into a carboxy group or by a group which is negatively charged under physiological conditions or
the amino and imino groups mentioned in the definition of the above-mentioned groups may also be substituted by a group which can be cleaved *in vivo,* while
by a group which may be converted into a carboxy group *in vivo* is meant a hydroxymethyl group, a carboxy group esterified with an alcohol wherein the alcoholic moiety is a C₁₋₆-alkanol, a phenyl-C₁₋₃-alkanol, a C₃₋₉-cycloalkanol, while a C₅₋₈-cycloalkanol may additionally be substituted by one or two C₁₋₃-alkyl groups, a C₅₋₈-cycloalkanol wherein a methylene group in the 3 or 4 position is replaced by an oxygen atom or by an imino group optionally substituted by a C₁₋₃-alkyl, phenyl-C₁₋₃-alkyl, phenyl-C₁₋₃-alkoxycarbonyl or C₂₋₆-alkanoyl group and the cycloalkanol moiety may additionally be substituted by one or two C₁₋₃-alkyl groups, a C₄₋₇-cycloalkenol, a C₃₋₅-alkenol, a phenyl-C₃₋₅-alkenol, a C₃₋₅-alkynol or phenyl- C₃₋₅-alkynol, with the proviso that no bond to the oxygen atom starts from a carbon atom which carries a double or triple bond, a C₃₋₈-cycloalkyl-C₁₋₃-alkanol, a bicycloalkanol with a total of 8 to 10 carbon atoms which may additionally be substituted in the bicycloalkyl moiety by one or two C₁₋₃-alkyl groups, a 1,3-dihydro-3-oxo-1-isobenzofuranol or an alcohol of formula
R_{d}-CO-O-(RₑCR_{f})-OH,
wherein
R_{d} denotes a C₁₋₈-alkyl, C₅₋₇-cycloalkyl, phenyl or phenyl-C₁₋₃-alkyl group
Rₑ denotes a hydrogen atom, a C₁₋₃-alkyl, C₅₋₇-cycloalkyl or phenyl group and
R_{f} denotes a hydrogen atom or a C₁₋₃-alkyl group,
by a group which is negatively charged under physiological conditions is meant a tetrazol-5-yl, phenylcarbonylaminocarbonyl, trifluoromethylcarbonylaminocarbonyl, C₁₋₆-alkylsulphonylamino, phenylsulphonylamino, benzylsulphonylamino, trifluoromethylsulphonylamino, C₁₋₆-alkylsulphonylaminocarbonyl, phenylsulphonylaminocarbonyl, benzylsulphonylaminocarbonyl or perfluoro-C₁₋₆-alkylsulphonylaminocarbonyl group
and by a group which can be cleaved from an imino or amino group *in vivo* is meant a hydroxy group, a benzoyl group optionally mono- or disubstituted by fluorine, chlorine, bromine or iodine atoms, by C₁₋₃-alkyl or C₁₋₃-alkoxy groups, while the substituents may be identical or different, a pyridinoyl group or a C₁₋₁₆-alkanoyl group, a 3,3,3-trichloropropionyl or allyloxycarbonyl group, a C₁₋₁₆-alkoxycarbonyl or C₁₋₁₆-alkylcarbonyloxy group, wherein hydrogen atoms may be wholly or partly replaced by fluorine or chlorine atoms, a phenyl-C₁₋₆-alkoxycarbonyl group, a 3-amino-propionyl group wherein the amino group may be mono- or disubstituted by C₁₋₆-alkyl or C₃₋₇-cycloalkyl groups and the substituents may be identical or different, a C₁₋₃-alkylsulphonyl-C₂₋₄-alkoxycarbonyl, C₁₋₃-alkoxy-C₂₋₄-alkoxy-C₂₋₄-alkoxycarbonyl, R_{d}-CO-O-(R_{d}CR_{f})-O-CO, C₁₋₆-alkyl-CO-NH-(R_{g}CRₕ)-O-CO or C₁₋₆-alkyl-CO-O-(R_{g}CRₕ)-(R_{g}CRₕ)-O-CO group, wherein R_{d} to R_{f} are as hereinbefore defined and
R_{g} and Rₕ, which may be identical or different, denote hydrogen atoms or C₁₋₃-alkyl groups,
the tautomers, stereoisomers, mixtures thereof and the salts thereof, and
(b) at least one active substance selected from the group consisting of inhibitors of platelet function such as in particular acetylsalicylic acid, fibrinogen receptor antagonists, inhibitors of ADP-induced aggregation, P₂T receptor antagonists and combined thromboxane receptor antagonists / synthetase inhibitors, thrombolytically active substances, such as in particular alteplase, reteplase, tenecteplase, urokinase, staphylokinase and streptokinase, physiological activators and inhibitors of the clotting system and their recombinant analogues such as in particular Protein C, recombinant human activated Protein C, TFPI and antithrombin, substances with an antagonistic effect on endotoxins, interleukins, TNF, bradykinin, prostaglandins, cyclooxygenases, NO, platelet activating factor acetylhydrolases, inflammation inhibitors, immunosuppressant substances, antibiotics and catecholamines

9. Pharmaceutical composition according to claim 8, containing
(a) (R)-2-(4-amidinophenylaminomethyl)-1-methyl-5-[1-(carboxymethylamino)-1-(pyrrolidinocarbonyl)-ethyl]-benzimidazole, optionally in the form of the pharmaceutically acceptable acid addition salts and optionally in the form of the hydrates or solvates thereof, and
(b) a PAF-AH or a PAF-AH derivative.

10. Pharmaceutical kit containing at least
(a) (R)-2-(4-amidinophenylaminomethyl)-1-methyl-5-[1-(carboxymethylamino)-1-(pyrrolidinocarbonyl)-ethyl]-benzimidazole, optionally in the form of the pharmaceutically acceptable acid addition salts and optionally in the form of the hydrates or solvates thereof, and
(b) a PAF-AH or a PAF-AH derivative.

11. Pharmaceutical kit containing at least
(a) (R)-2-(4-amidinophenylaminomethyl)-1-methyl-5-[1-(carboxymethylamino)-1-(pyrrolidinocarbonyl)-ethyl]-benzimidazole, optionally in the form of the pharmaceutically acceptable acid addition salts and optionally in the form of the hydrates or solvates thereof, and
(b) a tumour necrosis factor alpha (TNF-alpha) antagonist.

12. Use of a PAF-AH or a PAF-AH derivative or a TNF-alpha antagonist for preparing a pharmaceutical composition for combined use with a (R)-2-(4-amidinophenylaminomethyl)-1-methyl-5-[1-(carboxymethylamino)-1-(pyrrolidinocarbonyl)-ethyl]-benzimidazole.

## Revendications

1. Utilisation de benzimidazoles de formule générale (I) 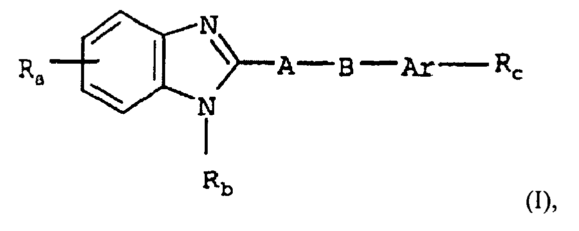 dans laquelle
Ar représente un groupe phénylène ou naphtylène éventuellement substitué par un atome de fluor, de chlore ou de brome, par un groupe trifluorométhyle, alkyle en C₁ à C₃ ou alcoxy en C₁ à C₃,
un groupe thiénylène, thiazolylène, pyridinylène, pyrimidinylène, pyrazinylène ou pyridazinylène éventuellement substitué sur le squelette carboné par un groupe alkyle en C₁ à C₃,
A représente un groupe alkylène en C₁ à C₃,
B représente un atome d'oxygène ou de soufre, un groupe méthylène, carbonyle, sulfinyle ou sulfonyle, un groupe imino éventuellement substitué par un groupe alkyle en C₁ à C₃, dans lequel la partie alkyle peut être mono- ou di-substituée par un groupe carboxy,
Rₐ représente un groupe R₁-CO-cycloalkyle en C₃ à C₅, dans lequel
R₁ représente un groupe alcoxy en C₁ à C₃, amino, alkylamino en C₁ à C₄ ou di-(alkyle en C₁ à C₄)-amino, dans lesquels la partie alkyle peut être substituée le cas échéant par un groupe carboxy,
un groupe cycloalkylènimino ou cycloalkènylènimino de 4 à 7 chaînons, qui peuvent être substitués par un ou deux groupes alkyle en C₁ à C₃, dans lequel un substituant alkyle peut être substitué en même temps par un groupe hydroxy, alcoxy en C₁ à C₃, carboxy, carboxy-alcoxy en C₁ à C₃, carboxy-alkylamino en C₁ à C₃, N-(alkyle en C₁ à C₃)-N-(carboxy-alkyle en C₁ à C₃)-amino, carboxy-alkylaminocabonyle en C₁ à C₃, N-(alkyle en C₁ à C₃)-N-(carboxy-alkyle en C₁ à C₃)-aminocarbonyle, carboxy-alkyle en C₁ à C₃-aminocarbonylamino, 1-(alkyle en C₁ à C₃)-3-(carboxy- alkyle en C₁ à C₃)-aminocarbonylamino, 3-(alkyle en C₁ à C₃)-3-(carboxy-alkyle en C₁ à C₃)-aminocarbonylamino ou 1,3-di-(alkyle en C₁ à C₃)-3-(carboxy-alkyle en C₁ à C₃)-aminocarbonylamino,
un groupe cycloalkylène-imino de 4 à 7 chaînons, substitué par un groupe hydroxy,
un groupe cycloalkylène-imino de 5 à 7 chaînons, éventuellement substitué par un groupe alkyle en C₁ à C₃, sur lequel un cycle phényle est condensé sur deux atomes de carbone voisins,
un groupe morpholino, pipérazino, N-(alkyle en C₁ à C₃)-pipérazino, pyrrolino, 3,4-déhydro-pipéridino ou pyrrol-1-yle,
un groupe R₂-CX-cycloalkyle en C₃ à C₅, dans lequel
R₂ représente un groupe phényle, naphtyle ou hétéroaryle monocyclique à 5 ou 6 chaînons éventuellement substitué par un groupe alkyle en C₁ à C₃, le groupe hétéroaryle à 6 chaînons contenant un, deux ou trois atomes d'azote et le groupe hétéroaryle à 5 chaînons contenant un groupe imino éventuellement substitué par un groupe alkyle en C₁ à C₃, un atome d'oxygène ou de soufre ou un groupe imino éventuellement substitué par un groupe alkyle en C₁ à C₃, et un atome d'oxygène ou de soufre ou un ou deux atomes d'azote, et le substituant alkyle mentionné précédemment pouvant être substitué par un groupe carboxy, carboxy-alcoxy en C₁ à C₃, carboxy-alkylamino en C₁ à C₃ ou N-(alkyle en C₁ à C₃)-carboxy-alkylamino en C₁ à C₃, et
X représente un atome d'oxygène, un groupe alkylimino en C₁ à C₃, alcoxyimino en C₁ à C₃, alkyle en C₁ à C₃-hydrazino, di-(alkyle en C₁ à C₃)-hydrazino, alcanoyl en C₂ à C₄-hydrazino, N-(alkyle en C₁ à C₃)-(alcanoyle en C₂ à C₄)-hydrazino ou alkylidène en C₁ à C₃, qui peuvent être substitués dans la partie alkyle ou alcanoyle ou dans la partie alkyle et alcanoyle par un groupe carboxy,
un groupe alkyle en C₁ à C₃ ou cycloalkyle en C₃ à C₅, substitué par un groupe imidazole ou imidazolone, dans lesquels
le cycle imidazole peut être substitué par un groupe phényle ou carboxy et par un ou deux groupes alkyle en C₁ à C₃ ou par un, deux ou trois groupes alkyle en C₁ à C₃, les substituants pouvant être identiques ou différents et un des substituants alkyle mentionnés précédemment pouvant être substitué en même temps par par un groupe carboxy ou, à la position 2 ou 3, par un groupe amino, alcanoylamino en C₂ à C₄, alkylamino en C₁ à C₃, N-(alcanoyl en C₂ à C₄)-alkyle en C₁ à C₃-amino ou di-(alkyle en C₁ à C₃)-amino, et
le cycle imidazolone peut être substitué par un groupe alkyle en C₁ à C₃, le substituant alkyle pouvant être substitué par un groupe carboxy ou, à la position 2 ou 3, par un groupe amino, alcanoylamino en C₂ à C₄, alkylamino en C₁ à C₃, N-(alcanoyl en C₂ à C₄)-(alkyle en C₁ à C₃)-amino ou di-(alkyle en C₁ à C₃)-amino, et
un cycle phényle ou pyridine peut de plus être condensé sur deux atomes de carbone voisins des cycles imidazole et imidazolone mentionnés précédemment,
un groupe imidazolidine-2,4-dion-5-yle, qui peut être substitué par un ou deux groupes alkyle en C₁ à C₃, un substituant alkyle pouvant être en même temps substitué par un groupe carboxy,
un groupe alkyle en C₁ à C₄, qui peut être substitué par un groupe alkyle en C₁ à C₃-Y₁-alkyle en C₁ à C₃, HOOC-alkyle en C₁ à C₃-Y₁-alkyle en C₁ à C₃, tétrazolyl-alkyle en C₁ à C₃-Y₂, R₃NR₄ ou R₃NR₄-alkyle en C₁ à C₃ et
par un groupe isoxazolidinyl-carbonyle éventuellement substitué par un groupe alkyle en C₁ à C₃, par un groupe pyrrolinocarbonyle, 3,4-déhydropipéridinocarbonyle, pyrrol-1-yl-carbonyle, carboxy, aminocarbonyle, alkyle en C₁ à C₃-aminocarbonyle, di-(alkyle en C₁ à C₃)-aminocarbonyle ou cycloalkylèniminocarbonyle de 4 à 7 chaînons, la partie cycloalkylène-imino des groupes mentionnés précédemment peut être substituée par un ou deux groupes alkyle en C₁ à C₃ et en même temps le cas échéant une partie alkyle ou un substitueant alkyle des groupes alkylaminocarbonyle en C₁ à C₃, di-(alkyle en C₁ à C₃)-aminocarbonyle ou cycloalkylèniminocarbonyle mentionnés précédemment peut être substitué par un groupe carboxy, et les atomes d'hydrogène restant du groupe alkyle en C₁ à C₄ peuvent être remplacés totalement ou partiellement par des atomes de fluor, dans lesquels
R₃ représente un atome d'hydrogène ou un groupe alkyle en C₁ à C₃ éventuellement substitué par un groupe carboxy et
R₄ représente un atome d'hydrogène, un groupe alkyle en C₁ à C₃-Y₁-alkyle en C₁ à C₃-Y₂, carboxy-alkyle en C₁ à C₃-Y₁-alkyle en C₁ à C₃-Y₂, alkyle en C₁ à C₃-Y₂ ou carboxy-alkyle en C₁ à C₃-Y₂ ou
R₃ et R₄ conjointement avec l'atome d'azote situé entre eux représentent un groupe cycloalkylène-imino de 4 à 7 chaînons substitué par un groupe carboxy, alkyle en C₁ à C₃ ou carboxy-alkyle en C₁ à C₃, dans lesquels
Y₁ représente une liaison carbone-carbone, un atome d'oxygène, un groupe sulfényle, sulfinyle, sulfonyle, -NH-, -NH-CO- ou -NH-CO-NH- et
Y₂ représente une liaison carbone-azote ou un groupe carbonyle, sulfonyle, imino ou -NH-CO-, le groupe carbonyle du groupe -NH-CO- étant rattaché à l'atome d'azote du groupe R₃NR₄, et les groupes imino existant de par la définition des résidus Y₁ et Y₂ pouvant de plus être substitués par un groupe alkyle en C₁ à C₃ ou carboxy-alkyle en C₁ à C₃,
un groupe alkyle en C₁ à C₃ ou cycloalkyle en C₃ à C₅ substitués par un groupe R₅NR₆, dans lesquels
R₅ représente un atome d'hydrogène, un groupe alkyle en C₁ à C₃, cycloalkyle en C₅ à C₇, phénylcarbonyle, phénylsulfonyle ou pyridinyle et
R₆ représente un groupe alkyle en C₁ à C₃, carboxy-alkyle en C₁ à C₃ ou carboxy-alkylcarbonyle en C₁ à C₃,
un groupe alkyle en C₁ à C₃, qui est substitué par un groupe alcanoyle en C₂ à C₄ ou cycloalcanoyle en C₅ à C₇ et par un groupe alkyle en C₁ à C₃ substitué par un atome de chlore, de brome ou d'iode,
R_{b} signifie un atome d'hydrogène ou un groupe alkyle en C₁ à C₃ et
R_{c} signifie un groupe cyano ou un groupe amidino éventuellement substitué par un ou deux groupes alkyle en C₁ à C₃, dans lequel
les groupes carboxy mentionnés dans la définition des résidus mentionnés précédemment peuvent en outre être remplacés par un groupe qui peut être converti in-vivo en un groupe carboxy ou par un groupe chargé négativement dans des conditions physiologiques ou
les groupes amino et imino mentionnés dans la définition des résidus mentionnés précédemment peuvent en outre être substitués par un résidu qui peut être coupé in-vivo,
un groupe qui peut être converti *in vivo* en un groupe carboxy étant un groupe hydroxyméthyle, un groupe carboxy estérifié avec un alcool, dans lequel la partie alcool peut être un alcanol en C₁ à C₆, un phényl-alcanol en C₁ à C₃, un cycloalcanol en C₃ à C₉, le cycloalcanol en C₅ à C₈ pouvant être substitué en outre par un ou deux groupes alkyle en C₁ à C₃, un cycloalcanol en C₅ à C₈, dans lequel un groupe méthylène en position 3 ou 4 est remplacé par un atome d'oxygène ou par un groupe imino éventuellement substitué par un groupe alkyle en C₁ à C₃, phényl-alkyle en C₁ à C₃, phényl-alcoxy en C₁ à C₃-carbonyle ou alcanoyle en C₂ à C₆ et la partie cycloalcanol peut de plus être substituée par un ou deux groupes alkyle en C₁ à C₃, un cycloalcénol en C₄ à C₇, un alcénol en C₃ à C₅, un phényl-alcénol en C₃ à C₅, un alcynol en C₃ à C₅, ou phényl-alcynol en C₃ à C₅, à la condition qu'aucune liaison de l'atome d'oxygène ne provienne d'un atome de carbone, qui porte une liaison double ou triple, un cycloalkyle en C₃ à C₈-alcanol en C₁ à C₃, un bicycloalcanol ayant en tout 8 à 10 atomes de carbone, qui peut de plus être substitué par un ou deux groupes alkyle en C₁ à C₃ dans la partie bicycloalkyle, un 1,3-dihydro-3-oxo-1-isobenzofuranol ou un alcool de formule
R_{d}-CO-O-(RₑCR_{f})-OH,
dans lequel
R_{d} représente un groupe alkyle en C₁ à C₈, cycloalkyle en C₅ à C₇, phényle ou ρhényl-alkyle en C₁ à C₃,
Rₑ représente un atome d'hydrogène, un groupe alkyle en C₁ à C₃, cycloalkyle en C₅ à C₇ ou phényle et
R_{f} représente un atome d'hydrogène ou un groupe alkyle en C₁ à C₃,
un groupe chargé négativement dans des conditions physiologiques étant un groupe tétrazol-5-yle, phénylcarbonylaminocarbonyle, tritluorométhylcarbonyl-aminocarbonyle, alkylsulfonylamino en C₁ à C₆, phénylsulfonylamino, benzylsulfonylamino, trifluorométhylsulfonylamino, alkylsulfonylaminocarbonyle en C₁ à C₆, phénylsulfonylaminocarbonyle, benzylsulfonylaminocarbonyle ou perfluoro-alkyle en C₁ à C₆-sulfonylaminocarbonyle, et
un résidu qui peut être coupé *in vivo* à partir d'un groupe imino ou amino étant un groupe hydroxy, un groupe benzoyle éventuellement mono- ou di-substitué par un atome de fluor, de chlore, de brome ou d'iode, par des groupes alkyle en C₁ à C₃, ou alcoxy en C₁ à C₃, les substituants pouvant être identiques ou différents, un groupe pyridinoyle ou un groupe alcanoyle en C₁ à C₁₆, un groupe 3,3,3-trichloropropionyle ou allyloxycarbonyle, un groupe alcoxy en C₁ à C₁₋₆-carbonyle ou alkyle en C₁ à C₁₆-carbonyloxy, dans lesquels les atomes d'hydrogène peuvent être totalement ou partiellement remplacés par des atomes de fluor ou de chlore, un groupe phényl-alcoxy en C₁ à C₆-carbonyle, un groupe 3-aminopropionyle, dans lequel le groupe amino peut être mono- ou di-substitué par des groupes alkyle en C₁ à C₆ ou cycloalkyle en C₃ à C₇, et les substituants pouvant être identiques ou différents, un groupe alkylsulfonyle en C₁ à C₃-alcoxycarbonyle en C₂ à C₄, alcoxy en C₁ à C₃-alcoxy en C₂ à C₄-alcoxy en C₂ à C₄-carbonyle, R_{d}-CO-O-(R_{d}CR_{f})-O-CO-, alkyle en C₁ à C₆-CO-NH-(R_{g}CRₕ)-O-CO- ou alkyle en C₁ à C₆-CO-O-(R_{g}CRₕ)-(R_{g}CRₕ)-O-CO-, dans lesquels R_{d} à R_{f} sont définis comme mentionné précédemment et
R_{g} et Rₕ, qui peuvent être identiques ou différents, représentent des atomes d'hydrogène ou des groupes alkyle en C₁ à C₃,
les tautomères, stéréoisomères, mélanges et sels de ceux-ci,
éventuellement sous forme de sels d'addition d'acide pharmaceutiquement acceptables tout comme éventuellement sous forme d'hydrates ou de solvates pour la fabrication d'un médicament pour prévenir ou traiter des maladies choisies dans le groupe constitué du syndrome de réponse inflammatoire systémique (« systemic inflammatory response syndrome ») (SIRS), de la septicémie et de l'infection bactérienne.

2. Utilisation selon la revendication 1, dans laquelle on utilise, en tant que benzimidazole, le (R)-2-(4-amidinophénylaminométhyl)-1-méthyl-5-[1-(carboxyméthylamino)-1-(pyrrolidinocarbonyl)-éthyl]-benzimidazole, éventuellement sous forme de sel d'addition d'acide pharmaceutiquement acceptable ainsi qu'éventuellement sous forme d'hydrate ou de solvate.

3. Utilisation selon la revendication 2, dans laquelle on utilise le sel de monohydrochlorure du (R)-2-(4-amidinophénylaminométhyl)-1-méthyl-5-[1-(carboxyméthylamino)-1-(pyrrolidinocarbonyl)-éthyl]-benzimidazole.

4. Utilisation selon l'une quelconque des revendications 1 à 3, dans laquelle la maladie est choisie dans le groupe constitué du SIRS provoqué par des agents pathogènes Gram-positifs, du SIRS provoqué par des agents pathogènes Gram-négatifs, du SIRS provoqué par des virus, du SIRS provoqué par des parasites eucaryotes monocellulaires, du SIRS provoqué par des champignons, de la septicémie sans défaillance d'organe, de la septicémie avec défaillance d'organe, du choc septique, du syndrôme septique, du SIRS provoqué par des pancréatites, du SIRS provoqué par des ischémies systémiques, du SIRS provoqué par des ischémies limitées à un organe, du SIRS provoqué par des traumatismes, du SIRS en relation avec des maladies tumorales, du SIRS provoqué par des blessures des tissus, du SIRS provoqué par des brûlures, du SIRS faisant suite à des opérations invasives, du SIRS faisant suite à une transplantation d'organes, du SIRS dû à des chocs de différentes origines, du SIRS faisant suite à un choc hémorragique, du SIRS faisant suite à une insuffisance cardio-vasculaire, du SIRS faisant suite à une insuffisance d'organe de cause immunitaire, du SIRS faisant suite à des réactions inflammatoires, du SIRS faisant suite à un traitement avec des médiateurs de l'inflammation comme par exemple les facteurs de nécrose tumorale alpha et/ou beta, et/ou d'autres cytokines et constitué de plus des dommages pulmonaires en relation avec le SIRS, de l'insuffisance pulmonaire aiguë et de l'ARDS (acute respiratory distress syndrome), de l'insuffisance cardio-vasculaire aigüe, de l'insuffisance d'organe après une réanimation, du choc, de l'insuffisance rénale, de l'insuffisance cardio-vasculaire, des dommages hématologiques, de l'azidose et du syndrome de dysfonctionnement d'organes multiples (« multiple organ dysfunction syndrome ») (MODS).

5. Utilisation selon l'une quelconque des revendications 1 à 4, dans laquelle le médicament est prévu comme traitement d'accompagnement lors d'infections bactériennes.

6. Utilisation selon l'une quelconque des revendications 1 à 5, dans laquelle le médicament est prévu pour une administration sous-cutanée ou parentérale et en particulier intravéneuse.

7. Utilisation selon l'une quelconque des revendications 1 à 6, dans laquelle on met en oeuvre, en plus d'une des substances nommées dans les revendications 1 et 2, une substance pharmaceutiquement active choisie dans le groupe constitué des inhibiteurs de la fonction plaquettaire comme en particulier l'acide acétylsalicylique, les antagonistes du récepteur du fibrinogène, les inhibiteurs de l'agrégation induite par l'ADP, les antagonistes du récepteur P₂T et des combinaisons antagonistes du récepteur du thromboxane / inhibiteurs de synthétase, consitué de plus de substances actives thrombolytiques, comme en particulier l'altéplase, la retéplase, la ténectéplase, l'urokinase, la staphylokinase et la streptokinase, constitué de plus d'activateurs physiologiques et d'inhibiteurs du système de la coagulation et de leurs analogues recombinants comme en particulier la protéine C, la protéine C recombinante humaine activée, le TFPI et l'antithrombine, constitué de plus de substances ayant une activité antagoniste contre l'endotoxine, ainsi que constitué de plus d'interleukines, du TNF, de la bradykinine, de prostaglandines, de cyclooxygénases, du NO, d'acétylhydrolases du facteur d'activation des plaquettes, d'inhibiteurs de l'inflammation, de substances immunosuppressives, d'antibiotiques et de catécholamines.

8. Composition pharmaceutique comprenant
(a) au moins une substance active choisie dans le groupe des benzimidazoles de formule générale (I) 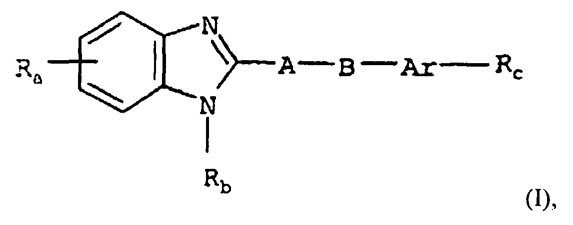 dans laquelle
Ar représente un groupe phénylène ou naphtylène éventuellement substitué par un atome de fluor, de chlore ou de brome, par un groupe trifluorométhyle, alkyle en C₁ à C₃ ou alcoxy en C₁ à C₃,
un groupe thiénylène, thiazolylène, pyridinylène, pyrimidinylène, pyrazinylène ou pyridazinylène éventuellement substitué sur le squelette carboné par un groupe alkyle en C₁ à C₃,
A représente un groupe alkylène en C₁ à C₃,
B représente un atome d'oxygène ou de soufre, un groupe méthylène, carbonyle, sulfinyle ou sulfonyle, un groupe imino éventuellement substitué par un groupe alkyle en C₁ à C₃, dans lequel la partie alkyle peut être mono- ou di-substituée par un groupe carboxy,
Rₐ représente un groupe R₁-CO-cycloalkyle en C₃ à C₅, dans lequel
R₁ représente un groupe alcoxy en C₁ à C₃, amino, alkylamino en C₁ à C₄ ou di-(alkyle en C₁ à C₄)-amino, dans lesquels la partie alkyle peut être substituée par un groupe carboxy,
un groupe cycloalkylène-imino ou cycloalkènylène-imino de 4 à 7 chaînons, qui peuvent être substitués par un ou deux groupes alkyle en C₁ à C₃, dans lequel un substituant alkyle peut être substitué en même temps par un groupe hydroxy, alcoxy en C₁ à C₃, carboxy, carboxy-alcoxy en C₁ à C₃, carboxy-alkylamino en C₁ à C₃, N-(alkyle en C₁ à C₃)-N-(carboxy-alkyle en C₁ à C₃)-amino, carboxy-alkylaminocabonyle en C₁ à C₃, N-(alkyle en C₁ à C₃)-N-(carboxy-alkyle en C₁ à C₃)-aminocarbonyle, carboxy-alkyle en C₁ à C₃-aminocarbonylamino, 1-(alkyle en C₁ à C₃)-3-(carboxy- alkyle en C₁ à C₃)-aminocarbonylamino, 3-(alkyle en C₁ à C₃)-3-(carboxy-alkyle en C₁ à C₃)-aminocarbonylamino ou 1,3-di-(alkyle en C₁ à C₃)-3-(carboxy-alkyle en C₁ à C₃)-aminocarbonylamino,
un groupe cycloalkylène-imino de 4 à 7 chaînons substitué par un groupe hydroxy,
un groupe cycloalkylène-imino de 5 à 7 chaînons éventuellement substitué par un groupe alkyle en C₁ à C₃, sur lequel un cycle phényle est condensé sur deux atomes de carbone voisins,
un groupe morpholino, pipérazino, N-(alkyle en C₁ à C₃)-pipérazino, pyrrolino, 3,4-déhydro-pipéridino ou pyrrol-1-yle,
un groupe R₂-CX-cycloalkyle en C₃ à C₅, dans lequel
R₂ représente un groupe phényle, naphtyle ou hétéroaryle monocyclique à 5 ou 6 chaînons éventuellement substitué par un groupe alkyle en C₁ à C₃, le groupe hétéroaryle à 6 chaînons contenant un, deux ou trois atomes d'azote et le groupe hétéroaryle à 5 chaînons contenant un groupe imino éventuellement substitué par un groupe alkyle en C₁ à C₃, un atome d'oxygène ou de soufre ou un groupe imino éventuellement substitué par un groupe alkyle en C₁ à C₃ et un atome d'oxygène ou de soufre ou un ou deux atomes d'azote et le substituant alkyle mentionné précédemment peut être substitué par un groupe carboxy, carboxy-alcoxy en C₁ à C₃, carboxy-alkylamino en C₁ à C₃ ou N-(alkyle en C₁ à C₃)-carboxy-alkylamino en C₁ à C₃, et
X représente un atome d'oxygène, un groupe alkylimino en C₁ à C₃, alcoxyimino en C₁ à C₃, alkyle en C₁ à C₃-hydrazino, di-(alkyle en C₁ à C₃)-hydrazino, alcanoylhydrazino en C₂ à C₄, N-(alkyle en C₁ à C₃)-alcanoylhydrazino en C₂ à C₄ ou alkylidène en C₁ à C₃, qui peuvent être substitués dans la partie alkyle ou alcanoyle ou dans la partie alkyle et alcanoyle par un groupe carboxy,
un groupe alkyle en C₁ à C₃ ou cycloalkyle en C₃ à C₅ substitués par un groupe imidazole ou imidazolone, dans lesquels
le cycle imidazole peut être substitué par un groupe phényle ou carboxy et par un ou deux groupes alkyle en C₁ à C₃ ou par un, deux ou trois groupes alkyle en C₁ à C₃, les substituants pouvant être identiques ou différents et un des substituants alkyle mentionnés précédemment pouvant être substitué en même temps par par un groupe carboxy ou à la position 2 ou 3 par un groupe amino, alcanoylamino en C₂ à C₄, alkylamino en C₁ à C₃, N-(alcanoyle en C₂ à C₄)-alkylamino en C₁ à C₃ ou di-(alkyle en C₁ à C₃)-amino, et
le cycle imidazolone peut être substitué par un groupe alkyle en C₁ à C₃, dans lequel le substituant alkyle peut être substitué par un groupe carboxy ou à la position 2 ou 3 par un groupe amino, alcanoylamino en C₂ à C₄, alkylamino en C₁ à C₃, N-(alcanoyl en C₂ à C₄)-alkylamino en C₁ à C₃ ou di-(alkyle en C₁ à C₃)-amino, et
un cycle phényle ou pyridine peut de plus être condensé sur deux atomes de carbone voisins des cycles imidazole et imidazolone mentionnés précédemment,
un groupe imidazolidin-2,4-dion-5-yle, qui peut être substitué par un ou deux groupes alkyle en C₁ à C₃, dans lequel un substituant alkyle peut être en même temps substitué par un groupe carboxy,
un groupe alkyle en C₁ à C₄,
qui peut être substitué par un groupe alkyle en C₁ à C₃-Y₁-alkyle en C₁ à C₃, HOOC-alkyle en C₁ à C₃-Y₁-alkyle en C₁ à C₃, tétrazolyl-alkyle en C₁ à C₃-Y₂, R₃NR₄ ou R₃NR₄-alkyle en C₁ à C₃ et
par un groupe isoxazolidinyl-carbonyle éventuellement substitué par un groupe alkyle en C₁ à C₃, par un groupe pyrrolinocarbonyle, 3,4-déhydropipéridinocarbonyle, pyrrol-1-yl-carbonyle, carboxy, aminocarbonyle, alkyle en C₁ à C₃-aminocarbonyle, di-(alkyle en C₁ à C₃)-aminocarbonyle ou cycloalkylène-iminocarbonyle de 4 à 7 chaînons, la partie cycloalkylène-imino des groupes mentionnés précédemment pouvant être substituée par un ou deux groupes alkyle en C₁ à C₃ et en même temps le cas échéant une partie alkyle ou un substituant alkyle des groupes alkyle en C₁ à C₃-aminocarbonyle, di-(alkyle en C₁ à C₃)-aminocarbonyle ou cycloalkylène-iminocarbonyle mentionnés précédemment pouvant être substitué par un groupe carboxy, et les atomes d'hydrogène restant du groupe alkyle en C₁ à C₄ pouvant être remplacés totalement ou partiellement par des atomes de fluor, dans lesquels
R₃ représente un atome d'hydrogène ou un groupe alkyle en C₁ à C₃ éventuellement substitué par un groupe carboxy et
R₄ représente un atome d'hydrogène, un groupe alkyle en C₁ à C₃-Y₁-alkyle en C₁ à C₃-Y₂, carboxy-alkyle en C₁ à C₃-Y₁-alkyle en C₁ à C₃-Y₂, alkyle en C₁ à C₃-Y₂ ou carboxy-alkyle en C₁ à C₃-Y₂ ou
R₃ et R₄ conjointement avec l'atome d'azote situé entre eux représentent un groupe cycloalkylènimino de 4 à 7 chaînons substitué par un groupe carboxy, alkyle en C₁ à C₃ ou carboxy-alkyle en C₁ à C₃, dans lesquels
Y₁ représente une liaison carbone-carbone, un atome d'oxygène, un groupe sulfényle, sulfinyle, sulfonyle, -NH-, -NH-CO- ou -NH-CO-NH- et
Y₂ représente une liaison carbone-azote ou un groupe carbonyle, sulfonyle, imino ou -NH-CO-, sachant que le groupe carbonyle du groupe -NH-CO- est rattaché à l'atome d'azote du groupe R₃NR₄, et les groupes imino dans la définition des résidus Y₁ et Y₂ peuvent de plus être substitués par un groupe alkyle en C₁ à C₃ ou carboxy-alkyle en C₁ à C₃,
un groupe alkyle en C₁ à C₃ ou cycloalkyle en C₃ à C₅ substitué par un groupe R₅NR₆, dans lesquels
R₅ représente un atome d'hydrogène, un groupe alkyle en C₁ à C₃, cycloalkyle en C₅ à C₇, phénylcarbonyle, phénylsulfonyle ou pyridinyle et
R₆ représente un groupe alkyle en C₁ à C₃, carboxy-alkyle en C₁ à C₃ ou carboxy-alkylcarbonyle en C₁ à C₃,
un groupe alkyle en C₁ à C₃, qui est substitué par un groupe alcanoyle en C₂ à C₄ ou cycloalcanoyle en C₅ à C₇ et par un groupe alkyle en C₁ à C₃ substitué par un atome de chlore, de brome ou d'iode,
R_{b} signifie un atome d'hydrogène ou un groupe alkyle en C₁ à C₃ et
R_{c} signifie un groupe cyano ou un groupe amidino éventuellement substitué par un ou deux groupes alkyle en C₁ à C₃, dans lequel
les groupes carboxy mentionnés dans la définition des résidus mentionnés précédemment peuvent en outre être remplacés par un groupe qui peut être converti in-vivo en un groupe carboxy ou par un groupe chargé négativement dans des conditions physiologiques ou
les groupes amino et imino mentionnés dans la définition des résidus mentionnés précédemment peuvent en outre être substitués par un résidu qui peut être coupé in-vivo,
un groupe qui peut être converti in-vivo en un groupe carboxy étant un groupe hydroxyméthyle, un groupe carboxy estérifié avec un alcool, dans lequel la partie alcool peut être un alcanol en C₁ à C₆, un phényl-alcanol en C₁ à C₃, un cycloalcanol en C₃ à C₉, le cycloalcanol en C₅ à C₈ pouvant être substitué par un ou deux groupes alkyle en C₁à C₃, un cycloalcanol en C₅ à C₈, dans lequel un groupe méthylène en position 3 ou 4 est remplacé par un atome d'oxygène ou par un groupe imino éventuellement substitué par un groupe alkyle en C₁ à C₃, phényl-alkyle en C₁ à C₃, phényl-alcoxycarbonyle en C₁ à C₃ ou alcanoyle en C₂ à C₆ et la partie cycloalcanol peut de plus être substituée par un ou deux groupes alkyle en C₁ à C₃, un cycloalcénol en C₄ à C₇, un alcénol en C₃ à C₅, un phényl-alcénol en C₃ à C₅, un alcynol en C₃ à C₅ ou phényl-alcynol en C₃ à C₅, à la condition qu'aucune liaison de l'atome d'oxygène ne provienne d'un atome de carbone qui porte une liaison double ou triple, un cycloalkyle en C₃ à C₈-alcanol en C₁ à C₃, un bicycloalcanol ayant en tout 8 à 10 atomes de carbone, qui peut de plus être substitué par un ou deux groupes alkyle en C₁ à C₃ dans la partie bicycloalkyle, un 1,3-dihydro-3-oxo-1-isobenzofuranol ou un alcool de formule
R_{d}-CO-O-(RₑCR_{f})-OH,
dans lequel
R_{d} représente un groupe alkyle en C₁ à C₈, cycloalkyle en C₅ à C₇, phényle ou phényl-alkyle en C₁ à C₃,
Rₑ représente un atome d'hydrogène, un groupe alkyle en C₁ à C₃, cycloalkyle en C₅ à C₇ ou phényle et
R_{f} représente un atome d'hydrogène ou un groupe alkyle en C₁ à C₃,
un groupe chargé négativement dans des conditions physiologiques étant un groupe tétrazol-5-yle, phénylcarbonylaminocarbonyle, tritluorométhylcarbonyl-aminocarbonyle, alkylsulfonylamino en C₁ à C₆, phénylsulfonylamino, benzylsulfonylamino, trifluorométhylsulfonylamino, alkylsulfonylaminocarbonyle en C₁ à C₆, phénylsulfonylaminocarbonyle, benzylsulfonylaminocarbonyle ou perfluoro-alkylsulfonylaminocarbonyle en C₁ à C₆
et un résidu qui peut être coupé in-vivo à partir d'un groupe imino ou amino étant un groupe hydroxy, un groupe benzoyle éventuellement mono- ou di-substitué par un atome de fluor, de chlore, de brome ou d'iode, par des groupes alkyle en C₁ à C₃, ou alcoxy en C₁ à C₃, les substituants pouvant être identiques ou différents, un groupe pyridinoyle ou un groupe alcanoyle en C₁ à C₁₆, un groupe 3,3,3-trichloropropionyle ou allyloxycarbonyle, un groupe alcoxy en C₁ à C₁₆-carbonyle ou alkyle en C₁ à C₁₆-carbonyloxy, dans lesquels les atomes d'hydrogène peuvent être totalement ou partiellement remplacés par des atomes de fluor ou de chlore, un groupe phényl-alcoxycarbonyle en C₁ à C₆, un groupe 3-aminopropionyle, dans lequel le groupe amino peut être mono- ou di-substitué par des groupes alkyle en C₁ à C₆ ou cycloalkyle en C₃ à C₇, et les substituants peuvent être identiques ou différents, un groupe alkylsulfonyle en C₁ à C₃-alcoxycarbonyle en C₂ à C₄, alcoxy en C₁ à C₃-alcoxy en C₂ à C₄-alcoxycarbonyle en C₂ à C₄, R_{d}-CO-O-(R_{d}CR_{f})-O-CO-, alkyle en C₁ à C₆-CO-NH-(R_{g}CRₕ)-O-CO- ou alkyle en C₁ à C₆-CO-O-(R_{g}CRₕ)-(RgCRₕ)-O-CO-, dans lesquels R_{d} à R_{f} sont définis comme mentionné précédemment et
R_{g} et Rₕ, qui peuvent être identiques ou différents, représentent des atomes d'hydrogène ou des groupes alkyle en C₁ à C₃,
les tautomères, stéréoisomères, mélanges et sels de ceux-ci, et
(b) au moins une substance active choisie dans le groupe constitué des inhibiteurs de la fonction plaquettaire - comme en particulier l'acide acétylsalicylique, les antagonistes du récepteur du fibrinogène, les inhibiteurs de l'agrégation induite par l'ADP, les antagonistes du récepteur P₂T et des combinaisons antagonistes du récepteur du thromboxane / inhibiteurs de synthétase -, de substances actives thrombolytiques - comme en particulier l'altéplase, la retéplase, la ténectéplase, l'urokinase, la staphylokinase et la streptokinase -, d'activateurs physiologiques et d'inhibiteurs du système de la coagulation et de leurs analogues recombinants - comme en particulier la protéine C, la protéine C recombinante humaine activée, le TFPI et l'antithrombine -, de substances ayant une activité antagoniste contre l'endotoxine, d'interleukines, du TNF, de la bradykinine, de prostaglandines, de cyclooxygénases, du NO, d'acétylhydrolases du facteur d'activation des plaquettes, d'inhibiteurs de l'inflammation, de substances immunosuppressives, d'antibiotiques et de catécholamines.

9. Composition pharmaceutique selon la revendication 8, comprenant
(a) le (R)-2-(4-amidinophénylaminométhyl)-1-méthyl-5-[1-(carboxyméthylamino)-1-(pyrrolidinocarbonyl)-éthyl]-benzimidazole, éventuellement sous forme de sel d'addition d'acide pharmaceutiquement acceptable ainsi qu'éventuellement sous forme d'hydrate ou de solvate, et
(b) une PAF-AH ou un dérivé de PAF-AH.

10. Kit de médicament comprenant au moins
(a) le (R)-2-(4-amidinophénylaminométhyl)-1-méthyl-5-[1-(carboxyméthylamino)-1-(pyrrolidinocarbonyl)-éthyl]-benzimidazole, éventuellement sous forme de sel d'addition d'acide pharmaceutiquement acceptable ainsi qu'éventuellement sous forme d'hydrate ou de solvate, et
(b) une PAF-AH ou un dérivé de PAF-AH.

11. Kit de médicament comprenant au moins
(a) le (R)-2-(4-amidinophénylaminométhyl)-1-méthyl-5-[1-(carboxyméthylamino)-1-(pyrrolidinocarbonyl)-éthyl]-benzimidazole, éventuellement sous forme de sel d'addition d'acide pharmaceutiquement acceptable ainsi qu'éventuellement sous forme d'hydrate ou de solvate, et
(b) un antagoniste du facteur de nécrose tumorale alpha (TNF-alpha).

12. Utilisation d'une PAF-AH ou d'un dérivé de PAF-AH ou d'un antagoniste du TNF-alpha afin de fabriquer un médicament pour une utilisation combinée avec le (R)-2-(4-amidinophénylaminométhyl)-1-méthyl-5-[1-(carboxyméthylamino)-1-(pyrrolidinocarbonyl)-éthyl]-benzimidazole.
